# EUROPEAN PATENT APPLICATION

(11) **EP 3 783 095 A1**
(43) Date of publication of application: **24.02.2021**
(21) Application number: 19789009.8
(22) Date of filing: 17.04.2019
(51) Int. Cl.: C12M 3/00, B01J 13/02

(54) **BIOLOGICAL CARTRIDGE, BIOLOGICAL CARTRIDGE ASSEMBLY, MICROSPHERE PREPARATION DEVICE, SHELL ASSEMBLING DEVICE, BIOBRICK PREPARATION INSTRUMENT, BIOLOGICAL INK PREPARATION INSTRUMENT, AND BIOLOGICAL INK PREPARATION SYSTEM**

(30) Priority: 17.04.2018 CN 201810341066
(71) Applicant: Revotek Co., Ltd, Chengdu, Sichuan 611731 (CN)
(72) Inventor: ZHANG, Yaya, Chengdu, Sichuan 611731 (CN); WANG, Deming, Chengdu, Sichuan 611731 (CN); HUANG, Yushi, Chengdu, Sichuan 611731 (CN); JING, Wende, Chengdu, Sichuan 611731 (CN)
(74) Representative: Kolster Oy Ab
(86) International application number: PCT/CN2019/083011
(87) International publication number: WO 2019/201270

(57) **Abstract**

A biological cartridge, a biological cartridge assembly, a microsphere preparation device, a shell assembling device, a biobrick preparation instrument, a biological ink preparation instrument, and a biological ink preparation system. A biological cartridge (131) comprises: a cartridge housing (1311), having a cavity; an isolation wall (1312), provided in the cavity, and dividing the cavity into a first accommodating cavity (1313) and a second accommodating cavity (1314) isolated from each other; a first cartridge inlet (13151) and a first cartridge outlet (13153), provided on the cartridge housing (1311), and communicated with the first accommodating cavity (1313) and fluidly isolated from the second accommodating cavity (1314); a second cartridge inlet (13152) and a second cartridge outlet (13154), provided on the cartridge housing (1311), and communicated with the second accommodating cavity (1314); and an internal flow channel (1316), located in the second accommodating cavity (1314), and communicated with the first accommodating cavity (1313) and the first cartridge outlet (13153).

## Description

### Cross-Reference to Related Applications

The present application claims priority to China Patent Application No. 201810341066.5 filed on April 17, 2018 for "Bio-ink cartridge, microsphere preparation device, shell assembly device and bio-block preparation instrument", the contents of which are incorporated herein by reference in its entirety.

### Technical Field

The present disclosure relates to the field of bio-printing, and more particularly to a bio-ink cartridge, a bio-ink cartridge assembly, a microsphere preparation device, a shell assembly device, a bio-block preparation instrument, a bio-ink preparation instrument and a bio-ink preparation system.

### Description of the Related Art

3D (three-dimensional) bio-printing technology develops in recent years for preparing biological products, and it has been applied in practically building some complex tissues and organs. An important development direction of the 3D bio-printing technology is to produce bio-ink adequate for the bio-printing requirements. Cells contained in the bio-ink prepared in the prior art lack mechanical protection. Therefore, the cells are susceptible to damage and death due to external pressure and shear force during the 3D bio-printing process, imposing noticeable restrictions on the application of bio-printing technology.

Chinese patent with application No. CN201610211570.4 titled "Bio-block and Purpose Thereof" filed by the applicant of the present disclosure provides a base component for printing tissues or organs, i.e., bio-block. A bio-block comprises cells, a nuclear layer covering the cells and a shell layer encapsulating the nuclear layer. The nuclear layer and the shell layer are independently made from a degradable material. Since the type and number of these cells in the bio-block are adjustable, and the bio-block size is changed on demand, the bio-block is suitable for preparing a standardized and controllable bio-ink. In addition, the bio-ink prepared therefrom allows accurate cell arrangement in the bio-printing process for precise bio-printing of tissues or organs. Moreover, the bio-block mechanically provides sufficient protection for the cells for necessary cell survival rate during the bio-printing process. Therefore, the bio-ink prepared with the bio-block satisfies the 3D bio-printing requirements. The bio-block has been verified to meet the requirements for 3D bio-printing from cell extraction, bio-block preparation, preparation of bio-printing ink, printing of tissue constructs, clinical application through to industrialization.

Massive bio-block is required when it is applied as the main component of printing ink to build micro tissue explants by using the 3D bio-printing technology. For example, 0.3g bio-block is required for printing a blood vessel 2cm long at least to replace a pathological vessel; and the weight increases for some patients requiring more than 10 cm blood vessels. Therefore, the preparation efficiency of the bio-block limits the application of the 3D bio-printing technology.

In addition, the bio-block is employed to generate micro-tissues, and even to build 3D organs with the 3D bio-printing technology. The bio-block with at least 60% viable cells is the precondition of starting the bio-printing, and the success rate of bio-printing increases with the cell viability. Insufficiency in viable cells will result in non-survivable tissues or 3D organs.

Therefore, it is a problem to be addressed to keep the cell viability of the bio-block and improve the yield thereof.

To the best knowledge of the inventors, there are instruments or devices for preparing similar structures (e.g. a device for preparing microcapsules), but there is no advanced device for preparing the bio-block. The device in the prior art is mainly applied to prepare substances without biological activity, but the bio-block is biologically active and available for production and reprocessing in the subsequent industry chain. For example, for the 3D bio-printing, the printed tissue constructs grow, proliferate, differentiate or migrate in vivo, and play corresponding roles ultimately. Therefore, the device in the prior art is not suitable for preparing the bio-block or the intermediate structure thereof.

Chinese patent application No. CN201610212883.1 relates to the field of preparation of core-shell structures, particularly relates to a method and a device for preparing a core-shell structure. The method for preparing a core-shell structure comprises a liquid core material titration step: adding a liquid core material dropwise to a generation component with an acting surface, wherein the acting surface is hydrophobic and, under the action of the acting surface, allows the liquid core material to form a spheroid individually, or to be combined with a formed spheroid therein to further form a spheroid; a liquid shell material titration step: dribbling a liquid shell material to the generation component to cover the formed spheroid therein as a shell, so as to further form a spheroid in the generation component. According to the application, the liquid core material is dripped into the generation component for forming on the hydrophobic acting surface, so as to effectively control the shape of a formed core-shell structure and guarantee the core-shell structure with a high sphericity.

In the process of discovering the present disclosure, the inventors found that the low preparation efficiency and low bio-block activity remain unsolved in the bio-block preparation technology claimed in CN201610212883.1.

### Summary of the Invention

An object of the present disclosure is to provide a bio-ink cartridge, a bio-ink cartridge assembly, a microsphere preparation device, a shell assembly device, a bio-block preparation instrument, a bio-ink preparation instrument and a bio-ink preparation system to improve the efficiency of at least one of microsphere preparation, shell assembly and bio-block preparation; and furthermore, improve the activity of the prepared bio-block and an intermediate product thereof.

A first aspect of the present disclosure provides a bio-ink cartridge, comprising:
an ink cartridge shell with a cavity;
a dividing wall arranged in the cavity and dividing the cavity into a first accommodating chamber and a second accommodating chamber which are fluidly isolated from each other;
a first ink cartridge inlet and a first ink cartridge outlet arranged on the ink cartridge shell and communicated with the first accommodating chamber;
a second ink cartridge inlet and a second ink cartridge outlet arranged on the ink cartridge shell and communicated with the second accommodating chamber; and
an inner flow channel located in the second accommodating chamber and isolated from a fluid therein, and communicating the first accommodating chamber with the first ink cartridge outlet.

In some embodiments, the first accommodating chamber is arranged to match with the second accommodating chamber, the first ink cartridge inlet is arranged adjacent to the second ink cartridge inlet, and the first ink cartridge outlet is arranged adjacent to the second ink cartridge outlet, and the first ink cartridge inlet, the second ink cartridge inlet, the first ink cartridge outlet and the second ink cartridge outlet are arranged on the top or side of the ink cartridge shell, respectively.

In some embodiments, the dividing wall comprises multiple wall segments arranged at an angle, and the inner flow channel is connected between the first ink cartridge outlet and one of the multiple wall segments minimizing length of the inner flow channel.

In some embodiments, the inner flow channel is a linear flow channel, a zigzag flow channel, a curved flow channel, or a combination of the linear flow channel and the curved flow channel.

In some embodiments, an inner bottom surface of the ink cartridge shell comprises a first bottom surface located in the first accommodating chamber and a second bottom surface located in the second accommodating chamber; wherein,
the first bottom surface comprises a first low zone or a first low point to which the first bottom surface gradually descends from a location away therefrom; and/or
the second bottom surface comprises a second low zone or a second low point to which the second bottom surface gradually descends from a location away therefrom.

In some embodiments,
the ink cartridge shell comprises an ink cartridge positioning structure and an elastic snap-in structure externally arranged on opposite sides; and/or
the ink cartridge shell comprises a connecting structure for connecting multiple bio-ink cartridges to form a bio-ink cartridge assembly.

In some embodiments, the bio-ink cartridge comprises first sealing structures having a central through hole, which are arranged in the first ink cartridge inlet, the first ink cartridge outlet, the second ink cartridge inlet and the second ink cartridge outlet, respectively.

In some embodiments, the first sealing structure comprises a hollow column and a flange located at the top thereof, the hollow column is located in the corresponding ink cartridge inlet or ink cartridge outlet, and the flange is located on an end face of the corresponding ink cartridge inlet or ink cartridge outlet.

In some embodiments, the bio-ink cartridge further comprises:
a first guide wall which is arranged in the first accommodating chamber and extends downwards from a radial outside of the first ink cartridge outlet; and/or
a second guide wall which is arranged in the second accommodating chamber and extends downwards from a radial outside of the second ink cartridge outlet.

In some embodiments,
the first ink cartridge outlet is located between the first guide wall and a shell wall of the ink cartridge shell and/or at least portion of the dividing wall; and/or
The second ink cartridge outlet is located between the second guide wall and the shell wall of the ink cartridge shell and/or at least portion of the dividing wall.

In some embodiments, the bio-ink cartridge further comprises a reinforcing structure which is arranged in the cavity and located at the first ink cartridge inlet, the first ink cartridge outlet, the second ink cartridge inlet, and/or the second ink cartridge outlet.

In some embodiments, the reinforcing structure comprises reinforcing ribs which are connected with an inner surface of the ink cartridge shell and/or the dividing wall.

In some embodiments, a chamber wall surface of the first accommodating chamber is hydrophobic; and/or a chamber wall surface of the second accommodating chamber is hydrophobic.

In some embodiments, a volume ratio of the first accommodating chamber to the second accommodating chamber is 10: 1 to 25: 1.

A second aspect of the present disclosure provides a bio-ink cartridge assembly comprising the bio-ink cartridge according to any one of the first aspect.

In some embodiments, the bio-ink cartridge assembly further comprises an integrated connector which comprises:
a connector body having at least one first connector flow channel and/or at least one second connector flow channel; wherein an outlet end of the first connector flow channel is communicated with the first ink cartridge inlet of the bio-ink cartridge, and an outlet end of the second connector flow channel is communicated with the second ink cartridge inlet thereof;
fluid connectors connected onto the connector body, wherein an inlet end of the first connector flow channel is connected with a fluid connector and an inlet end of the second connector flow channel is connected with a fluid connector, respectively.

In some embodiments,
the inlet end of the first connector flow channel and the inlet end of the second connector flow channel are located on the same side of the connector body; and/or
the outlet end of the first connector flow channel and the outlet end of the second connector flow channel are located on the same side of the connector body; and/or
the inlet end and the outlet end of the first connector flow channel are located on different sides of the connector body; and/or
the inlet end and the outlet end of the second connector flow channel are located on different sides of the connector body.

In some embodiments, the bio-ink cartridge assembly further comprises an outlet integration head which comprises an integration head body having:
at least one first through hole for a pipe line connected with the first ink cartridge outlet of the bio-ink cartridge to pass through; and/or
at least one second through hole for a pipe line communicated with the second ink cartridge outlet of the bio-ink cartridge to pass through.

In some embodiments, the integration head body has:
at least one third through hole for a pipe line connecting the first ink cartridge outlet of the bio-ink cartridge with a second chip inlet of a microfluidic chip to pass through; and/or
at least one fourth through hole for a pipe line connecting the second ink cartridge outlet of the bio-ink cartridge with a first chip inlet of the microfluidic chip to pass through.

In some embodiments, the outlet integration head comprises a guide structure which leads the pipe line passing through the first through hole through the first ink cartridge outlet of the bio-ink cartridge into the first accommodating chamber, and/or leads the pipe line passing through the second through hole through the second ink cartridge outlet thereof into the second accommodating chamber.

In some embodiments, the guide structure comprises a guide plate which is movably arranged relative to the integration head body and located between the first ink cartridge outlet and/or the second ink cartridge outlet of the bio-ink cartridge and the integration head body, and the guide plate comprises a first guide hole corresponding to the first through hole and/or a second guide hole corresponding to the second through hole.

A third aspect of the present disclosure provides a microsphere preparation device comprising the bio-ink cartridge according to any one of the first aspect or the bio-ink cartridge assembly according to any one of the second aspect.

A fourth aspect of the present disclosure relates to a bio-block preparation instrument characterized by comprising the bio-ink cartridge according to any one of the first aspect, or comprising the bio-ink cartridge assembly according to any one of the second aspect.

A fifth aspect of the present disclosure relates to a microsphere preparation device comprising:
a microsphere preparation material storage device which comprises a shear liquid storage space for storing a shear liquid and a microsphere stock solution storage space for storing a microsphere stock solution for preparation of the microspheres;
a microfluidic chip which comprises a first chip inlet communicated with the microsphere stock solution storage space and a second chip inlet communicated with the shear liquid storage space, wherein the microsphere stock solution is sheared into microdroplets by the shear liquid in the microfluidic chip, and a microdroplet-shear liquid mixture is output from a chip outlet of the microfluidic chip; and
a curing device which comprises a curing device inlet connected with the chip outlet of the microfluidic chip to receive the microdroplet-shear liquid mixture, wherein the microdroplets form the cured microspheres in the curing device.

In some embodiments, the microsphere preparation device comprises a microsphere preparation material pumping device connected with the microsphere preparation material storage device to drive the microsphere stock solution and the shear liquid into the microfluidic chip, drive the shear liquid to shear the microsphere stock solution in the microfluidic chip to form the microdroplets, and drive the microdroplet-shear liquid mixture to flow out of the microfluidic chip; wherein the microsphere preparation material pumping device pumps a gas to the shear liquid storage space of the microsphere preparation material storage device and the microsphere stock solution storage space thereof respectively to enable the shear liquid to flow by changing pressure in the shear liquid storage space, and enable the microsphere stock solution to flow by changing pressure in the microsphere stock solution storage space; and gas on-off switches are arranged between the microsphere preparation material pumping device and the shear liquid storage space as well as between the microsphere preparation material pumping device and the microsphere stock solution storage space, respectively.

In some embodiments, the microsphere preparation material storage device comprises the bio-ink cartridge according to any one of the first aspect of the present disclosure, or comprises the bio-ink cartridge assembly according to any one of the second aspect; the first accommodating chamber of the bio-ink cartridge forms the shear liquid storage space; and the second accommodating chamber thereof forms the microsphere stock solution storage space.

In some embodiments, the microsphere preparation material storage device comprises at least two storage containers with a storage space formed thereinto respectively, and the storage space acts as the microsphere stock solution storage space or the shear liquid storage space.

In some embodiments, the microsphere preparation device comprises a buffer device which is arranged between the microfluidic chip and the curing device and comprises a buffer flow channel communicated with the chip outlet of the microfluidic chip and the curing device inlet of the curing device, and the buffer flow channel is configured to buffer or guide flow of the microdroplet-shear liquid mixture.

In some embodiments, the buffer device comprises a buffer body which comprises:
a mixture receiving port communicated with the chip outlet of the microfluidic chip to receive the microdroplet-shear liquid mixture;
a mixture output port communicated with the curing device inlet to output the microdroplet-shear liquid mixture from the buffer device; and
the buffer flow channel arranged between the mixture receiving port and the mixture output port; and
the buffer flow channel comprises a tapered segment with cross sectional area gradually decreasing from the mixture receiving port to the mixture output port.

In some embodiments, the tapered segment comprises an arc flow channel with cross sectional area decreasing from the mixture receiving port to the mixture output port.

In some embodiments, the buffer flow channel further comprises an equal cross-sectional segment located downstream of the tapered segment in the flow direction.

In some embodiments, the microfluidic chip comprises multiple chip outlets which are simultaneously communicated with the mixture receiving port.

In some embodiments, the buffer device comprises a third sealing structure through which the mixture receiving port is directly connected with the chip outlet of the microfluidic chip hermetically.

In some embodiments, the buffer device further comprises a shear liquid receiving port which receives the shear liquid made up to the buffer flow channel to maintain a level in the buffer flow channel equal to or above a level of the microdroplet-shear liquid mixture received at the mixture receiving port.

In some embodiments, the buffer body has a top with a top opening in communication with the buffer flow channel, the buffer device comprises a buffer cover body on the top opening, and the shear liquid receiving port is arranged on the buffer cover body.

In some embodiments, the curing device comprises a curing device outlet and a curing flow channel connecting the curing device inlet with the curing device outlet; the curing device inlet is communicated with the chip outlet of the microfluidic chip to receive the microdroplet-shear liquid mixture; the microdroplets form cured microspheres in a process that the microdroplet-shear liquid mixture flows through the curing flow channel; and a microsphere-shear liquid mixture is formed of the microspheres and the shear liquid and output from the curing device outlet.

In some embodiments, the curing flow channel is a helical flow channel coiled outwards or inwards.

In some embodiments, the curing flow channel comprises a tapered segment with a cross sectional area decreasing from the curing flow channel inlet to the curing flow channel outlet.

In some embodiments, the curing device comprises a coiled pipe in which the curing flow channel is formed.

In some embodiments, the curing device further comprises a disk body which is provided with a helical groove coiled outwards or inwards, and the coiled pipe is arranged in the helical groove.

In some embodiments, the curing device comprises:
a receiving container which is configured to accommodate a receiving liquid immiscible with the microdroplets, allow the microdroplets output from the chip outlet of the microfluidic chip to enter the receiving liquid, the microdroplets cured into the microspheres in the receiving liquid; and
an isolation control unit which is configured to separate the microdroplets apart from each other in the receiving liquid before the cured microspheres are formed.

In some embodiments, the isolation control unit separates the microdroplets apart from each other in the receiving liquid to form the microspheres by manipulating at least one of the receiving container, the receiving liquid, the chip outlet of the microfluidic chip, or an outlet of an output pipe line connected with the chip outlet of the microfluidic chip.

In some embodiments, the isolation control unit comprises at least one of following devices:
a container shifting device which is in driving connection with the receiving container for driving the receiving container to change position when the receiving liquid receives the microdroplets;
a fluid circulation driving device which is configured to drive the receiving liquid to circulate when the receiving liquid receives the microdroplets;
a bubbling device which is configured to generate bubbles in the receiving liquid when the receiving liquid receives the microdroplets; or
a stirring device which is configured to agitate the receiving liquid when the receiving liquid receives the microdroplets.

In some embodiments, the microsphere preparation device comprises the output pipe line connected with the chip outlet of the microfluidic chip, and the curing device further comprises an outlet position adjustment unit for adjusting position of an outlet of the output pipe line.

In some embodiments, the outlet position adjustment unit comprises a clamping device for clamping a tail end of the output pipe line, and the outlet position adjustment unit further comprises at least one of following devices:
a first position adjustment device which is configured to switch the clamping device between a first position and a second position above the first position; or
a second position adjustment device which is configured to vertically adjust height of the outlet of the output pipe line relative to the clamping device.

In some embodiments, when the receiving liquid receives the microdroplets, at least one of the chip outlet of the microfluidic chip or the outlet of the output pipe line connected with the chip outlet of the microfluidic chip are located below the receiving liquid level.

In some embodiments, the microsphere preparation device comprises a collection device located downstream of the curing device and configured to collect the microsphere-shear liquid mixture output therefrom.

In some embodiments, the microsphere preparation device comprises a collection device temperature control unit for keeping the collection device at a preset temperature or within a preset temperature range.

In some embodiments, the collection device comprises:
a mixture collection container which comprises a mixture receiving port and a shear liquid output port; the curing device is provided with the curing device outlet, and the mixture receiving port of the mixture collection container is communicated with the curing device outlet to receive the microsphere-shear liquid mixture; and
a shear liquid collection container which comprises a shear liquid receiving port communicated with the shear liquid output port of the mixture collection container to receive the shear liquid therefrom.

In some embodiments,
the microsphere preparation device comprises a buffer device which is arranged between the microfluidic chip and the curing device and comprises the buffer flow channel communicated with the chip outlet of the microfluidic chip and the curing device inlet of the curing device respectively, and the buffer flow channel is configured to buffer or guide the flow of the microdroplet-shear liquid mixture; and
the collecting device comprises a shear liquid circulating pump which is provided with an inlet communicated with the shear liquid collection container and an outlet communicated with the buffer flow channel; and the shear liquid circulating pump injects the shear liquid from the shear liquid collection container back to the buffer flow channel of the buffer device.

In some embodiments, the microsphere preparation device further comprises a monitoring device for monitoring preparation process of the microspheres, and the monitoring device comprises a position-adjustable camera.

In some embodiments, the microsphere preparation device further comprises a control device for controlling microsphere preparation process.

In some embodiments, the microsphere preparation device comprises a chip fixture for holding the microfluidic chip; the chip fixture comprises a base plate and a clamp plate for jointly clamping the microfluidic chip; the clamp plate comprises a connecting port communicated with the microfluidic chip and a sealing ring arranged corresponding to the connecting port; the sealing ring comprises a tubular segment and an annular segment connected orderly; the tubular segment is arranged in the corresponding connecting port; and the annular segment is arranged at a side of the connecting port towards the base plate.

In some embodiments, the microsphere preparation device comprises a microsphere preparation material temperature control unit for keeping the microsphere preparation material storage device and/or the microfluidic chip at a preset temperature or within a preset temperature range; and/or the microsphere preparation device comprises a curing device temperature control unit for keeping the curing device at a preset temperature or within a preset temperature range.

In some embodiments, the microsphere preparation device comprises a first preparation chamber and a second preparation chamber isolated from each other; at least one of the microsphere preparation material storage device or the microfluidic chip are arranged in the first preparation chamber, and the curing device is arranged in the second preparation chamber.

In some embodiments, the microsphere preparation device comprises the microsphere preparation material temperature control unit which adjusts the temperature of the first preparation chamber to keep at least one of the microsphere preparation material storage device or the microfluidic chip at the preset temperature or within the preset temperature range; and/or the curing device temperature control unit which adjusts the temperature of the second preparation chamber to keep the curing device at the preset temperature or within the preset temperature range.

In some embodiments, the microsphere preparation material temperature control unit comprises a cooling device for cooling the first preparation chamber.

A sixth aspect of the present disclosure provides a bio-block preparation instrument comprising the microsphere preparation device according to any one of the fifth aspect.

A seventh aspect of the present disclosure provides a shell assembly device for coating microspheres or compositional structures composed of a microsphere and at least one shell, comprising:
a shell preparation material storage device comprising multiple storage spaces which comprise a cleaning liquid storage space for storing a cleaning liquid and a shell liquid storage space for storing a shell liquid;
an assembling device in which the microspheres or the compositional structures are cleaned with the cleaning liquid, and then coated by the shell liquid to form a shell around each of the microspheres or the compositional structures.

In some embodiments, the shell assembly device comprises a shell preparation material pumping device which drives the cleaning liquid and/or the shell liquid to flow.

In some embodiments, the assembling device comprises a single assembling space or a partitioned assembling space in which the microspheres or the compositional structures are cleaned with the cleaning liquid, and then coated by the shell liquid to form a shell around each of the microspheres or the compositional structures.

In some embodiments, the assembling device comprises the assembling space in communication with the cleaning liquid storage space and the shell liquid storage space; and the shell assembly device comprises a fluid switching tube which comprises a tube body as well as multiple inlet connectors and an outlet connector arranged on and communicated with the tube body, the multiple inlet connectors comprise a cleaning liquid inlet connector connected with the cleaning liquid storage space and a shell liquid inlet connector connected with the shell liquid storage space, and the outlet connector is in communication with the assembling space of the assembling device.

In some embodiments, the multiple inlet connectors are arranged side-by-side on the tube body and located on a side of the shell preparation material storage device, and the multiple outlet connectors are arranged at an angle with the multiple inlet connectors and on a side of the tube body far away from the inlet connectors with an offset from the vertical direction.

In some embodiments, the assembling device comprises the assembling space in communication with the cleaning liquid storage space and the shell liquid storage space, and the shell assembly device further comprises a switching control device which selectively controls the assembling space to be communicated with one of the storage spaces and disconnect from the others.

In some embodiments, the switching control device comprises a rotating shaft and multiple control cams arranged on the rotating shaft in a non-rotatable way; the multiple control cams are arranged one-to-one corresponding to the multiple inlet connectors; and the rotating shaft rotates to change rotation angles of the multiple control cams to manipulate on-off state of the pipe lines communicating the assembling space with the storage spaces.

In some embodiments, the multiple control cams comprise at least two control cams having identical cam surface profile and mounted at different angles, or comprise at least two control cams having different cam surface profiles.

In some embodiments, the assembling space is connected with the corresponding storage space through a hose, and the switching control device further comprises multiple transmission units arranged one-to-one corresponding to the multiple inlet connectors; the transmission unit comprise a stopper and a moving member, the moving member is located between the stopper and the corresponding control cam; and the hose is located between the stopper and the moving member of the corresponding transmission unit, and manipulated to open or close by adjusting a gap between the moving member and the stopper through changing the angle of the control cam.

In some embodiments, the switching control device further comprises a limit sensing device which limits the rotation angle of the rotating shaft.

In some embodiments, the assembling device comprises an assembling container and a filter element, and the microspheres are subject to cleaning and shell assembly in the assembling space defined by the assembling container and the filter element.

In some embodiments, the assembling container comprises an assembling tube in which the assembling space is formed; a liquid receiving port is provided at an upper end of the assembling tube, and an opening is provided at a lower end thereof; and the filter element is arranged at the bottom of the opening.

In some embodiments, the assembling device further comprises a receiving seat which comprises a liquid receiving space, and the assembling tube and the filter element are arranged on the receiving seat and located above the liquid receiving space.

In some embodiments, the receiving seat further comprises a drainage flow channel in communication with the liquid receiving space; the shell assembly device further comprises a suction device and a liquid waste storage container; and the suction device is connected between the drainage flow channel and the liquid waste storage container to suck the liquid in the liquid receiving space to the liquid waste storage container.

In some embodiments, the filter element comprises a tube seat and a strainer, the tube seat is mounted on the receiving seat and has a central through hole, the strainer is mounted on the tube seat and located in the central through hole of the tube seat, the assembling tube is mounted in the tube seat and located above the strainer, and the opening of the assembling tube is faced to the strainer and the central through hole of the tube seat.

In some embodiments, the assembling device further comprises a gland which comprises a central through hole; the assembling tube is matched with the central through hole of the gland; and the gland covers the tube seat to fix the assembling tube and the filter element in the receiving seat.

In some embodiments, the shell assembly device further comprises an oscillation device which oscillates the cleaning liquid and/or the shell liquid when the microspheres or the compositional structures are cleaned and the shells are assembled.

In some embodiments, the assembling device comprises a sprayer in communication with the cleaning liquid storage space, and the sprayer is configured to spray the cleaning liquid to rinse the microspheres or the compositional structures in the assembling space.

In some embodiments, the assembling device comprises a filtrate container and a first accommodating screen which is located on the filtrate container to accommodate the microspheres or the compositional structures; the assembling space comprises an accommodating space of the first accommodating screen; the sprayer is arranged towards a screen opening on the first accommodating screen to rinse the microspheres or the compositional structures in the accommodating space of the first accommodating screen; and the filtrate container is configured to receive the cleaning liquid from the first accommodating screen.

In some embodiments, the assembling device comprises a first accommodating screen jacket which is arranged at an opening of the filtrate container, and the first accommodating screen is arranged on the first accommodating screen jacket.

In some embodiments, the assembling device comprises a seal cover and a suction device; the seal cover is located between the sprayer and the screen opening of the first accommodating screen, and peripherally covers the sprayer and the screen opening of the first accommodating screen; and the suction device is communicated with the filtrate container to suck a fluid in the filtrate container.

In some embodiments, the shell assembly material storage device comprises a shell liquid container having the shell liquid storage space; the assembling device comprises a second accommodating screen which is arranged in the shell liquid container; the assembling space comprises an accommodating space of the second accommodating screen; and the microspheres or the compositional structures in the accommodating space of the second accommodating screen is below the liquid level of the shell liquid container so as to be coated by the shell liquid to form a shell around each of the microspheres or the compositional structures.

In some embodiments, the assembling device comprises a second accommodating screen jacket which is at least partially arranged in the shell liquid container to form the shell, and the second accommodating screen is arranged on the second accommodating screen jacket.

In some embodiments, the shell assembly device comprises a separation device which is configured to separate the microspheres or the compositional structures from the liquid mixed therewith.

In some embodiments, the shell assembly device further comprises a control device for controlling shell assembly process.

In some embodiments, the assembling device comprises:
an assembling tube with an assembling space;
an assembling tube control unit comprising an assembling tube fixing portion and an assembling tube driving portion; wherein the assembling tube fixing portion is configured to fix the assembling tube, and the assembling tube driving portion is in driving connection with the assembling tube fixing portion to drive the assembling tube fixing portion and the assembling tube fixed thereby to rotate; and
a liquid adding/sucking unit which comprises a feeding needle and a feeding needle moving mechanism; wherein the feeding needle moving mechanism is in driving connection with the feeding needle to drive the feeding needle to move relative to the assembling tube fixing portion so as to change a relative position between the feeding needle and the assembling tube.

In some embodiments, the assembling tube control unit comprises a mounting base on which the assembling tube driving portion and the assembling tube fixing portion are arranged, and the assembling tube fixing portion is fixedly connected with an output end of the assembling tube driving portion.

In some embodiments, the assembling tube fixing portion comprises:
an assembling tube accommodating base which is fixedly connected with the output end of the assembling tube driving portion and has an assembling tube accommodating space for accommodating the assembling tube;
a limit stop mounting base which is arranged on the assembling tube accommodating base and has a mounting base threaded hole in communication with the assembling tube accommodating space;
a limit stop which is arranged in the limit stop mounting base and has a limit surface positioned towards the assembling tube accommodating space; and
a limit screw rod which is in thread fit with the mounting base threaded hole, wherein an end of the limit screw rod close to the assembling tube accommodating space is fixedly connected with the limit stop.

In some embodiments, the feeding needle moving mechanism comprises a feeding needle vertical driving mechanism configured to drive the feeding needle to move in a vertical direction; and the feeding needle vertical driving mechanism comprises:
a vertical guide rail assembly which comprises a guide rail and a translation portion vertically moving along the guide rail;
a driving device in driving connection with the translation portion and configured to drive the translation portion to vertically move along the guide rail; and
a moving arm which is fixedly connected with the translation portion of the vertical guide rail assembly, and the feeding needle is arranged on the moving arm.

In some embodiments, the liquid adding/sucking unit further comprises a limit mechanism which is configured to define movement range of the feeding needle in the vertical direction.

In some embodiments, the feeding needle moving mechanism further comprises a horizontal position adjustment mechanism for adjusting horizontal position of the feeding needle; and the feeding needle is connected with the moving arm through the horizontal position adjustment mechanism.

In some embodiments,
The liquid adding/sucking unit comprises a liquid level sensor which is configured to acquire a detection signal characterizing a stratified interface position of substances in the assembling tube; and
the shell assembly device comprises a control device which is in signal connection with the liquid level sensor and the feeding needle moving mechanism to operate the feeding needle moving mechanism to adjust the position of the feeding needle according to the detection signal from the liquid level sensor.

In some embodiments, the shell preparation material storage device comprises:
a storage box mounting base; and
a storage box which is arranged on the storage box mounting base and has the cleaning liquid storage space and the shell liquid storage space.

In some embodiments, the shell preparation material storage device comprises:
two storage box mounting rails mounted on both sides of the storage box mounting base side by side, wherein the storage box is mounted on the two storage box mounting rails;
a storage box cover which is movably arranged relative to the storage box, and covers above the storage box in a closure state and leaves above the storage box in an open state; and
a cover moving mechanism which is in driving connection with the storage box cover and configured to drive the storage box cover to move relative to the storage box so as to switch the storage box cover between the closure state and the open state.

In some embodiments, the assembling device comprises:
a centrifuge tube having an assembling space in which the microspheres or the compositional structures are rinsed by the cleaning liquid; and
a centrifugal unit which is configured to centrifuge substances in the centrifuge tube.

In some embodiments, the centrifugal unit comprises:
a centrifuge rack mounting portion;
a centrifuge rack which is rotatably mounted on the centrifuge rack mounting portion about a centrifugal axis and comprises a centrifuge tube bearing portion for mounting the centrifuge tube; wherein the centrifuge tube bearing portion is spaced apart from the centrifugal axis; and
a centrifugal motor which is mounted on the centrifuge rack mounting portion and in driving connection with the centrifuge rack to drive the centrifuge rack to rotate.

In some embodiments, the centrifuge rack comprises multiple centrifuge tube bearing portions of different specifications uniformly distributed about the centrifugal axis.

In some embodiments, the centrifuge tube bearing portion is rotatably arranged relative to the centrifuge rack about a rotating shaft perpendicular to the centrifugal axis.

In some embodiments, the assembling device further comprises a centrifuge tube storage unit which comprises a centrifuge tube mounting rack having a centrifuge tube storage portion for storing the centrifuge tube.

In some embodiments,
the centrifuge tube mounting rack further has a positioning member mounting hole in communication with the centrifuge tube storage portion;
the centrifuge tube storage unit comprises a centrifuge tube positioning member which is mounted in the positioning member mounting hole, and the centrifuge tube positioning member is configured to fix the centrifuge tube together with the centrifuge tube storage portion.

In some embodiments, the centrifuge tube storage unit comprises a liquid level sensor which is configured to detect a stratified interface of substances in the centrifuge tube.

In some embodiments, the centrifuge tube storage unit comprises a sensor mounting rack which has a centrifuge tube detection zone; the liquid level sensor is arranged on the sensor mounting rack, a detection end of the liquid level sensor is located in the centrifuge tube detection zone; a lower end of the centrifuge tube storage portion is located in the centrifuge tube detection zone; and at least one side of the centrifuge tube storage portion facing the detection end of the liquid level sensor is communicated with the centrifuge tube detection zone.

In some embodiments, the centrifuge tube storage unit comprises a sensor moving mechanism which is in driving connection with the sensor mounting rack for driving the liquid level sensor to change position.

In some embodiments, the assembling device further comprises a balance tube storage unit which comprises a balance tube and has a balance tube storage space for storing the balance tube; and the balance tube is configured to balance the centrifuge tube when the centrifugal unit centrifuges the substances in the centrifuge tube.

In some embodiments, the balance tube storage unit comprises two or more than two balance tubes in different specifications and two or more than two corresponding balance tube storage spaces.

In some embodiments, the assembling device comprises a liquid adding/sucking unit which is configured to add a liquid to or suck the liquid from the assembling space.

In some embodiments, the liquid adding/sucking unit comprises a pipette.

In some embodiments, the assembling device comprises a three-dimensional motion unit on which the liquid adding/sucking unit is mounted.

In some embodiments, the assembling device comprises a grabbing unit which is configured to operate the centrifuge tube.

In some embodiments, the grabbing unit comprises:
a manipulator mounting rack; and
a manipulator arranged on the manipulator mounting rack.

In some embodiments, the assembling device comprises a three-dimensional motion unit on which the grabbing unit is mounted.

In some embodiments, the assembling device comprises:
a three-dimensional motion unit;
a liquid adding/sucking unit which is configured to add a liquid to or suck the liquid from the assembling space, and mounted on the three-dimensional motion unit; and
a grabbing unit which is configured to operate the centrifuge tube and mounted on the three-dimensional motion unit.

In some embodiments, the three-dimensional motion unit comprises:
a first driving component on which the grabbing unit is arranged, and configured to drive the grabbing unit to move in a vertical direction;
a second driving component 5131 on which the first driving component is arranged, and configured to drive the first driving component to move in a second horizontal direction;
a third driving component on which the second driving component is arranged, and configured to drive the second driving component to move in a first horizontal direction perpendicular to the second horizontal direction;
a fourth driving component on which the liquid adding/sucking unit is arranged, and configured to drive the liquid adding/sucking unit to move in the vertical direction; and
a fifth driving component arranged on the third driving component, wherein the third driving component is further configured to drive the fifth driving component to move in the first horizontal direction; the fourth driving component is arranged on the fifth driving component; and the fifth driving component is configured to drive the fourth driving component to move in the second horizontal direction.

In some embodiments, the assembling device comprises a residual liquid absorbing unit.

In some embodiments, the residual liquid absorbing unit comprises a residual liquid absorbing mounting rack and an oil-absorbing sponge arranged thereon.

In some embodiments, the shell assembly device comprises a liquid waste collection unit.

In some embodiments, the assembling device comprises a tip storage unit.

An eighth aspect of the present disclosure provides a bio-block preparation instrument comprising the shell assembly device according to any one of the seventh aspect.

A ninth aspect of the present disclosure provides a bio-block preparation instrument which comprises the microsphere preparation device according to any one of the fifth aspect and the shell assembly device according to any one of the seventh aspect, and the microsphere preparation device and the shell assembly device are arranged integrally or separately.

A tenth aspect of the present disclosure provides a bio-ink preparation instrument comprising:
a shell assembly device according to any one of the seventh aspect; and
a bio-ink generation device which is configured to mix the bio-block formed by the shell assembly device with a carrier material to form the bio-ink.

In some embodiments, the bio-ink generation device comprises:
a barrel having a mixing space for mixing the bio-block and the carrier material;
and a carrier material loading unit for injecting the carrier material into the mixing space.

In some embodiments, the assembling device of the shell assembly device comprises the liquid adding/sucking unit for adding the liquid to or sucking the liquid from the assembling space, and the carrier material loading unit is mounted on the liquid adding/sucking unit.

In some embodiments, the carrier material loading unit comprises a syringe.

In some embodiments, the carrier material loading unit comprises a syringe pushing portion in driving connection with the syringe and configured to push a piston of the syringe to move.

In some embodiments, the syringe pushing portion comprises:
a slider movably arranged along an axis of the syringe for pushing the piston of the syringe; and
a syringe driving motor in driving connection with the slider and configured to drive the slider to move to push the piston of the syringe.

In some embodiments, the carrier material loading unit comprises a syringe thermal insulation device arranged on the surface of the syringe for thermal insulation thereof.

In some embodiments, the bio-ink generation device comprises a weighing unit.

In some embodiments, the weighing unit comprises an electronic scale and a weighing tray arranged thereon, and the weighing tray comprises a hollow cylinder structure for storing the syringe.

In some embodiments, the bio-ink generation device comprises a scraping unit which comprises a correction mounting rack as well as a scraping plate, a scraping line and a distance measuring sensor arranged thereon.

In some embodiments, the scraping unit comprises two distance measuring sensors arranged oppositely.

In some embodiments, the bio-ink generation device comprises a barrel thermal insulation unit for thermal insulation of the barrel.

In some embodiments, the barrel thermal insulation unit comprises a thermal insulation box and a heating device for heating the thermal insulation box.

In some embodiments, the bio-ink generation device comprises a mixing unit for mixing the bio-block and the carrier material in the barrel.

In some embodiments, the mixing unit comprises:
a mixing unit mounting rack;
a rotary motor mounted on the mixing unit mounting rack; and
a swing arm mounted on an output shaft of the rotary motor and having a barrel bearing portion for mounting the barrel.

In some embodiments, the mixing unit comprises an angular sensor mounted on the output shaft of the rotary motor for detecting a rotating position of the rotary motor.

In some embodiments, the mixing unit comprises a buffer assembly mounted on the mixing unit mounting rack for buffering and limiting the swing arm.

In some embodiments, two buffer assemblies are respectively located at two ends of a swing path of the swing arm, and each of the buffer assemblies comprises:
a buffer post mounted on the mixing unit mounting rack;
and a buffer cushion arranged on the buffer post and located between the buffer post and a corresponding end of the swing path of the swing arm.

In some embodiments, the barrel bearing portion comprises a barrel mounting hole and a fixing piece mounting portion;
The mixing unit comprises a fixing piece, a torsion spring and an pneumatic cylinder; the pneumatic cylinder is mounted on the mixing unit mounting rack; the fixing piece comprises a pressing end, a driving end and a hinge portion therebetween; the hinge portion is hinged to the fixing piece mounting portion through the torsion spring; the driving end is matched with the pneumatic cylinder; and the pneumatic cylinder drives the fixing piece to rotate about the fixing piece mounting portion so as to switch the pressing end between a pressing state that the pressing end is adjacent to an inlet of the barrel mounting hole and a relief state that the pressing end is away from the inlet of the barrel mounting hole.

An eleventh aspect of the present disclosure provides a bio-ink preparation system comprising the bio-ink preparation instrument according to any one of the tenth aspect.

In some embodiments, the bio-ink preparation system comprises the microsphere preparation device according to any one of the fifth aspect.

According to the bio-ink cartridge and the bio-ink cartridge assembly of the present disclosure, the arrangement of the inner flow channel is beneficial to preventing a cross of the external gas inlet pipe line and the liquid discharge pipe line, and facilitates quick and accurate connection between the bio-ink cartridge and the connected components. The bio-ink cartridge is used as the microsphere preparation material storage device of the microsphere preparation device, which is beneficial to improving microsphere production efficiency.

According to the microsphere preparation device of the present disclosure, the liquid microdroplets are generated from a microsphere preparation raw material by the microfluidic chip and cured in the curing device to form the microspheres for producing microsphere products, which is beneficial to improving the microsphere preparation efficiency.

According to the shell assembly device of the present disclosure, each of the formed microspheres is externally and directly assembled with at least one shell through the assembling device to produce bio-block products meeting the requirements for biological activity and bio-printing, which is beneficial to improving efficiencies of shell assembly and bio-block preparation.

According to the bio-block preparation instrument of the present disclosure, the liquid microdroplets are generated from the microsphere preparation raw material by the microfluidic chip and cured in the curing device to form the microspheres for producing the microsphere products; and each of the formed microspheres is externally and directly assembled with at least one shell through the assembling device to produce the bio-block products meeting the requirements for biological activity and bio-printing, which is beneficial to improving the bio-block preparation efficiency. According to the bio-ink preparation instrument and the bio-ink preparation system of the present disclosure, it is possible to generate the bio-ink meeting the requirements for biological activity and bio-printing.

Other features and advantages of the present disclosure will become clear from the following detailed description of exemplary embodiments in conjunction with the accompanying drawings.

### Brief Description of the Drawings

The drawings, which constitute part of the present disclosure, are intended to facilitate further understanding of the present disclosure. The exemplary embodiments of the present disclosure and description thereof are used to explain the present disclosure, but not improperly limit thereto. In the drawings:
Figure 1 is a perspective view of a microsphere preparation device in a bio-block preparation instrument according to an embodiment of the present disclosure.
Figure 2 is a top plan view of partial structure of the microsphere preparation device as shown in Figure 1.
Figure 3 is a structural view of a partial sectional view of the microsphere preparation device as shown in Figure 1.
Figure 4 is a structural diagram of a bio-ink cartridge assembly according to an embodiment of the present disclosure.
Figure 5 is a cross sectional view of a bio-ink cartridge in the bio-ink cartridge assembly as shown in Figure 4.
Figure 6 is a schematic diagram of the bio-ink cartridge as shown in Figure 5.
Figure 7 is a schematic diagram of a bio-ink cartridge according to a comparative example.
Figure 8 is a perspective view of an integrated connector of a bio-ink cartridge assembly according to an embodiment of the present disclosure.
Figure 9 is a cross sectional view of the integrated connector as shown in Figure 8.
Figure 10 is a structural diagram of an outlet integration head of a bio-ink cartridge assembly according to an embodiment of the present disclosure.
Figure 11 is a cross sectional view of the outlet integration head as shown in Figure 10.
Figure 12 is a schematic diagram of a microfluidic chip of a microsphere preparation device according to an embodiment of the present disclosure.
Figure 13 is a perspective view of a buffer body in a buffer device of a microsphere preparation device according to an embodiment of the present disclosure from an angle.
Figure 14 is a perspective view of the buffer body in the buffer device as shown in Figure 13 from another angle.
Figure 15 is a perspective view of a compositional structure of a buffer device and a curing device in a microsphere preparation device according to an embodiment of the present disclosure.
Figure 16 is a cross sectional view of the compositional structure of the buffer device and the curing device as shown in Figure 15.
Figure 17 is a structural diagram of a collection device of a microsphere preparation device according to an embodiment of the present disclosure.
Figure 18 is a cross sectional view of partial structure of a monitoring device of a microsphere preparation device according to an embodiment of the present disclosure.
Figure 19 is a perspective view of the partial structure of the monitoring device as shown in Figure 18 from an angle.
Figure 20 is a perspective view of the partial structure of the monitoring device as shown in Figure 18 from another angle.
Figure 21 is a schematic diagram of preparation of microspheres through the microsphere preparation device according to the embodiment as shown in Figure 1.
Figure 22 is a perspective view of a shell assembly device in a bio-block preparation instrument according to an embodiment of the present disclosure.
Figure 23 is a perspective view of a shell assembly device in another bio-block preparation instrument according to an embodiment of the present disclosure.
Figure 24 is a cross sectional view of partial structure of the shell assembly device as shown in Figure 23.
Figure 25 is a perspective view of a fluid switching tube in the shell assembly device as shown in Figure 22.
Figure 26 is a perspective view of a switching control device in the shell assembly device as shown in Figure 22.
Figure 27 is a perspective view of partial structure of the switching control device as shown in Figure 26.
Figure 28 is a cross sectional view of partial structure of the shell assembly device as shown in Figure 22.
Figure 29 is a front sectional view of the partial structure of the shell assembly device as shown in Figure 22.
Figure 30 is a cross sectional view of an assembling device in the shell assembly device as shown in Figure 22.
Figure 31 is a perspective view of a mounting base of the assembling device as shown in Figure 30.
Figure 32 is a schematic diagram of shell assembly through the shell assembly device according to the embodiment as shown in Figure 22.
Figure 33 is a perspective view of a bio-block preparation instrument according to another embodiment of the present disclosure.
Figure 34 is a structural diagram of partial structure of the bio-block preparation instrument comprising a microsphere preparation material storage device and a microfluidic chip according to the embodiment as shown in Figure 33.
Figure 35 is a structural diagram of the partial structure of the bio-block preparation instrument comprising the microfluidic chip and a curing device according to the embodiment as shown in Figure 33.
Figure 36 is a structural diagram of a chip fixture and the microfluidic chip of the bio-block preparation instrument as shown in Figure 33.
Figure 37 is a top plan view of the chip fixture and the microfluidic chip as shown in Figure 36.
Figure 38 is a sectional view of partial structure of the chip fixture as shown in Figure 36.
Figure 39 is a structural diagram of a preparation case of the bio-block preparation instrument as shown in Figure 33.
Figure 40 is a structural diagram of the preparation case as shown in Figure 39, with a first cover body closed and a second cover body open.
Figure 41 is a structural diagram of a connection structure between an output pipe line and the curing device of the bio-block preparation instrument as shown in Figure 33.
Figure 42 is an view of partial structure of the output pipe line and the curing device of the bio-block preparation instrument as shown in Figure 33.
Figure 43 is a perspective view of partial structure of the output pipe line and the curing device of the bio-block preparation instrument as shown in Figure 33.
Figure 44 is a structural diagram of a shell assembly device of the bio-block preparation instrument as shown in Figure 33.
Figure 45 is an assembly diagram of a first accommodating screen and a first accommodating screen jacket of a flush module of the bio-block preparation instrument as shown in Figure 33.
Figure 46 is a perspective view of a shell assembly device in another bio-block preparation instrument according to an embodiment of the present disclosure.
Figure 47 is a view of the shell assembly device as shown in Figure 46.
Figure 48 is a perspective view of an assembling tube control unit in the shell assembly device as shown in Figure 46.
Figure 49 is a perspective view of a liquid adding/sucking unit in the shell assembly device as shown in Figure 46.
Figure 50 is a perspective view of a compositional structure of a feeding needle, a needle clamping portion, a connecting member and a horizontal position adjustment mechanism of the liquid adding/sucking unit in the shell assembly device as shown in Figure 46.
Figure 51 is a structural diagram of a bio-ink preparation instrument according to an embodiment of the present disclosure.
Figure 52 is a structural diagram of the bio-ink preparation instrument as shown in Figure 51 with a housing removed.
Figure 53 is a structural diagram of the structure as shown in Figure 52 from another angle.
Figure 54 is a structural diagram of the structure as shown in Figure 52 from another angle.
Figure 55 is a structural diagram of a shell preparation material storage device with a storage box cover removed in the bio-ink preparation instrument as shown in Figure 51.
Figure 56 is a structural diagram of the shell preparation material storage device in the bio-ink preparation instrument as shown in Figure 51.
Figure 57 is a structural diagram of a centrifugal unit in the bio-ink preparation instrument as shown in Figure 51.
Figure 58 is a structural diagram of a centrifuge tube storage unit in the bio-ink preparation instrument as shown in Figure 51.
Figure 59 is a structural diagram of a centrifuge tube storage unit in the bio-ink preparation instrument as shown in Figure 51.
Figure 60 is a structural diagram of a balance tube storage unit in the bio-ink preparation instrument as shown in Figure 51.
Figure 61 is a structural diagram of a compositional structure of a liquid adding/sucking unit and a carrier material loading unit in the bio-ink preparation instrument as shown in Figure 51.
Figure 62 is a structural diagram of a grabbing unit in the bio-ink preparation instrument as shown in Figure 51.
Figure 63 is a structural diagram of a three-dimensional motion unit in the bio-ink preparation instrument as shown in Figure 51.
Figure 64 is a structural diagram of a weighing unit in the bio-ink preparation instrument as shown in Figure 51.
Figure 65 is a structural diagram of a scraping unit in the bio-ink preparation instrument as shown in Figure 51.
Figure 66 is a structural diagram of a mixing unit in the bio-ink preparation instrument as shown in Figure 51.
Figure 67 is a structural diagram of a bio-ink preparation system according to an embodiment of the present disclosure.

### Detailed Description of Embodiments

The technical solutions in the embodiments of the present disclosure will be described below clearly and completely with reference to drawings in the embodiments of the present disclosure. Obviously, the embodiments described are only merely part of embodiments of the present disclosure, but not all of the embodiments thereof. The following description of at least one exemplary embodiment is in fact illustrative only and is in no way intended to limit the present disclosure and application or use thereof. All other embodiments obtained by those of ordinary skill in the art based on the embodiments of the present disclosure without making creative efforts fall within the protection scope of the present disclosure.

Relative arrangement of components and steps, numerical expressions, and numerical values set forth in these embodiments, unless specifically stated otherwise, should not be construed as a limitation to the present disclosure. It should be understood that the dimensions of the various portions shown in the accompanying drawings are for descriptive purposes and are not necessarily drawn according to the actual scale. Techniques, methods, and apparatuses known to those of ordinary skill in the relevant art may not be discussed in detail, but where appropriate, these techniques, methods, and apparatuses should be considered as part of this specification. In all the examples shown and discussed herein, any specific value should be construed as merely exemplary rather than a restriction. Therefore, other examples of the exemplary embodiments may have different values. It should be noted that similar marks and letters represent similar items in the accompanying drawings below, and therefore, any item already defined in one accompanying drawing is unnecessary to be further discussed in the subsequent drawings.

The use of the wordings "first", "second" and the like when describing the present disclosure are merely used to distinguish between different components and have no special meaning unless otherwise stated, therefore cannot be construed as limitation to the protection scope of the present disclosure.

It should be noted that nouns of locality used to describe the present disclosure, such as "front, rear, top, bottom, left and right", "transverse, vertical, perpendicular and horizontal" and "top and bottom", usually indicate the orientation or positional relationship shown in the accompanying drawings for the convenience of describing the present disclosure and simplifying the description only and, in the absence of a statement to the contrary, do not indicate or imply that the device or element in question must have a particular orientation or be constructed and operated in a particular orientation, and therefore cannot be understood as a limitation to the protection scope of the present disclosure. Nouns of locality "inner" and "outer" refer to the inside and outside of the outline of each component.

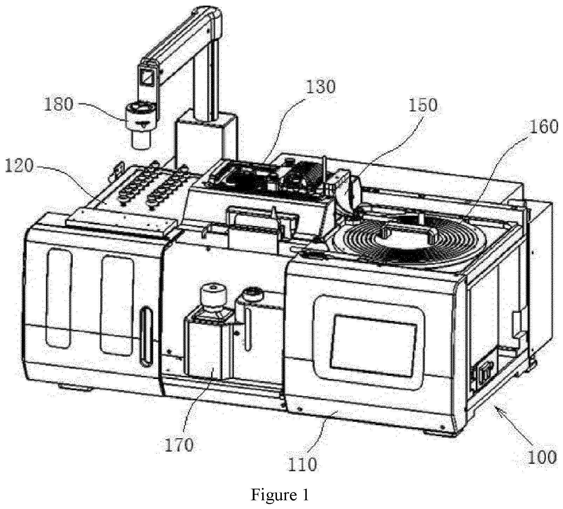

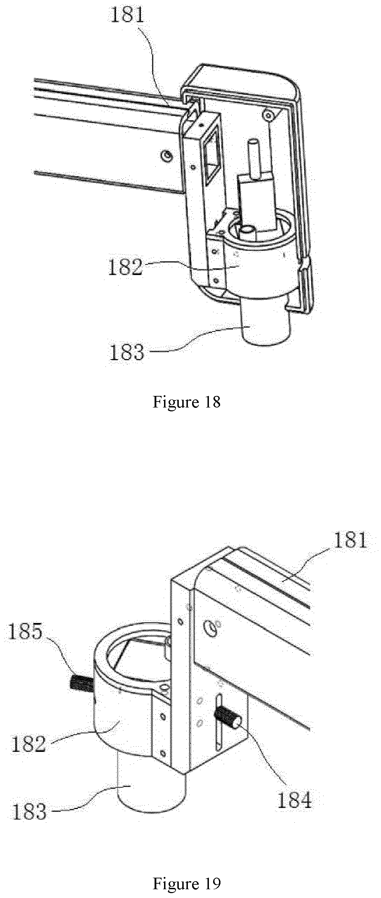

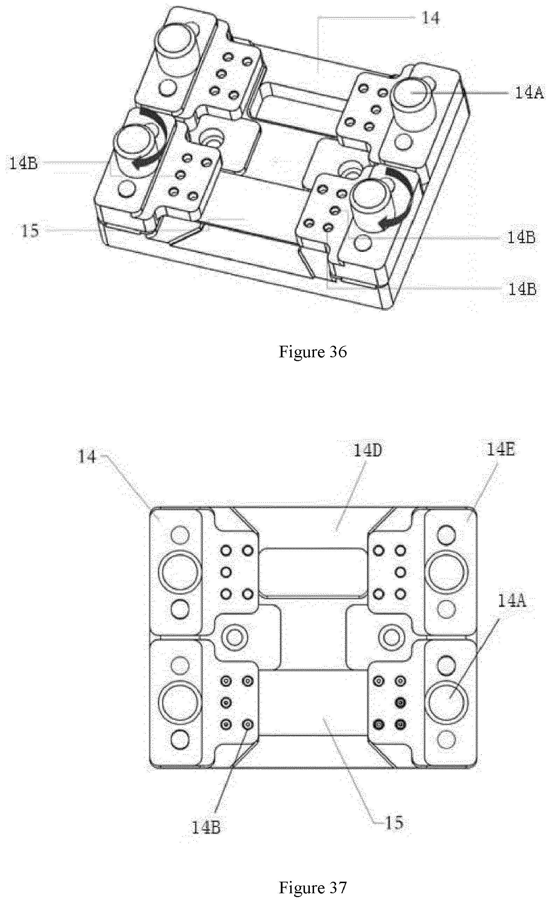

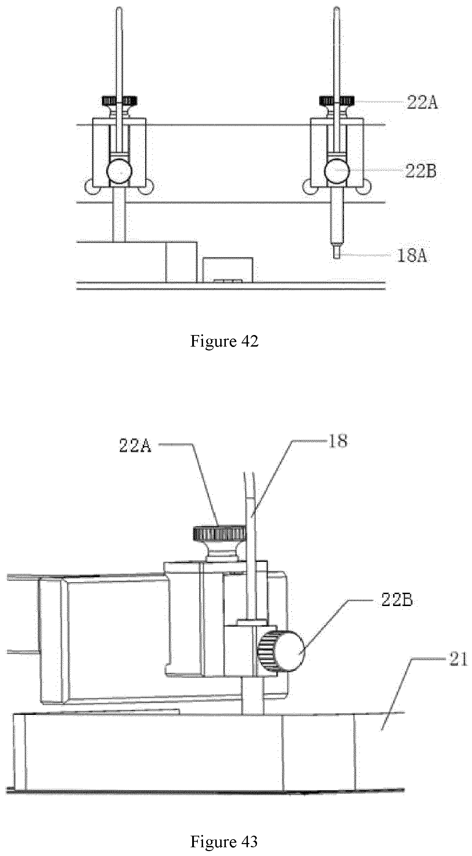

As shown in Figures 1 to 45, the embodiments of the present disclosure provide a bio-ink cartridge, a bio-ink cartridge assembly, a microsphere preparation device, a shell assembly device, a bio-block preparation instrument, a bio-ink preparation instrument and a bio-ink preparation system. Further, the embodiments of the present disclosure further provide a switching control device for switching on or off multiple pipe lines or pipe fittings.

As shown in Figures 1 to 21, the embodiments of the present disclosure provide a bio-ink cartridge 131. The bio-ink cartridge 131 comprises an ink cartridge shell 1311 with a cavity; a dividing wall 1312 arranged in the cavity and dividing the cavity into a first accommodating chamber 1313 and a second accommodating chamber 1314 which are fluidly isolated from each other; a first ink cartridge inlet 13151 and a first ink cartridge outlet 13153 arranged on the ink cartridge shell 1311 and communicated with the first accommodating chamber 1313; a second ink cartridge inlet 13152 and a second ink cartridge outlet 13154 arranged on the ink cartridge shell 1311 and communicated with the second accommodating chamber 1314; and an inner flow channel 1316 located in the second accommodating chamber 1314 and isolated from a fluid therein, and communicating the first accommodating chamber 1313 with the first ink cartridge outlet 13153.

As shown in Figures 1 to 21, the embodiments of the present disclosure further provide a bio-ink cartridge assembly comprising the bio-ink cartridge according to the embodiments of the present disclosure.

According to the bio-ink cartridge and the bio-ink cartridge assembly of the present disclosure, the inner flow channel is arranged in such a way that a cross of the external gas inlet pipe line and the liquid discharge pipe line is prevented, which facilitates quick and accurate connection between the bio-ink cartridge and the connected components. The bio-ink cartridge is used as the microsphere preparation material storage device of the microsphere preparation device, which is beneficial to improving microsphere production efficiency.

As shown in Figures 1 to 21, the embodiments of the present disclosure provide a microsphere preparation device and a bio-block preparation instrument which comprise the bio-ink cartridge or the bio-ink cartridge assembly according to the embodiments of the present disclosure. The microsphere preparation device and the bio-block preparation instrument have advantages of the bio-ink cartridge or the bio-ink cartridge assembly according to the embodiments of the present disclosure.

As shown in Figures 1 to 45, the microsphere preparation device of the embodiments of the present disclosure comprises: a microsphere preparation material storage device which comprises a shear liquid storage space for storing the shear liquid and a microsphere stock solution storage space for storing a microsphere stock solution for preparation of the microspheres; a microfluidic chip which comprises a first chip inlet communicated with the microsphere stock solution storage space and a second chip inlet communicated with the shear liquid storage space, wherein the microsphere stock solution is sheared into microdroplets by the shear liquid in the microfluidic chip, and a microdroplet-shear liquid mixture is output from a chip outlet of the microfluidic chip; a curing device which comprises a curing device inlet connected with the chip outlet of the microfluidic chip to receive the microdroplet-shear liquid mixture, wherein the microdroplets form the cured microspheres in the curing device.

According to the microsphere preparation device of the present disclosure, the liquid microdroplets are generated from a microsphere preparation raw material by the microfluidic chip and cured in the curing device to form the microspheres for producing microsphere products, which is beneficial to improving the microsphere preparation efficiency.

As shown in Figures 1 to 45, the embodiments of the present disclosure provide a bio-block preparation instrument which comprises the microsphere preparation device according to the embodiments of the present disclosure. The bio-block preparation instrument has advantages of the microsphere preparation device according to the embodiments of the present disclosure.

As shown in Figures 1 to 45, a shell assembly device of the embodiments of the present disclosure is configured to coat the microspheres or compositional structures composed of a microsphere and at least one shell, and comprises: a shell preparation material storage device which comprises multiple storage spaces comprising a cleaning liquid storage space for storing a cleaning liquid and a shell liquid storage space for storing a shell liquid; and an assembling device in which the microspheres or the compositional structures composed of a microsphere and at least one shell are rinsed with the cleaning liquid, and then coated by the shell liquid to form a shell around each of the microspheres or the compositional structures.

According to the shell assembly device of the present disclosure, each of the formed microspheres is externally and directly assembled with at least one shell through the assembling device to produce bio-block products meeting the requirements for biological activity and bio-printing, which is beneficial to improving efficiencies of shell assembly and bio-block preparation.

As shown in Figures 1 to 45, the embodiments of the present disclosure further provide a bio-block preparation instrument which comprises the shell assembly device according to the embodiments of the present disclosure. The bio-block preparation instrument has advantages of the shell assembly device according to the embodiments of the present disclosure.

As shown in Figures 1 to 45, the embodiments of the present disclosure further provide a bio-block preparation instrument which comprises the microsphere preparation device and the shell assembly device according to the embodiments of the present disclosure; and the microsphere preparation device and the shell assembly device are arranged integrally or separately.

According to the bio-block preparation instrument of the present disclosure, the liquid microdroplets are generated from the microsphere preparation raw material by the microfluidic chip and cured in the curing device to form the microspheres for producing the microsphere products; and each of the formed microspheres is externally and directly assembled with at least one shell through the assembling device to produce the bio-block products meeting the requirements for biological activity and bio-printing, which is beneficial to improving the bio-block preparation efficiency.

The embodiments of the present disclosure are described in detail in combination with Figure 1 to 45 below.

As shown in Figures 1 to 33, according to an embodiment of the present disclosure, the bio-block preparation instrument comprises a microsphere preparation device 100 and a shell assembly device 200. The microsphere preparation device 100 and the shell assembly device 200 are arranged independently.

As shown in Figures 1 to 21, the microsphere preparation device 100 comprises a microsphere preparation rack 110, a microsphere preparation material pumping device 120, a microsphere preparation material storage device, a microfluidic chip 140, a buffer device 150, a curing device 160, a collection device 170 and a monitoring device 180.

The microsphere preparation rack 110 provides support for components of the microsphere preparation device 100, integrates the components as a whole, and defines relatively functional areas of the components to simplify related operations (e.g. temperature control). As shown in Figures 1 to 3, the microsphere preparation material pumping device 120, the microsphere preparation material storage device, the microfluidic chip 140, the buffer device 150, the curing device 160, the collection device 170 and the monitoring device 180 are mounted on the microsphere preparation rack 110.

The microsphere preparation material storage device comprises a shear liquid storage space and a microsphere stock solution storage space. The shear liquid storage space is used for storing a shear liquid. The microsphere stock solution storage space is used for storing a microsphere stock solution for preparation of the microspheres.

The microsphere preparation material pumping device 120 is connected with the microsphere preparation material storage device to drive the microsphere stock solution and the shear liquid into the microfluidic chip 140, drive the shear liquid to shear the microsphere stock solution into microdroplets in the microfluidic chip 140, and drive a microdroplet-shear liquid mixture to flow out of the microfluidic chip 140.

According to some embodiments, the microsphere preparation material pumping device 120 pumps a gas to the shear liquid storage space of the microsphere preparation material storage device and the microsphere stock solution storage space thereof respectively to enable the shear liquid to flow by changing pressure in the shear liquid storage space, and enable the microsphere stock solution to flow by changing pressure in the microsphere stock solution storage space.

The microsphere preparation material pumping device 120 comprises multiple pressure control portions which are respectively connected with a corresponding storage space of the microsphere preparation material storage device through a connecting tube.

In the embodiment, the microsphere preparation material pumping device is a pneumatic device for the entire microsphere preparation device. The microsphere preparation material pumping device 120 has 16 pressure control portions, each of which is connected with a storage space through a connecting tube, and offers power to the microsphere stock solution or the shear liquid for flow and controls flow rate thereof by changing gas pressure in the storage space.

In some embodiments, the pressure control portions are independent of each other and may be partially enabled according to different requirements; and operating parameters of each of the pressure control portions can be independently controlled to adjust pressure value of the storage space connected therewith, and further regulate the flow rate of material stored in the corresponding storage space. By regulating the flow rates of the microsphere stock solution and the shear liquid, parameters of microdroplets (e.g., size and generation speed) may be controlled.

In this embodiment, the microsphere preparation material pumping device 120 comprises a pressure pump capable of accurately controlling the pump pressure. The pressure pump, for example, is a 2bar micro-fluid pressure pump with 16 channels and feeding in a pressure-driven manner. Each of the channels of the pressure pump is connected with a corresponding storage space of the microsphere preparation material storage device to deliver a gas to the corresponding storage space, so that the microsphere stock solution or the shear liquid is pressed via a gas.

The pressure pump is in a pressure-driven feeding mode, and a gas inlet and a liquid outlet are arranged in the storage space storing the microsphere stock solution or the shear liquid. The gas inlet is connected with a pump outlet of the pressure pump, and the liquid outlet is connected with the microfluidic chip through a connecting pipe line. The pressure pump realizes passive feeding without generation of impulse impact or contact with the microsphere stock solution, thus reducing damage to cells in the microsphere stock solution. With the use of the pressure pump capable of accurately controlling the pump pressure, the flow rates of the microsphere preparation materials can be regulated in an improved way, and a continuous phase fluid and a dispersed phase fluid in the microfluidic chip can be controlled in a relatively accurate manner, thus appropriately adjusting shear force and reducing the damage to the cells. In addition, it is also beneficial to guaranteeing quality, activity and size of the formed microdroplets and microspheres.

The microsphere stock solution forming the microdroplets is used as a dispersed phase fluid in the microfluidic chip 140, and the microsphere stock solution is an ingredient of the microdroplet. The shear liquid for preparing the microdroplets is used as a continuous phase fluid in the microfluidic chip 140, and the shear liquid is not an ingredient of the microdroplets. In the embodiment, the microsphere stock solution is a substance with biological activity (e.g., a collagen solution containing cells); and the shear liquid is an oil phase liquid immiscible with the collagen solution.

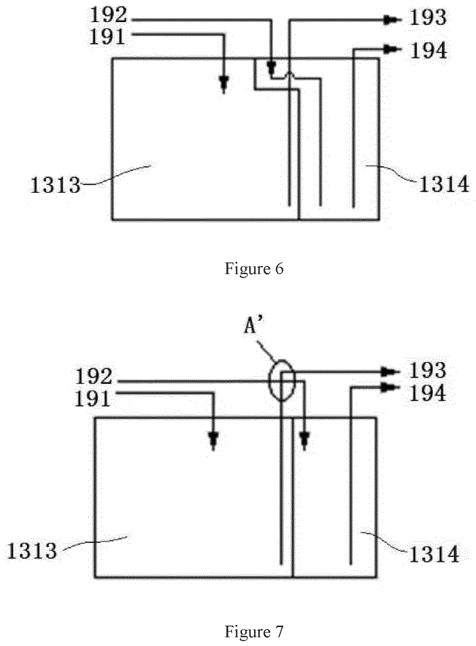

In some embodiments, gas on-off switches are arranged between the microsphere preparation material pumping device 120 and the shear liquid storage space as well as between the microsphere preparation material pumping device 120 and the microsphere stock solution storage space, respectively. For example, in the embodiment, as shown in Figure 1 and Figure 6, the microsphere preparation material pumping device 120 is connected with a first accommodating chamber 1313 of the bio-ink cartridge 131 which serves as the shear liquid storage space through a first pipe line 191, and a gas on-off switch can be arranged on the first pipe line 191 to control on-off state of the gas delivered to the first accommodating chamber 1313. The microsphere preparation material pumping device 120 is connected with a second accommodating chamber 1314 of the bio-ink cartridge 131 which serves as the microsphere stock solution storage space through a second pipe line 192, and a gas on-off switch can be arranged on the second pipe line 192 to control on-off state of the gas delivered to the second accommodating chamber 1314.

In the embodiment, the microsphere preparation material storage device comprises the bio-ink cartridge 131 or a bio-ink cartridge assembly 130.

As shown in Figures 1 to 11, the bio-ink cartridge 131 essentially comprises an ink cartridge shell 1311, a dividing wall 1312, a first ink cartridge inlet 13151, a first ink cartridge outlet 13153, a second ink cartridge inlet 13152, and a second ink cartridge outlet 13154.

The dividing wall 1312 is arranged in a cavity inside the ink cartridge shell 1311 and divides the cavity into a first accommodating chamber 1313 and a second accommodating chamber 1314 independently. The first ink cartridge inlet 13151 and the first ink cartridge outlet 13153 are arranged on the ink cartridge shell 1311 and communicated with the first accommodating chamber 1313. The second ink cartridge inlet 13152 and the second ink cartridge outlet 13154 are arranged on the ink cartridge shell 1311 and communicated with the second accommodating chamber 1314.

In the embodiment, the first accommodating chamber 1313 of the bio-ink cartridge 131 forms a shear liquid storage space, and the second accommodating chamber 1314 of the bio-ink cartridge 131 forms a microsphere stock solution storage space.

The pressure control portions of the microsphere preparation material pumping device are respectively communicated with the first ink cartridge inlet 13151 and the second ink cartridge inlet 13152 of the bio-ink cartridge 131. A second chip inlet 1421 of the microfluidic chip 140 is communicated with the first ink cartridge outlet 13153. A first chip inlet 1411 of the microfluidic chip 140 is communicated with the second ink cartridge outlet 13154. Therefore, the microsphere preparation material pumping device is capable of pumping the shear liquid in the first accommodating chamber 1313 and the microsphere stock solution in the second accommodating chamber 1314 of the bio-ink cartridge 131 into the microfluidic chip 140 to form microdroplets.

As shown in Figures 1 to 7, the bio-ink cartridge 131 of the embodiment further comprises an inner flow channel 1316. The inner flow channel 1316 is located in the second accommodating chamber 1314 and communicates the first accommodating chamber 1313 with the first ink cartridge outlet 13153.

As shown in Figure 6, the inner flow channel 1316 is located in the second accommodating chamber 1314 without communication with the second accommodating chamber 1314, and a tunnel-like structural design is generated in the cavity of the bio-ink cartridge 131. The first pipe line 191 is connected with the first ink cartridge inlet 13151 of the bio-ink cartridge 131, the second pipe line 192 is connected with the second ink cartridge inlet 13152 of the bio-ink cartridge 131, a third pipe line 193 is connected with the first ink cartridge outlet 13153 of the bio-ink cartridge 131, and a fourth pipe line 194 is connected with the second ink cartridge outlet 13154 of the bio-ink cartridge 131.

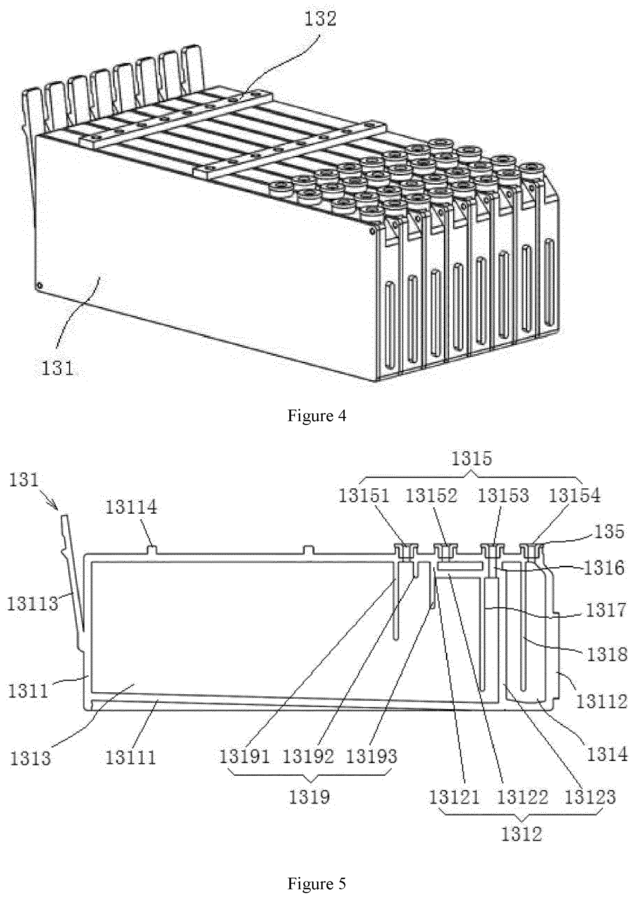

As shown in Figure 6, the first ink cartridge inlet 13151, the second ink cartridge inlet 13152, the first ink cartridge outlet 13153, and the second ink cartridge outlet 13154 are orderly arranged in the longitudinal direction (the left-right direction as shown in Figure 5) due to the arrangement of the inner flow channel 1316. In this case, the first pipe line 191 and the second pipe line 192 are arranged on the left of the bio-ink cartridge 131, and the third pipe line 193 and the fourth pipe line 194 are arranged on the right side of the bio-ink cartridge 131. A gas supply pipe line does not cross with a liquid discharge pipe line outside the bio-ink cartridge 131. Therefore, the bio-ink cartridge 131 of the embodiment is quickly and accurately connected with the corresponding microsphere preparation material pumping device and the microfluidic chip 140 through the ink cartridge inlets and the ink cartridge outlets.

As shown in Figure 7, if the inner flow channel 1316 is not arranged, when the bio-ink cartridge 131 is relatively small in the lateral size (corresponding to the anterior-posterior direction as shown in Figure 5), the two ink cartridge inlets of the bio-ink cartridge 131 cannot be arranged adjacent to each other, and an ink cartridge outlet is required to be arranged therebetween; and meanwhile, the two ink cartridge outlets cannot be arranged adjacent to each other, and an ink cartridge inlet is required to be arranged therebetween for crossing arrangement between the second pipe line 192 and the third pipe line 193 outside the bio-ink cartridge. It will cause trouble in connection of the pipe lines with the corresponding components, and is unfriendly to quick and accurate connection of the ink cartridge inlets and the ink cartridge outlets of the bio-ink cartridge 131 with the corresponding microsphere preparation material pumping device and the microfluidic chip 140.

The inner flow channel 1316 is arranged in the bio-ink cartridge 131, so that the ink cartridge inlets and the ink cartridge outlets of the bio-ink cartridge 131 are arranged in a way satisfying the requirements of convenient pipe line connection. In some embodiments, the first accommodating chamber 1313 is arranged to match with the second accommodating chamber 1314. The first ink cartridge inlet 13151 is arranged adjacent to the second ink cartridge inlet 13152, the first ink cartridge outlet 13153 is arranged adjacent to the second ink cartridge outlet 13154. The first ink cartridge inlet 13151, the second ink cartridge inlet 13152, the first ink cartridge outlet 13153, and the second ink cartridge outlet 13154 are arranged on the top or side of the ink cartridge shell 1311, respectively. The matched arrangement means that the first accommodating chamber and the second accommodating chamber can be arranged in a side-by-side way, a parallel way, or a way one is inside the other. Two ink cartridge inlets are arranged adjacent to each other to avoid an ink cartridge outlet arranged therebetween, and two ink cartridge outlets are arranged adjacent to each other to avoid an ink cartridge inlet arranged therebetween. Therefore, the gas pipe lines and the liquid pipe lines connected with the bio-ink cartridge 131 are not crossed to facilitate the quick and accurate connection of the bio-ink cartridge 131 with the outside.

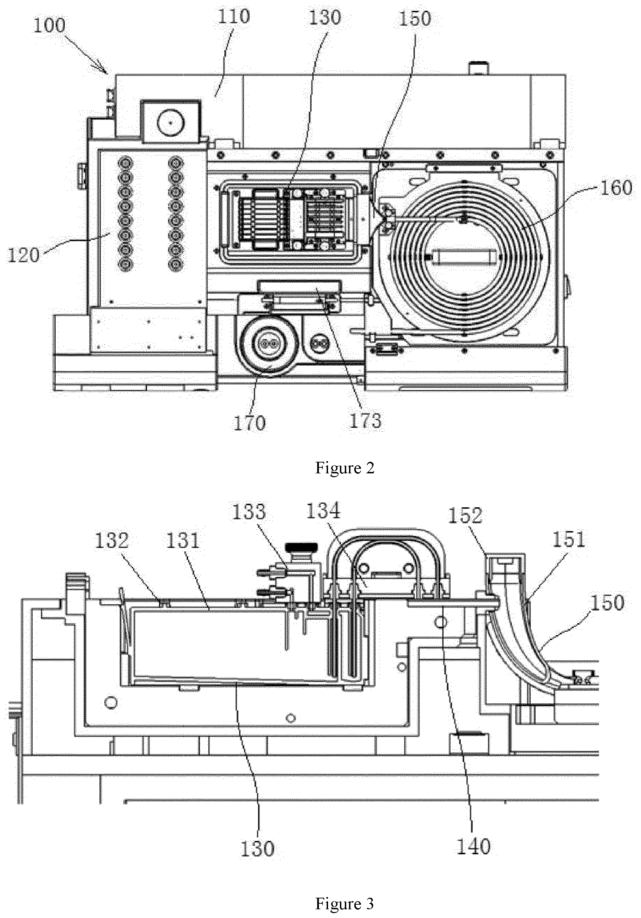

As shown in Figures 3 to 5, in the embodiment, the first accommodating chamber 1313 and the second accommodating chamber 1314 are arranged side by side in the longitudinal direction. The first ink cartridge inlet 13151, the second ink cartridge inlet 13152, the first ink cartridge outlet 13153 and the second ink cartridge outlet 13154 are orderly arranged in the longitudinal direction, from the side of the first accommodating chamber 1313 to the side of the second accommodating chamber 1314, on the top of the ink cartridge shell 1311.

It can be seen that the inner flow channel 1316 is arranged to rationalize positions of the ink cartridge inlets and the ink cartridge outlets.

As shown in Figure 5, the dividing wall 1312 comprises multiple wall segments arranged at an angle, and the inner flow channel 1316 is connected between the first ink cartridge outlet 13153 and one of the multiple wall segments minimizing length of the inner flow channel 1316.

In the embodiment, the multiple wall segments of the dividing wall 1312 comprise a first vertical wall segment 13121, a horizontal wall segment 13122, and a second vertical wall segment 13123. The horizontal wall segment 13122 is spaced apart from a top wall of the ink cartridge shell 13111. The second ink cartridge inlet 13152 and the first ink cartridge outlet 13153 are located above the horizontal wall segment 13122. A vertical inner flow channel 1316 is connected between the first ink cartridge outlet 13153 and the horizontal wall segment to communicate the first accommodating chamber 1313 with the first ink cartridge outlet 13153. The arrangement minimizes the length of the inner flow channel 1316 to minimize resistance of a fluid flowing through the inner flow channel 1316.

As shown in Figure 5, in some embodiments, the inner flow channel 1316 is a linear flow channel. The fluid resistance is minimized as the linear flow channel is the shortest channel.

In some embodiments, the inner flow channel is a zigzag flow channel, a curved flow channel, or a combination of the linear flow channel and the curved flow channel to meet configuration requirements. For example, the dividing wall can be arranged as a single vertical wall, and the inner flow channel is in the form of a curved flow channel or a multi-section zigzag flow channel to realize the purpose of communicating the first accommodating chamber with the first ink cartridge outlet.

In some embodiments, an inner bottom surface of the ink cartridge shell 1311 comprises a first bottom surface located in the first accommodating chamber 1313 and a second bottom surface located in the second accommodating chamber 1314. The first bottom surface comprises a first low zone or a first low point to which the first bottom surface gradually descends from a location away therefrom; and/or the second bottom surface comprises a second low zone or a second low point to which the second bottom surface gradually descends from a location away therefrom.

The arrangement is advantageous for emptying the first accommodating chamber 1313 and the second accommodating chamber 1314 to minimize waste of raw materials.

As shown in Figures 3 and 5, in some embodiments, the ink cartridge shell 1311 comprises an ink cartridge positioning structure 13112 and an elastic snap-in structure 13113 arranged on opposite sides of the outside of the ink cartridge shell 1311 to position and fix the bio-ink cartridge 131 in the microsphere preparation device.

As shown in Figures 3 and 5, in the embodiment, the ink cartridge positioning structure 13112 comprises a strip-type positioning protrusion extending in the superior-inferior direction and arranged on the right side of the ink cartridge shell 1311. The elastic snap-in structure 13113 comprises an elastic clamping strip extending in the superior-inferior direction and arranged on the left side of the ink cartridge shell 1311. A lower end of the elastic clamping strip is connected with a shell wall of the ink cartridge shell 1311, and a snap-in boss is arranged on a side of an upper end of the elastic clamping strip away from the shell wall of the ink cartridge shell 1311.

As shown in Figure 3, the microsphere preparation rack 110 is provided with an ink cartridge mounting position, as well as a positioning matching structure and a snap-in matching structure which are respectively matched with the ink cartridge positioning structure 13112 and the elastic snap-in structure 13113. To mount the bio-ink cartridge, the bio-ink cartridge 131 is pressed down to the corresponding ink cartridge mounting position, the ink cartridge positioning structure 13112 is matched with the positioning matching structure of the ink cartridge mounting position, and the elastic snap-in structure 13113 is matched with the snap-in matching structure of the ink cartridge mounting position, in this way, the bio-ink cartridge 131 is mounted in place. To remove the bio-ink cartridge 131, the elastic snap-in structure 13113 is pressed towards the side of the shell wall of the ink cartridge shell 1311 and the bio-ink cartridge 131 is pulled upwards to smoothly take out the bio-ink cartridge 131.

As shown in Figures 4 and 5, the ink cartridge shell 1311 comprises a connecting structure 13114 for connecting multiple bio-ink cartridges 131 to form the bio-ink cartridge assembly 130. The connecting structure 13114 may comprise a connecting protrusion and/or a connecting recess arranged on the ink cartridge shell 1311. In the embodiment, the connecting structure comprises two connecting protrusions.

As shown in Figure 5, the bio-ink cartridge 131 comprises first sealing structures 135. The first sealing structure 135 has a central through hole. The first sealing structures 135 are arranged in the first ink cartridge inlet 13151, the first ink cartridge outlet 13153, the second ink cartridge inlet 13152 and the second ink cartridge outlet 13154, respectively.

As shown in Figure 5, the first sealing structure 135 comprises a hollow column and a flange located at the top thereof, the hollow column is located in the corresponding ink cartridge inlet or ink cartridge outlet, and the flange is located on an end face of the corresponding ink cartridge inlet or ink cartridge outlet. The arrangement facilitates a sealing connection between the bio-ink cartridge 131 and an integrated connector 133 aftermentioned. Preferably, the first sealing structure 135 is made of a rubber material to guarantee that the corresponding ink cartridge inlet or ink cartridge outlet is well sealed by the deformed first sealing structure 135.

As shown in Figure 5, the bio-ink cartridge 131 further comprises: a first guide wall 1317 arranged in the first accommodating chamber 1313 and extending downwards from a radial outside of the first ink cartridge outlet 13153; and/or a second guide wall 1318 which is arranged in the second accommodating chamber 1314 and extends downwards from a radial outside of the second ink cartridge outlet 13154.

In actual use, the third pipe line 193 and the fourth pipe line 194 connecting the bio-ink cartridge 131 and the microfluidic chip 140 are normally capillary hoses. The capillary hose is flexible and prone to curling up. The first guide wall 1317 and the second guide wall 1318 are arranged; and the first guide wall leads and limits the position of a portion of the third pipe line 193 extending into the first accommodating chamber 1313, and the second guide wall 1318 leads and limits the position of a portion of the fourth pipe line 194 extending into the second accommodating chamber 1314, thus avoiding the problem that the raw materials fail to be completely pressed out due to a curled up a portion of the pipe line extending into the bio-ink cartridge 131, and reducing the waste of the raw materials.

As shown in Figure 5, the first ink cartridge outlet 13153 is located between the first guide wall 1317 and a shell wall of the ink cartridge shell 1311 and/or at least portion of the dividing wall 1312; and/or the second ink cartridge outlet 13154 is located between the second guide wall 1318 and a shell wall of the ink cartridge shell 1311 and/or at least portion of the dividing wall 1312. Proper positioning and arrangement of the first ink cartridge outlet 13153 and/or the second ink cartridge outlet 13154 reduces the area required for arranging the first guide wall 1317 or the second guide wall 1318.

As shown in Figure 5, the bio-ink cartridge 131 further comprises a reinforcing structure 1319 which is arranged in the cavity of the ink cartridge shell 1311 and located at the first ink cartridge inlet 13151, the first ink cartridge outlet 13153, the second ink cartridge inlet 13152 and/or the second ink cartridge outlet 13154.

In some embodiments, the reinforcing structure 1319 comprises reinforcing ribs which are connected with an inner surface of the ink cartridge shell 1311 and/or the dividing wall 1312.

As shown in Figure 5, in the embodiment, the reinforcing structure 1319 comprises a first reinforcing rib 13191, a second reinforcing rib 13192, and a third reinforcing rib 13193. The first reinforcing rib 13191 is a vertical rib connected with the top wall of the ink cartridge shell 1311 and located at the side of the first ink cartridge inlet 13151 away from the dividing wall 1312; and a front end and a rear end of the first reinforcing rib 13191 are connected with a front shell wall and a rear shell wall of the ink cartridge shell 1311, respectively. The second reinforcing rib 13192 is a vertical rib connected with the top wall of the ink cartridge shell 1311 and located at the side of the first ink cartridge inlet 13151 adjacent to the dividing wall 1312; and a front end and a rear end of the second reinforcing rib 13192 are connected with the front shell wall and the rear shell wall of the ink cartridge shell 1311, respectively. The third reinforcing rib 13193 is a vertical rib connected with a lower end of a first vertical wall segment 13121 of the dividing wall 1312 and located at the side of the second ink cartridge inlet 13152 adjacent to the first ink cartridge inlet 13151; and a front end and a rear end of the third reinforcing rib 13193 are connected with the front shell wall and the rear shell wall of the ink cartridge shell 1311, respectively.

The reinforcing ribs are arranged to effectively improve bearing capacity of the bio-ink cartridge 131, beneficial for improving sealing performance of connections between the bio-ink cartridge 131 and the corresponding components, and further beneficial for improving reliability and service life of the bio-ink cartridge 131.

In some embodiments, a chamber wall surface of the first accommodating chamber is hydrophobic; and/or a chamber wall surface of the second accommodating chamber is hydrophobic. The hydrophobic surface is beneficial for maintaining biological activity of cells for the accommodating chamber storing the cell-contained liquid. In addition, the hydrophobic surface is conducive to smooth output of a liquid.

In some embodiments, a volume ratio of the first accommodating chamber 1313 to the second accommodating chamber 1314 of the bio-ink cartridge 131 is 10:1 to 25:1. For example, the volume ratio is 10:1, 12:1, 13.5:1, 15:1, 16.5:1, 17:1, 19:1, 20:1, 21.5:1, 23:1 or 25:1. When the microfluidic chip forms microdroplets required by the bio-blocks by shearing the microsphere stock solution with the shear liquid, a flow ratio of the shear liquid to the microsphere stock solution is close to the volume ratio. Therefore, when the first accommodating chamber 1313 is filled with the shear liquid and the second accommodating chamber 1314 is filled with the microsphere stock solution, the bio-ink cartridge 131 is more suitable for being used together with the microfluidic chip, because two microsphere preparation materials are filled at the same time, consumed around the same time, and fed at the same time to avoid frequent feeding of the microsphere preparation materials due to difference in consumed time therebetween, thus the microsphere preparation efficiency is improved.

As shown in Figures 3 to 11, the bio-ink cartridge assembly 130 of the embodiment comprises the bio-ink cartridge 131.

As shown in Figure 4, the bio-ink cartridge assembly 130 comprises multiple bio-ink cartridges 131 and an ink cartridge connecting member 132 connecting the multiple bio-ink cartridges 131.

The ink cartridge connecting member 132 comprises a strip-type connecting member which comprises a connection fitting structure matched with the connecting structure 13114 on the bio-ink cartridge 131. As shown in Figure 4, the strip-type connecting member is located at a top end of each bio-ink cartridge 131.

The connecting structure 13114 on the bio-ink cartridge 131 comprises a connecting protrusion, and the connection fitting structure comprises a fitting recess matched with the connecting protrusion; and/or the connecting structure 13114 on the bio-ink cartridge 131 comprises a connecting recess, and the connection fitting structure comprises a fitting protrusion matched with the connecting recess. In the embodiment, there are two strip-type connecting members with each comprising multiple fitting recesses matched with the connecting protrusions of the bio-ink cartridges 131. In the embodiment, eight bio-ink cartridges 131 are arranged in parallel. The arrangement allows each bio-ink cartridge 131 to be assembled and disassembled in a convenient way, and positioned accurately relative to each other.

As shown in Figures 3, 8 and 9, in some embodiments, the bio-ink cartridge assembly 130 further comprises an integrated connector 133. The integrated connector 133 comprises a connector body 1331 and fluid connectors 1332.

The connector body 1331 has at least one first connector flow channel 13311 and/or at least one second connector flow channel 13312, an outlet end of the first connector flow channel 13311 is communicated with the first ink cartridge inlet 13151 of the bio-ink cartridge 131, and an outlet end of the second connector flow channel 13312 is communicated with the second ink cartridge inlet 13152 of the bio-ink cartridge 131.

The fluid connector 1332 is connected onto the connector body 1331. Each of inlet end of the first connector flow channel 13311 and an inlet end of the second connector flow channel 13312 is connected with a fluid connector 1332.

The integrated connector facilitates quick and accurate connection between the channels of the microsphere preparation material pumping device 120 and the corresponding ink cartridge inlets of the bio-ink cartridges 131.

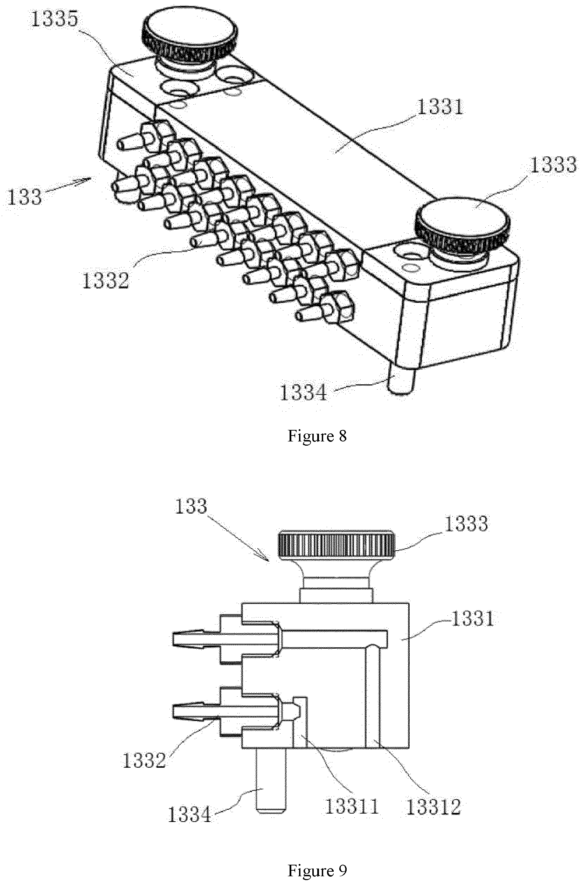

As shown in Figure 8, the integrated connector 133 comprises multiple first connector flow channels 13311 arranged side by side and multiple second connector flow channels 13312 arranged side by side. The arrangement forms a unified interface assembly including the ink cartridge inlets of the bio-ink cartridge assembly 130 comprising the multiple bio-ink cartridges 131, and facilitates the quick and accurate connection between the channels of the microsphere preparation material pumping device 120 and corresponding ink cartridge inlets of the bio-ink cartridges 131 of the bio-ink cartridge assembly 130.

As shown in Figures 3, 8 and 9, the inlet end of the first connector flow channel 13311 and the inlet end of the second connector flow channel 13312 are located on the same side of the connector body 1331. The outlet end of the first connector flow channel 13311 and the outlet end of the second connector flow channel 13312 are located on the same side of the connector body 1331. The inlet end and the outlet end of the first connector flow channel 13311 are located on different sides of the connector body 1331 respectively. The inlet end and the outlet end of the second connector flow channel 13312 are located on different sides of the connector body 1331 respectively. The arrangement facilitates the quick and accurate connection between the channels of the microsphere preparation material pumping device and the corresponding ink cartridge inlets of the bio-ink cartridges 131 of the bio-ink cartridge assembly 130.

As shown in Figures 3, 8 and 9, the integrated connector 133 further comprises a connector positioning structure 1334 which is arranged on the connector body 1331 for positioning the integrated connector 133 relative to the bio-ink cartridge 131.

In the embodiment, the connector positioning structure 1334 comprises a locating pin arranged on a front side and a rear side of the integrated connector 133, respectively. When the integrated connector 133 is assembled with the bio-ink cartridges 131, the integrated connector 133 is accurately positioned relative to the bio-ink cartridges 131 by inserting the locating pin into a corresponding locating hole on the microsphere preparation rack 110.

As shown in Figures 3, 8 and 9, the integrated connector 133 further comprises a connector connecting member 1333 for fixing the integrated connector 133 relative to the bio-ink cartridge 131.

In the embodiment, the connector connecting member 1333 is a threaded component, and the integrated connector 133 further comprises a backing plate 1335 which is located between a head of the threaded component and the connector body 1331.

After the integrated connector 133 is positioned, the integrated connector 133 is fixed by rotating the threaded component into a screw hole correspondingly arranged on the microsphere preparation rack 110. The threaded component can be provided with a spinning head with which the integrated connector 133 is fixed relative to the bio-ink cartridge 131 without a screwing tool.

The backing plate 1335 can be fixed onto the connector body 1331 by screws or the like. The backing plate 1335 can be made of a material with a strength higher than the connector body 1331 to prevent local damage of the connector body 1331 caused by the threaded component.

After the integrated connector 133 is press-mounted on the top of the multiple bio-ink cartridges 131, the connector flow channels of the integrated connector 133 are communicated with the corresponding ink cartridge inlets. After the integrated connector 133 is press-mounted on the top of each bio-ink cartridges 131, the flange of the first sealing structure 135 is located between the end face of the corresponding ink cartridge inlet and the end face of the connector flow channel outlet of the integrated connector 133, and is deformed to realize the sealing connection between the connector flow channel of the integrated connector 133 and the ink cartridge inlet of the corresponding bio-ink cartridge 131.

As shown in Figures 3, 10 and 11, in some embodiments, the bio-ink cartridge assembly 130 further comprises an outlet integration head 134. The outlet integration head 134 comprises an integration head body 1341 which has at least one first through hole 13413 for the third pipe line 193 connected with the first ink cartridge outlet 13153 of the bio-ink cartridge 131 to pass through; and/or at least one second through hole 13415 for the fourth pipe line 194 connected with the second ink cartridge outlet 13154 of the bio-ink cartridge 131 to pass through. The arrangement is easy to realize the quick and accurate connection between the ink cartridge outlets of the bio-ink cartridges 131 and the chip inlets of the microfluidic chip 140 at one end of the bio-ink cartridge 131.

As shown in Figures 3, 10 and 11, in some embodiments, multiple first through holes 13413 are arranged in the form of a first through hole row, multiple second through holes 13415 are arranged in the form of a second through hole row, and the first through hole row and the second through hole row are arranged side by side. This arrangement facilitates the quick and accurate connection between the ink cartridge outlets of the multiple bio-ink cartridges 131 of the bio-ink cartridge assembly 130 and the chip inlets of the microfluidic chip 140 at one end of the bio-ink cartridge 131.

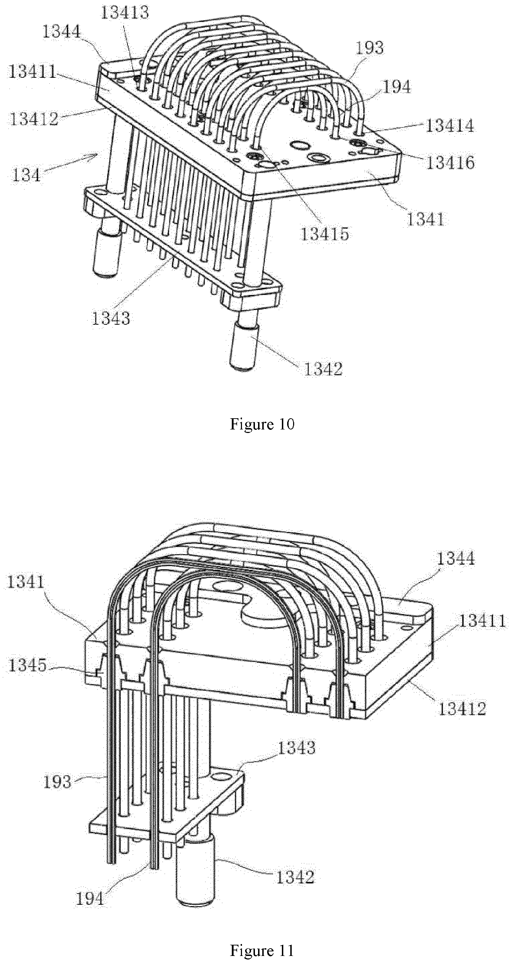

As shown in Figure 11, the integration head body 1341 comprises second sealing structures 1345 having a central through hole. The second sealing structure 1345 is arranged in the first through hole 13413 and/or the second through hole 13415, and pipe lines pass through the central through holes of the second sealing structure 1345 in the corresponding through hole.

As shown in Figures 3, 10 and 11, in some embodiments, the integration head body 1341 has at least one third through hole 13414 for the third pipe line 193 connected with the first ink cartridge outlet 13153 of the bio-ink cartridge 131 and the second chip inlet 1421 of the microfluidic chip 140 to pass through; and/or at least one fourth through hole 13416 for the fourth pipe line 194 connected with the second ink cartridge outlet 13154 of the bio-ink cartridge 131 and the first chip inlet 1411 of the microfluidic chip 140 to pass through. This arrangement is beneficial for the quick and accurate connection between the ink cartridge outlets of the bio-ink cartridges 131 and the corresponding chip inlets of the microfluidic chip 140 at one side of the microfluidic chip 140.

As shown in Figures 3, 10 and 11, multiple first through holes 13413 are arranged in the form of the first through hole row, multiple second through holes 13415 are arranged in the form of the second through hole row, multiple third through holes 13414 are arranged in the form of a third through hole row, and multiple fourth through holes 13416 are arranged in the form of a fourth through hole row. The first through hole row, the second through hole row, the fourth through hole row and the third through hole row are orderly arranged side by side. This arrangement is beneficial for the quick and accurate connection between the ink cartridge outlets of the multiple bio-ink cartridges 131 of the bio-ink cartridge assembly 130 and the chip inlets of the microfluidic chip 140 at one side of the bio-ink cartridges 131 and at one side of the microfluidic chip 140.

As shown in Figures 3 and 11, in some embodiments, the integration head body 1341 comprises multiple second sealing structures 1345 having a central through hole. The second sealing structures 1345 are arranged in the first through hole 13413, the second through hole 13415, the third through hole 13414 and/or the fourth through hole 13416, and pipe lines pass through the central through holes of the second sealing structure 1345 in the corresponding through hole.

As shown in Figures 3, 10 and 11, in the embodiment, the integration head body 1341 comprises a first plate body 13411 and a second plate body 13412, and the second sealing structure 1345 is clamped between the first plate body 13411 and the second plate body 13412.

As shown in Figure 11, the overall second sealing structure 1345 is a tubular structure which is provided with a central radial boss. A bottom of the first plate body 13411 is provided with a stepped hole, and an upper small-diameter segment of the stepped hole is matched with an upper tube segment of the tubular structure of the second sealing structure 1345. A lower large-diameter segment of the stepped hole is matched with the radial boss. The second plate body 13412 is located at a lower portion of the first plate body 13411, and the second plate body 13412 is provided with a through hole. When the second plate body 13412 approaches the first plate body 13411, a lower tube segment of the tubular structure of the second sealing structure 1345 is matched with the through hole, and the radial boss is arranged between the first plate body 13411 and the second plate body 13412 in a clamping manner to realize the assembly and matching of the second sealing structure 1345 and the integration head body 1341. The large-diameter segment and the small-diameter segment of the stepped hole as well as the through hole are matched with the corresponding portions of the second sealing structure 1345 in shape. For example, in the embodiment, the outline of the upper tube segment of the second sealing structure 1345 is frustum-shaped with a diameter increasing from top to bottom, and the small-diameter segment of the stepped hole is also frustum-shaped with a diameter increasing from top to bottom.

Preferably, the second sealing structure 1345 is made of such materials as rubber to guarantee that the corresponding ink cartridge outlet or the corresponding chip inlet 1412 are well sealed by the deformed second sealing structure 1345.

As shown in Figures 3, 10 and 11, in some embodiments, the outlet integration head 134 comprises a guide structure. The guide structure is configured to guide the third pipe line 193 passing through the first through hole 13413 to pass through the first ink cartridge outlet 13153 of the bio-ink cartridge 131 and enter the first accommodating chamber 1313; and/or configured to guide the fourth pipe line 194 passing through the second through hole 13415 to pass through the second ink cartridge outlet 13154 of the bio-ink cartridge 131 and enter the second accommodating chamber 1314.

In some embodiments, the guide structure comprises a guide plate 1343 which is movably arranged relative to the integration head body 1341 and located between the first ink cartridge outlet 13153 and/or the second ink cartridge outlet 13154 of the bio-ink cartridge 131 and the integration head body 1341; and the guide plate 1343 comprises a first guide hole corresponding to the first through hole 13413 and/or a second guide hole corresponding to the second through hole 13415. When inserting pipe line, as the third pipe line 193 or the fourth pipe line 194 continuously penetrates into the corresponding accommodating chamber, the guide plate 1343 continuously approaches the integration head body 1341 until the guide plate 1343 is attached to the integration head body 1341.

As shown in Figures 10 and 11, the guide structure further comprises a guide post 1342. The guide post 1342 is connected with the integration head body 1341, and the guide plate 1343 is slidably connected onto the guide post 1342. The guide post 1342 also can be matched with the guide hole on the microsphere preparation rack 110 to define the position of the integration head body 1341 relative to the bio-ink cartridge 131.

As shown in Figures 10 and 11, in the embodiment, the outlet integration head 134 further comprises an integration head positioning structure 1344 which is arranged on the integration head body 1341 for positioning the outlet integration head 134 relative to the bio-ink cartridge 131.

In the embodiment, the integration head positioning structure 1344 comprises two positioning plates which are oppositely arranged on the front side and the rear side, and two positioning plate mounting grooves are correspondingly provided on a top end of the integration head body 1341. When the outlet integration head 134 is mounted on the microsphere preparation rack 110, the positioning plates are located between the microsphere preparation rack 110 and the integration head body 134 to define the position of the outlet integration head 134 relative to the microsphere preparation rack 110, and further define the position of the bio-ink cartridge 131 relative to the outlet integration head 134.

Optionally, the outlet integration head 134 is fixedly connected with the microsphere preparation rack 110 through a threaded component. In that case, the integration head body 134 is press-mounted above the ink cartridge outlet of the corresponding bio-ink cartridge, the flange of the first sealing structure 135 is located between the corresponding ink cartridge outlet and the integration head body 1341, and is deformed to realize the sealing connection between the outlet integration head 134 and the bio-ink cartridge 131.

In the embodiment, the third pipe line 193 is connected between the first ink cartridge outlet 13153 of the bio-ink cartridge 131 and the second chip inlet 1421 of the microfluidic chip 140, and the fourth pipe line 194 is connected between the second ink cartridge outlet 13154 of the bio-ink cartridge 131 and the first chip inlet 1411 of the microfluidic chip 140.

The embodiment provides a miniaturized and integrated bio-ink cartridge 131 and bio-ink cartridge assembly 130. The bio-ink cartridge 131 or the bio-ink cartridge assembly is particularly suitable for storing the microsphere stock solution containing cells and the corresponding shear liquid. The bio-ink cartridge 131 is beneficial for maintaining the biological properties (e.g., sternness of stem cells and cell viability) of the cells and other active substances. The miniaturization and integration of the bio-ink cartridge 131 facilitate realization of the miniaturization of the overall microsphere preparation device and the bio-block preparation instrument, and further facilitate the realization of a relatively high preparation efficiency of the microspheres and the bio-blocks.

A tunnel-like structural design is generated in the bio-ink cartridge 131 through the arrangement of the internal flow channel 1316, which solves the problem of crossed external pipe lines when the liquid in the bio-ink cartridge 131 is driven by pressure.

With the first sealing structures 135 arranged at all the ink cartridge inlets and ink cartridge outlets and the second sealing structure 1345 arranged in the through hole of the outlet integration head 134, the bio-ink cartridge 131 and the bio-ink cartridge assembly 130 realize the sealing connections between the integrated connector 133 and the ink cartridge inlets of the bio-ink cartridge 131, as well as the outlet integration head 134 and the ink cartridge outlets of the bio-ink cartridge 131 and the chip inlets of the microfluidic chip 140, so that the bio-ink cartridge 131 is capable of bearing certain gas pressure when the liquid in the bio-ink cartridge 131 is driven by gas pressure.

The reinforcing structure 1319 in the bio-ink cartridge 131 ensures the bearing capacity thereof and avoids the problem of deformation or poor sealing of the bio-ink cartridge 131 under pressure.

The microsphere preparation materials are relatively expensive, in particular the microsphere stock solution. Two accommodating chambers of the bio-ink cartridge 131 are provided with low zones or low points, so that the raw materials automatically converge to the low zones or the low points when there is relatively less microsphere preparation material left, and the microsphere preparation material is led out from the low zones or the low points for utilization to the maximum extent.

The first guide wall 1317 and the second guide wall 1318 are arranged in a way avoiding the problem that the raw materials fail to be completely pressed out due to the curled up portion of the pipe lines extending into the bio-ink cartridge 131, and reducing the waste of the raw materials.

The bio-ink cartridge 131 of the embodiment can be used alone, or multiple bio-ink cartridges 131 are assembled into a set of bio-ink cartridge assembly 130 as required.

The integrated connector 133 of the bio-ink cartridge assembly 130 facilitates the quick and accurate connection of the ink cartridge inlets of the bio-ink cartridge 131 with the microsphere preparation material pumping device 120, and realizes the sealing connections with the ink cartridge inlets of the bio-ink cartridge 131.

The outlet integration head 134 of the bio-ink cartridge assembly 130 facilitates the quick and accurate connection of the ink cartridge outlets of the bio-ink cartridge 131 with the chip inlets of the microfluidic chip 140, and realizes the sealing connection with the ink cartridge outlets of the bio-ink cartridge 131 and the chip inlets of the microfluidic chip 140.

In some embodiments, the microsphere preparation device comprises a microsphere preparation material temperature control unit which keeps the microsphere preparation material storage device 120 and/or the microfluidic chip 140 at a preset temperature or within a preset temperature range.

Preferably, the microsphere preparation material temperature control unit keeps the microsphere stock solution and the shear liquid at about 4°C before entering the buffer device. The temperature setting is beneficial to sternness expression of the cells in the microsphere stock solution and the microdroplets and beneficial for keeping the cell sternness.

In the embodiment, the microsphere preparation device has a first temperature zone in which the bio-ink cartridge assembly 130, the bio-ink cartridge 131 and the microfluidic chip 140 are mounted. The microsphere preparation material temperature control unit regulates the temperatures of the microsphere preparation material storage device, the microfluidic chip 140 and the microsphere preparation material therein by adjusting the temperature of the first temperature zone.

To realize an accurate temperature control of the microsphere preparation material storage device and the microfluidic chip 140, the microsphere preparation material temperature control unit comprises a microsphere preparation material temperature measuring element which is configured to monitor the temperature of the microsphere preparation material storage device and/or the microfluidic chip. For example, the microsphere preparation material temperature measuring element monitors the temperature of the microsphere preparation material storage device and/or the microfluidic chip by monitoring the temperature of the first temperature zone.

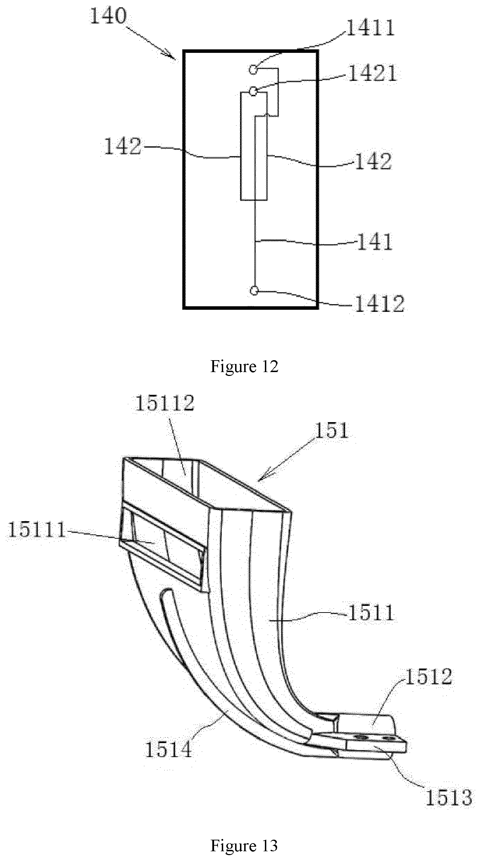

Figure 12 is a structural diagram of a microfluidic chip of a microsphere preparation device according to an embodiment of the present disclosure. In Figure 12, to illustrate the working principle of the microfluidic chip 140, only a set of first chip channels 141 and corresponding second chip channels 142 are shown. When the microfluidic chip 140 is matched with multiple bio-ink cartridges 131, the microfluidic chip 140 can comprise more sets of the first chip channels 141 and the corresponding second chip channels 142. Definitely, it is also possible to assemble a desired number of sets of the first chip channels 141 and the corresponding second chip channels 142 through multiple independent microfluidic chip bodies in parallel. For example, in the embodiment, eight sets of the first chip channels 141 and the corresponding second chip channels 142 are arranged according to the number of the bio-ink cartridge assemblies 130.

As shown in Figure 12, the microfluidic chip 140 comprises the first chip inlet 1411 in communication with the microsphere stock solution storage space and the second chip inlet 1421 in communication with the shear liquid storage space. In the microfluidic chip 140, the microsphere stock solution entering the first chip channel 141 through the first chip inlet 1411 is sheared into microdroplets by the shear liquid entering the second chip channel 142 through the second chip inlet 1421, and the microdroplet-shear liquid mixture flows through the portion of the first chip channel 141 located downstream of a shear site and is output from the chip outlet 1412 of the microfluidic chip.

As shown in Figures 1 to 3, the microfluidic chip 140 is mounted on a base of the microsphere preparation rack 110. The microfluidic chip 140 is connected with the corresponding ink cartridge outlets of the bio-ink cartridge 131 through the outlet integration head 134, the third pipe line 193 and the fourth pipe line 194 at the left side, and connected with the buffer device 150 through the third sealing structure 153 at the right side.

The microfluidic chip 140 is described below in combination with Figure 12. The microfluidic chip 140 described in the embodiment is exemplary, and those skilled in the art can make corresponding changes on such basis.

The principle of generating the microdroplets by the microfluidic chip is: two immiscible fluids are introduced into different chip channels of the microfluidic chip, the one is a dispersed phase fluid and the other is a continuous phase fluid used as the shear liquid. The two fluids meet at the intersection between the different chip channels, and the dispersed phase fluid is separated into dispersed microdroplets by the continuous phase fluid.

Particularly, the continuous phase fluid and the dispersed phase fluid respectively entering the corresponding chip channels in the microfluidic chip will form an interface of the continuous phase fluid and the dispersed phase fluid at the intersection between the different chip channels. The dispersed phase fluid moves forwards with the continuous phase fluid under the action of an external force and a shear force of the continuous phase fluid. When an interfacial tension at the interface is not sufficient to maintain the shear force applied by the continuous phase fluid to the dispersed phase fluid, the dispersed phase fluid is broken to generate an independent small volume units (i.e., microdroplets) surrounded by the continuous phase fluid.

The microfluidic chip 140 is the core component of the microsphere preparation device and the bio-block preparation instrument. The microsphere stock solution is sheared into microdroplets in the microfluidic chip 140. The microfluidic chip 140 is also the component causing the greatest damage to cells in the overall system. The yield is improved and the shear force on the cells is minimized to maintain the cell viability by improving the channel size and channel depth-to-width ratio of the microfluidic chip 140 and regulating the flow ratio of the two phases.

In some embodiments, the microfluidic chip 140 comprises a chip body as well as the first chip channel 141 and the second chip channel 142 arranged therein. The first chip channel 141 and the second chip channel 142 communicate with each other and form an intersection zone. The microsphere stock solution enters from the first chip channel 141 and the shear liquid enters from the second chip channel 142 to shear the microsphere stock solution into the dispersed microdroplets in the intersection zone, and the cross sectional area of the first chip channel 141 and the second chip channel 142 ranges from 0.1 mm² to 1 mm². Preferably, the cross sectional area is selected to be 0.1mm², 0.2mm², 0.3mm², 0.4mm², 0.5mm², 0.6mm², 0.7mm², 0.8mm², 0.9mm² and 1mm².

If the cross sectional area of the first chip channel 141 and the second chip channel 142 is set to be a value ranging from 0.1 mm² to 1 mm², the flow rate increases with the fluid passing rate, and the generation efficiency of the microdroplets improves. Further, the chip channel at a size within the range decreases the flow rate of the fluid, so that the shear force caused by a friction between the cells and a chip channel wall when the fluid is flowing in the chip channel decreases correspondingly. Therefore, the damage to the cells reduces to ensure the cell viability. Meanwhile, sufficient shear force is still available to shear the cell stock solution into the dispersed microdroplets by finely tuning the flow rate in the generation process. Therefore, the microfluidic chip 140 of the embodiment can increase the efficiency of microdroplet generation while ensuring the cell viability to meet the requirements of 3D bio-printing.

When the microfluidic technology is employed to form the microdroplets, the chip channel size has a direct impact on the yield and cell viability of the microdroplets. Therefore, to meet the requirements of 3D bio-printing, it is necessary to select the appropriate chip channel size according to the desired yield and cell viability of the microdroplets, and to select the appropriate flow rate with reference to fluid viscosity, so as to make all coupling parameters match with each other and finally meet the requirements for the yield and cell viability of the microdroplets. This is essentially different from the principle of a chip that realizes a liquid separation by the microfluidic technology. A series of bifurcated chip channels are arranged in the liquid separation chip to divide the liquid, and the cross sectional area of each chip channel is directly designed according to the allocated flow rate. It can be seen that due to the different microfluidic purposes, when the microdroplets are formed by the microfluidic technology, the size of the liquid separation chip channel is unable to be referred for selecting the chip channel size.

When the chip channel size increases, more fluid is allowed to pass through the same cross section, and the flow rate of the fluid passing through in a unit time increases. The consumption of raw materials (e.g., cell solution) increases with the flow rate of the microsphere stock solution, and the shear force on the cell solution increases with the flow rate of the shear liquid. Both of the increases affect the efficiency of generating the microdroplets through the microfluidic chip 140.

Further, the increase in the chip channel size is beneficial for adjusting the flow rates of the shear liquid and the cell stock solution, in particular to be beneficial to decrease the flow rates of the two phases; after the flow rate of the cell stock solution decreases, the shear force caused by the friction between the cells and a chip channel inner wall when the fluid is flowing in the chip channel decreases correspondingly, and the cell viability is guaranteed before being sheared at the intersection zone; and the decrease in the flow rate of the shear liquid properly reduces the shear force applied to the cell stock solution by the shear liquid at the intersection zone of different chip channels, and minimizes the damage to the raw materials containing the cells when the microsphere stock solution is separated into microdroplets at a predetermined size.

Although the flow rate of the shear liquid decreases and the shear force applied to the microsphere stock solution correspondingly decreases, there is still sufficient shear force applied to separate the microsphere stock solution due to the increase in the flow rate of the shear liquid passing through the intersection zone in a unit time. If a relatively great shear force is needed due to the relatively high viscosity of the cell stock solution, the shear liquid still has sufficient shear force to disperse the cell solution into the microdroplets by finely tuning the flow rate of the two phases. According to analysis, the increase in the channel size of the microfluidic chip 140 can improve the efficiency of microdroplet generation on the basis of ensuring the cell viability.

One or more second chip channels 142 are connected with the specific first chip channel 141, and every segment, on the sides of the first chip channel 141, that is individually used for introducing the shear liquid is defined as one second chip channel 142.

In some embodiments, the cross sections of the first chip channel 141 and the second chip channel 142 are rectangular.

The chip channel with rectangular cross section has the following advantages:
1. The chip channel is convenient to be processed in the chip body, and the size is easy to be guaranteed to reduce the manufacturing difficulty of the microfluidic chip 140;
2. The chip channel size is easy to be guaranteed to control various microfluidic parameters (e.g., the flow rate of the fluids at the intersection zone) more accurately;
3. The resistance to the fluids flowing in the chip channels decreases, so the pressure is maintained as required to keep the desired flow rate at the intersection zone;
4. To reduce adhesion between the inner surface of the chip channel and the microdroplets and further reduce the flow resistance, preferably, the inner surface of the chip channel is coated with a hydrophobic material to make the microdroplets flow out of the chip channel more easily. The chip channel with the rectangular cross section is relatively convenient to be coated with the hydrophobic material, and the uniform thickness of the coating is easy to be guaranteed.
5. It is beneficial for manufacturing the microfluidic chip with chip channels stacked in a height direction.

Optionally, according to application requirements, the first chip channel 141 and the second chip channel 142 are designed to have a circular cross section, a trapezoidal cross section or the cross sections in other shapes allowing smooth fluid flow without damage to the cells in the microsphere stock solution.

In some embodiments, the size of the portion of the first chip channel 141 upstream of the intersection zone for transporting the fluid is consistent. Alternatively, the size of the first chip channel 141 located at the upstream of the intersection zone increases along the flow direction of the microsphere stock solution. The advantage of the embodiment is that the damage to the cells when the microsphere stock solution is flowing in the first chip channel 141 decreases, so that the cell viability is guaranteed on the premise of improving the microdroplet yield. In case a greater shear force is needed due to the relatively high viscosity of the cell stock solution, the shear liquid still has sufficient shear force to disperse the cell solution into the microdroplets by finely tuning the flow rate of the two phases.

Alternatively, the microfluidic chip 140 uses a pinched-flow focusing chip channel or a T-type chip channel. The pinched-flow focusing chip channel or the T-type chip channel is beneficial for adjusting the size and generation rate of the microdroplets, and is also beneficial for reducing the damage to the active substances in the microdroplets during the generation thereof.

In the embodiments of one type of the channel, two the second chip channels 142 are respectively provided on two sides of the first chip channel 141, and the shear liquid is respectively introduced into the two second chip channels 142 to form a pinched-flow focusing chip channel at the intersection zone. The microsphere stock solution is introduced from one end of the first chip channel 141, and the shear liquid is respectively introduced from the two second chip channels 142. A certain shear force is applied to the microsphere stock solution by adjusting the flow rate of the shear liquid. Meanwhile, the microsphere stock solution is separated into the dispersed microdroplets by utilizing the fluid instability. The pinched-flow focusing chip channel produces better monodisperse microdroplets, controls the microdroplet size accurately, and applies the shear force less than that of the T-type chip channel to maintain the cell viability.

In some embodiments, the first chip channel 141 and the two second chip channels 142 form a cross-shaped structure at the intersection zone. That is, an angle between the first chip channel 141 and the two second chip channels 142 is 90°.

The microsphere stock solution enters the first chip channel 141 from the first chip inlet 1411, and the shear liquid enters the second chip channels 142 from the second chip inlet s 1421. In the zone where the first chip channel 141 meets the two second chip channels 142, the shear liquid produces pinching and focusing effect on the microsphere stock solution, and the microsphere stock solution is separated into dispersed microdroplets under the action of symmetric shear forces applied by the shear liquid from both sides.

Each of two second chip channels 142 form an L-shaped structure at the intersection zone; a transverse portion of the L-shaped structure is communicated with and perpendicular to the first chip channel 141; and a longitudinal portion of the L-shaped structure is parallel to the first chip channel 141 and consistent with the first chip channel 141 in the extending direction along which the first chip channel 141 introduces the microsphere stock solution.

The second chip channel 142 designed as an L-shaped structure reduces the width of a single microfluidic unit in the microfluidic chip 140, which is beneficial to arranging more microfluidic units at intervals in the width direction of the microfluidic unit without expansion of the chip body area and improving the yield of the microdroplets thereby. In addition, when different microdroplets need to be prepared, the structure is relatively easy to ensure the desired yield of microdroplets of each type.

Preferably, the distance between the longitudinal portion of the L-shaped structure and the first chip channel 141 ranges from 0.1 mm to 20 mm, which is calculated depending on the center lines of these two chip channels. For example, the distance between the longitudinal portion of the L-shaped structure and the first chip channel 141 is 0.1mm, 0.5mm, 1mm, 3mm, 5mm, 8mm, 10mm, 12mm, 15mm, 18mm or 20mm.

When the shear liquid entering from the longitudinal portion of the L-shaped structure passes through a bending portion, fluid units are quite different in the flow direction and the flow rate. If the distance between the longitudinal portion of the L-shaped structure and the first chip channel 141 is too short, the shear liquid entering the transverse portion of the L-shaped structure from the bending portion shears the microsphere stock solution at an unstable flow rate and flow direction. The unstable shear force is prone to cause damage to cells and unable to guarantee the size of the finally formed microdroplets. If the distance between the longitudinal portion of the L-shaped structure and the first chip channel 141 is too long, the flow rate of the microsphere stock solution entering the transverse portion of the L-shaped structure easily attenuates due to the resistance from the inner wall of the second chip channel 142, and the shear force applied to the microsphere stock solution is affected ultimately.

Therefore, in the embodiment, the distance between the longitudinal portion of the L-shaped structure and the first chip channel 141 is selected within the range to enable the shear liquid to flow at a stable state in the transverse portion of the L-shaped structure and apply a stable shear force to the microsphere stock solution at the intersection zone, which is beneficial for ensuring the cell viability and improving the uniformity and controllability of the microdroplet size. Further, the attenuation of the flow rate of the shear liquid flowing in the transverse portion of the L-shaped structure is reduced to apply the sufficient shear force to the microsphere stock solution.

In addition to use of the cross chip channels, in other embodiments, the two second chip channels 142 and the first chip channel 141 are arranged at an angle, and the angle between the second chip channels 142 and the first chip channel 141 is less than 90°. Preferably, the two second chip channels 142 incline in the same direction.

In some embodiments, the two second chip channels 142 are symmetrically arranged relative to the first chip channel 141. The two symmetrically arranged second chip channels 142 incline at the same angle relative to the first chip channel 141. Preferably, the two second chip channels 142 has identical length. The symmetrical arrangement of the inclination angles ensures that consistent shear forces are applied by the shear liquid from both sides, which is beneficial to ensuring uniform size and activity of the microdroplets; and the symmetrical lengths allow the shear liquid introduced at the same flow rate from both sides to meet at the intersection zone at the same flow rate, so that the shear forces applied by the shear liquid from both sides are consistent.

In some alternative embodiments, the microfluidic chip 140 uses the T-type chip channel. The T-type chip channel comprises a third chip channel and a fourth chip channel intersecting therewith. The third chip channel is configured to introduce the shear liquid, the fourth chip channel configured to introduce the microsphere stock solution, and the shear liquid separates the microsphere stock solution into the microdroplets at the intersection between the third chip channel and the fourth chip channel.

In some embodiments, on the basis of the microfluidic chip 140, the inner surfaces of the first chip channel 141 and the second chip channel 142 are hydrophobic surfaces with hydrophobicity, which can be realized by coating a hydrophobic material. Preferably, the inner surface of the first chip channel 141 and the inner surface of the second chip channel 142 are superhydrophobic. When water-containing microdroplets are generated, the arrangement reduces the adhesion between the inner surface of the chip channel and the microdroplets, thereby reducing the flow resistance.

Preferably, the microfluidic chip 140 is made of polydimethylsiloxane (PDMS), and the inner surface of the chip channel of the microfluidic chip 140 is hydrophobically treated so that the inner surface for the chip channel of the microfluidic chip 140 is hydrophobic. Further, the microfluidic chip 140 is made of glass or polymethyl methacrylate (PMMA), and the inner surface of the chip channel is hydrophobically treated so that the inner surface of the chip channel of the microfluidic chip 140 is hydrophobic. The inner surface of the chip channel is hydrophobically treated by means of, for example, octadecyltrichlorosilane (OTS).

Since the cross-sectional sizes of the first chip channel 141 and the second chip channel 142 have an important impact on the yield and activity of the microdroplets, the microfluidic chip 140 channel with rectangular cross section is taken as an example to describe the impact of the chip channel size selection on the cell viability and yield in the microdroplets.

Preferably, the first chip channel 141 and the second chip channel 142 have rectangular cross sections with a length ranging from 0.7mm to 2mm, and/or a width ranging from 0.2mm to 0.6mm. Any one or both of the ranges of the length and the width of the rectangular cross section should be satisfied.

Preferably, the length of the rectangular cross section is 0.7mm, 0.8mm, 0.9mm, 1.0mm, 1.1mm, 1.2mm, 1.3mm, 1.4mm, 1.5mm, 1.6mm, 1.7mm, 1.8mm, 1.9mm and 2mm; and the width of the rectangular section is 0.2mm, 0.25mm, 0.3mm, 0.35mm, 0.4mm, 0.45mm, 0.5mm, 0.55mm and 0.6mm.

The use of a cross-type passive microfluidic chip 140 of the related art can increase the flow rate to improve the microdroplet yield, but the microdroplet size is significantly limited by the flow rate, and the shear force increases with the flow rate. As a result, the yield increases, and the cell viability is adversely affected. Therefore, compared with the related art, the microfluidic chip 140 of the present disclosure increases the chip channel size, including increase in the length and the width of the rectangular cross section individually or concurrently.

If the length of the cross section of the chip channel is increased individually to form a flat chip channel, the yield in the microfluidic chip 140 increases due to the increase in the cross sectional area of the chip channel. However, in case of a constant flow rate, a relatively great shear force is applied to the cell stock solution flowing in the chip channel due to the flat cross section thereof and prone to causing damage to the cells. Reduction in the shear force means reduction in the flow rate that decreases the yield. It can be seen that the yield and cell viability of the microdroplets have mutual restriction relation.

When the microfluidic chip 140 works, the flow rates of the two phases are appropriately reduced to lower the shear force to meet the necessary biological activity. The decrease in the flow rate is compensated for by the increase in the cross sectional area of the chip channel to guarantee the increase in the yield of the microdroplets. Therefore, the increase in the yield depending on the increase in the cross sectional area of the chip channel should be greater than the negative effect on the yield by the decrease in the flow rate.

If the width of the cross section of the chip channel is increased individually, another flat chip channel is formed, which is similar to the case that the cross section length of the chip channel is increased individually.

If the length and the width of the cross section of the chip channel are increased simultaneously (e.g., the length of the cross chip channel is increased to 1mm and the width thereof is increased to 0.2mm), the yield of the microdroplets significantly improves due to the increase in the overall cross section size of the chip channel and the flow passing through the cross section of the chip channel in a unit time. Experiments show that the yield is increased to a predetermined value and the cell viability is higher than that of the related art. For example, the dimensions of the rectangular chip channel can be designed as: 1.5mm × 0.2mm, 1mm × 0.2mm, 1mm × 0.3mm, 0.75mm × 0.3mm and 0.75mm × 0.5mm.

Preferably, diameter size of the microdroplet generated by the microfluidic chip 140 of the present disclosure ranges from 200µm to 400µm. In practice, the microdroplet size is controlled by adjusting the loading flow rate ratio of the shear liquid to the microsphere stock solution. The microdroplet size increases with the flow rate of the microsphere stock solution. The microdroplet size decreases with the increasing flow rate of the shear liquid. Therefore, the microfluidic chip 140 is characterized by simple operation and high repeatability in control of the microdroplet size.

In a specific embodiment, the microfluidic chip 140 with the chip channel size of 0.75mm × 0.3mm is used to obtain the microdroplets with an average size of 300µm in case of the dispersed phase viscosity of 1200cp, the dispersed phase flow rate of 15µL/min and the continuous phase flow rate of 150µL/min. The microdroplets with diameters ranging from 280µm to 290µm account for 40% of the total number of microdroplets, the microdroplets with diameters ranging from 290µm to 300µm account for 50% of the total number, and the average diameter of the microdroplets is 291µm.

The microfluidic chip 140 of the embodiment of the present disclosure is available in the following scenarios: the viscosity ratio of the microsphere stock solution to the shear liquid ranges from 10:1 to 30:1 (e.g., 10:1, 12:1, 14:1, 15:1, 17:1, 18:1, 20:1, 22:1, 24:1, 26:1, 28:1 and 30:1).

Preferably, the viscosity of the microsphere stock solution ranges from 500cp to 3000cp (e.g., 500cp, 700cp, 1000cp, 1200cp, 1500cp, 1800cp, 2000cp, 2200cp, 2400cp, 2600cp, 2800cp and 3000cp). The microfluidic chip 140 of the embodiment of the present disclosure is more suitable for the scenario that the viscosity of the microsphere stock solution is more than or equal to 2000cp, and the viscosity of the shear liquid is relatively low, generally not more than 100cp.

In some embodiments, the shear force applied to form the microdroplets at the intersection zone of the first chip channel 141 and the second chip channel 142 is less than 100Pa. The shear force is related to a shear rate and the viscosity. The shear force increases with the flow rate of the shear liquid, and the microsphere stock solution with a high viscosity requires to be sheared by a great shear force.

The microsphere stock solution for forming the microdroplets is a cell-loaded microgel or any cell-containing solution (e.g., a naturally occurring solution, an artificially synthesized solution, a sufficiently produced solution, a modified solution, or any combination thereof). The naturally occurring raw materials are naturally occurring biodegradable materials derived from animals and plants (e.g., collagen, fibrin, chitosan, alginate, starch, hyaluronic acid, laminin, agarose, gelatin, glucan and any combination thereof).

In some embodiments, the microsphere stock solution is a collagen solution containing cells (e.g., a collagen mixture solution). In a specific embodiment, bovine type I collagen and adipose-derived mesenchymal stem cells (ADSCs) are mixed as a core material of the cell-loaded microgel.

In some embodiments, the shear liquid is an oily solution with a certain biocompatibility (e.g., a mineral oil, an edible oil, a corn oil or a peanut oil).

In an embodiment not illustrated, multiple microfluidic units (e.g., eight independent microfluidic units) are simultaneously arranged in the chip body. Each of the microfluidic units has a pinched-flow focusing chip channel (e.g., the cross-shaped structure) for working simultaneously or alternatively. Preferably, the multiple microfluidic units are arranged in the same direction, or these microfluidic units are arranged at equal intervals.

The yield and the cell viability of the microdroplets obtained by using the microfluidic chip 140 are the results of a combined action of various parameters, such as the chip channel shape, the chip channel size, the fluid viscosity, and flow rate of the shear liquid and the microsphere stock solution. When the microdroplets are made of a certain raw material by employing the specific microfluidic chip 140, the properties in terms of the yield and the cell viability mainly depend on the flow rate control of the dispersed phase fluid (i.e., the microsphere stock solution in the embodiment) and the continuous phase fluid (i.e., the shear liquid in the embodiment).

In some preferred embodiments, the flow rate ratio of the microsphere stock solution to the shear liquid flowing in a single channel ranges from 1:10 to 1:20 at the intersection zone (e.g., 1:10, 1:11, 1:12, 1:13, 1:14, 1:15, 1:16, 1:17, 1:18, 1:19 and 1:20).

More preferably, the flow rate of the shear liquid flowing in the single channel ranges from 100µL/min to 400µL/min at the intersection zone (e.g., 100µL/min, 150µL/min, 200µL/min, 250µL/min, 300µL/min, 350µL/min or 400µL/min). The flow rate of the microsphere stock solution ranges from 10µL/min to 40µL/min at the intersection zone (e.g., 10µL/min, 15µL/min, 20µL/min, 25µL/min, 30µL/min, 35µL/min or 40µL/min).

For example, for the chip channel of the cross-shaped structure, the shear liquid is introduced into the second chip channels 142 on both sides of the first chip channel 141 at the flow rate ranging from 100µL/min to 400µL/min. Preferably, the flow rates of the microsphere stock solution flowing in the second chip channels 142 on both sides are consistent at the intersection zone to ensure the uniform force to form the microdroplets.

In some embodiments, the first chip channel 141 and the second chip channel 142 have rectangular cross sections with the length ranging from 0.7mm to 2mm and the width from 0.2mm to 0.6mm. For example, the first chip channel 141 and the second chip channel 142 form the cross-shaped structure. The microsphere stock solution enters from the first chip channel 141, and the shear liquid enters from two second chip channels 1412 on both sides of the first chip channel 141. Preferably, the viscosity ratio of the microsphere stock solution to the shear liquid ranges from 10:1 to 30:1.

In some embodiments, the viscosity of the microsphere stock solution ranges from 500cp to 3000cp. To prepare the microdroplets, the flow rates of the two phase fluids can be controlled. For example, the flow rate ratio of the microsphere stock solution to the shear liquid flowing in the single channel ranges from 1:10 to 1:20 at the intersection zone. More preferably, the flow rate of the microsphere stock solution flowing in each second chip channel 142 ranges from 10µL/min to 40µL/min at the intersection zone, and the flow rate of the shear liquid in each of the second chip channels 142 ranges from 100µL/min to 400µL/min at the intersection zone. The microdroplets with the diameter sizes ranging from 200µm to 400µm are obtained through the flow rate control applied to the microfluidic chip 140 of the embodiment.

According to simulation analysis, in the process of forming the microdroplets, the continuous phase fluid gradually coats the dispersed phase fluid at the cross intersection zone, and makes the dispersed phase fluid become an inverted-cone portion at the intersection zone. The fluid at the intersection zone continues to flow forwards under the pressure of the dispersed phase fluid, and the inverted-cone portion is gradually separated from a mainstream under a shear action of the continuous phase fluid, and the microdroplets are formed ultimately to flow downstream of the intersection zone. In that case, the microdroplets are still covered with a certain amount of continuous phase fluid. Meanwhile, the chip channel of the first chip channel 141 located downstream of the intersection zone is filled with the continuous phase fluid to drive the dispersed microdroplets to flow outwards.

According to experiments on the microfluidic chip 140 of the present disclosure and comparison with the related art, it can be seen that:
the microfluidic chip 140 of the embodiment is capable of improving the yield of microdroplets and ensuring the cell viability for the fluid of high-viscosity microsphere stock solution.

The formed microdroplets with the average sizes of 200µm, 220µm, 300µm and 350µm conform to the microdroplet size range (200µm to 400µm), and meet the requirements of 3D bio-printing in a better way. Moreover, the microdroplet size increases with the flow rate of the microsphere stock solution, and the microdroplet size decreases with the increasing flow rate of the shear liquid. The microdroplets at a desired size can be stably obtained by integrated control of the flow rates of the two phase fluids.

In the embodiment, the microsphere preparation material pumping device 120 is communicated with the first chip channel 141 and the second chip channel 142 through the bio-ink cartridge 131 to control the flow rate and amount of the microsphere stock solution entering the first chip channel 141 as well as the flow rate and amount of the shear liquid entering the second chip channel 142. The microsphere preparation material pumping device 120 can regulate the flow rate of the shear liquid and the microsphere stock solution by adjusting the pressures of the first accommodating chamber and the second accommodating chamber, which facilitates accurately controls the flow rates of the two phase fluids.

The buffer device 150 is arranged between the microfluidic chip 140 and the curing device 160 and comprises a buffer flow channel communicated with the chip outlet 1412 of the microfluidic chip 140 and the curing device inlet of the curing device 160, and the buffer flow channel is configured to buffer or guide the flow of the microdroplet-shear liquid mixture.

As shown in Figures 1, 2, 15 and 16, the buffer device 150 comprises a buffer body 151 which comprises a mixture receiving port 15111, a mixture output port 15121 and the buffer flow channel. The mixture receiving port 15111 is communicated with the chip outlet 1412 of the microfluidic chip 140 to receive the microdroplet-shear liquid mixture. The mixture output port 15121 is communicated with the curing device inlet to output the microdroplet-shear liquid mixture from the buffer device 150. The buffer flow channel is arranged between the mixture receiving port 15111 and the mixture output port 15121; and the buffer flow channel comprises a tapered segment with cross sectional area gradually decreasing from the mixture receiving port 15111 to the mixture output port 15121.

As shown in Figures 1, 2 and 13 to 16, the tapered segment comprises an arc flow channel with cross sectional area gradually decreasing from the mixture receiving port 15111 to the mixture output port 15121.

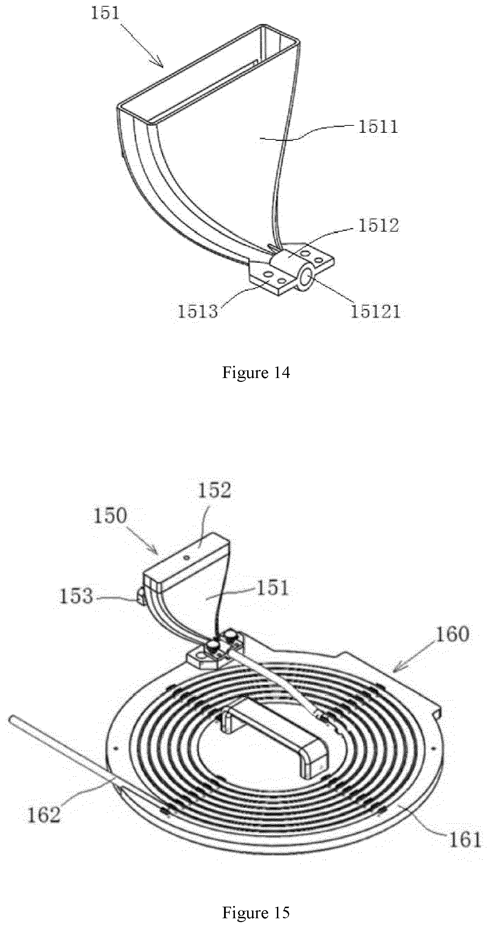

As shown in Figure 15, the buffer flow channel further comprises an equal cross-sectional segment located downstream of the tapered segment in the flow direction.

As shown in Figures 13 and 14, the buffer body 151 comprises a tapered segment body 1511 and an equal cross-sectional segment body 1512. A large-diameter end face of the tapered segment body 1511 has a square cross section, and a small-diameter end face thereof has a circular cross section, and the tapered segment flow channel gradually changes from the square cross section to the circular cross section. The equal cross-sectional segment body 1512 has a circular cross section. The small-diameter end face of the tapered segment body 1511 is connected with the equal cross-sectional segment body 1512. Preferably, an inner diameter of the small-diameter end face of the tapered segment body 1511 is equal to an inner diameter of the equal cross-sectional segment body 1512, and the tapered segment of the buffer flow channel is smoothly connected with the equal cross-sectional segment. The arrangement buffers the flow of the microdroplet-shear liquid mixture in a better way. The mixture output port 15121 is an outlet of the equal cross-sectional segment body 1512.

As shown in Figures 13 and 14, to fix the buffer device 150, the buffer body 151 further comprises a fixing portion 1513. The fixing portion 1513 includes two wing plates arranged on both sides of the equal cross-sectional segment body 1512, and each of the wing plates is provided with a mounting hole through which the screw is screwed into a threaded hole at a corresponding position on the microsphere preparation rack 110 to fix the buffer device 150.

In addition, to strengthen the buffer body 151, a reinforcing rib 1514 is additionally arranged at the bottom of an arc-shaped portion of the buffer body 151.

In the embodiment, the microfluidic chip 140 comprises multiple microfluidic units and multiple chip outlets 1412 thereof, wherein the mixture receiving port 15111 is in communication with the multiple chip outlets 1412 simultaneously. The arrangement allows the microdroplets formed by the microfluidic units to be fed into the same buffer device 150 for curing, collection and other processes without difference in the subsequent preparation process.

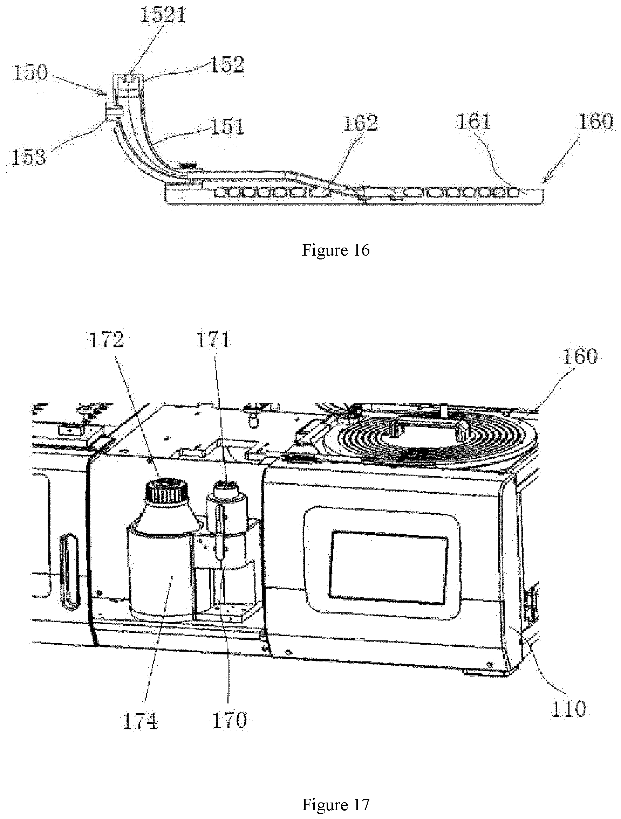

As shown in Figures 15 and 16, the buffer device 150 further comprises a third sealing structure 153 through which the mixture receiving port 15111 is directly connected with the chip outlet 1412 of the microfluidic chip 140 hermetically.

Two ends of the third sealing structure 153 are respectively communicated with one end of the chip outlet 1412 of the microfluidic chip 140 and the mixture receiving port 15111. Preferably, the third sealing structure 153 is made of a rubber material. The third sealing structure 153 forms a good seal with the chip outlet 1412 of the microfluidic chip 140 and the mixture receiving port 15111 of the buffer body 151 through the elastic deformation after being pressed.

As shown in Figure 13, to realize a good connection between the buffer body 151 and the third sealing structure 153, a mounting flange is additionally arranged on an outer edge of the mixture receiving port 15111 to adapt to an arc-shaped surface of the tapered segment body 1511. The mounting flange protrudes more from the bottom than the top relative to the arc-shaped surface, so that the mounting flange is perpendicular to the surface connected with the third sealing structure 153 after the buffer device 150 is fixed, thereby facilitating a good sealing connection with the third sealing structure 153.

In some embodiments, the buffer device 150 further comprises a shear liquid receiving port 1521 which receives the shear liquid made up to the buffer flow channel to maintain a level in the buffer flow channel equal to or above a level of the microdroplet-shear liquid mixture received at the mixture receiving port 15111.

As shown in Figure 16, in the embodiment, the buffer body 151 has a top with a top opening 15112 in communication with the buffer flow channel. The buffer device 150 comprises a buffer cover body 152 that covers the top opening 15112, and the shear liquid receiving port 1521 is arranged on the buffer cover 152.

The following technical effects can be realized by arrangement of the buffer device 150:
The microdroplets containing cells formed by employing the microfluidic chip 140 form the core spheres of the bio-blocks formed after curing and assembly with the shells. In the buffer device 150, the buffer flow channel is configured to receive the microdroplet-shear liquid mixture made by employing the microfluidic chip 140, and protects and buffers the uncured bio-block core sphere.

The microdroplet-shear liquid mixture is discharged to the buffer flow channel. Due to the difference in specific gravity, the microdroplets will settle in oil. The buffer flow channel is designed as the arc flow channel with gradually decreasing cross sectional area (i.e., an arc funnel shape) to prevent blockage at the mixture output port 15121 and guide a smooth flow.

The overall buffer flow channel is an arc-shaped flow channel, and the mixture receiving port 15111 is arranged on a side wall of the buffer body and directly and hermetically connected with the microfluidic chip 140 through the third sealing structure 153 to avoid arrangement of lead-out capillary pipes at the chip outlet 1412 of the microfluidic chip 140 for drainage, reduce the influence of resistance to the cells in the capillary pipes, and ensure the cell viability.

The shear liquid in the microsphere preparation device is circularly fed into the buffer flow channel through the shear liquid receiving port 1521 arranged on the buffer cover body 152 at the top of the buffer body 151. The level of the shear liquid in the buffer flow channel is maintained to be above the chip outlet 1412, so that the microdroplets formed by the shear action are directly discharged into the shear liquid acting as a buffer.

The outlet of the buffer flow channel is connected with the curing device inlet of the curing device 160, so that the buffered microdroplets are introduced into the curing device 160 by settlement.

The curing device inlet of the curing device 160 is connected with the chip outlet 1412 of the microfluidic chip 140 to receive the microdroplet-shear liquid mixture, and the microdroplets form the cured microspheres in the curing device. The curing device 160 of the embodiment further comprises a curing device outlet.

As shown in Figures 1, 2, 15 and 16, the curing device 160 comprises a curing flow channel connected between the curing device inlet and the curing device outlet, and the curing device inlet is communicated with the chip outlet 1412 of the microfluidic chip 140. In the embodiment, the curing device inlet is communicated with the chip outlet 1412 through the buffer device 150 to receive the microdroplet-shear liquid mixture. The microdroplets in the shear liquid form the cured microspheres when the microdroplet-shear liquid mixture flows through the curing flow channel, and a microsphere-shear liquid mixture is formed and output from the curing device outlet.

In some embodiments, the microsphere preparation device comprises a curing device temperature control unit which keeps the curing device 160 at a preset temperature or within a preset temperature range. The temperature of the microdroplet-shear liquid mixture is controlled by regulating the temperature of the curing device 160, thereby controlling the curing temperature to allow the microdroplets to be cured at an appropriate curing temperature to form the microspheres. For example, the curing device 160 is maintained at about 37°C to facilitate maintenance of the cell viability of the microdroplets and the microspheres, and accelerate the curing of the microdroplets to form the microspheres. The microsphere forms the bio-block after being assembled with at least one shell, and the microsphere coated by the shell is the core sphere of the bio-block.

In the embodiment, a second temperature zone is arranged in the microsphere preparation device. The curing device 160 is arranged in the second temperature zone, and the curing device temperature control unit adjusts the temperature of the curing device 160 and the mixture therein by regulating the temperature of the second temperature zone.

To realize the accurate temperature control of the curing device 160, the curing device temperature control unit comprises a curing device temperature measuring element which is configured to monitor the temperature of the curing device 160. For example, the curing device temperature measuring element can monitor the temperature of the curing device 160 by monitoring the temperature of the second temperature zone.

As shown in Figures 15 and 16, in some embodiments, the curing flow channel is a helical flow channel coiled outwards or inwards. The spiral flow channel enables a relatively long curing flow channel to be arranged in the effectively occupied space to fully cure the microdroplets.

As shown in Figure 16, in some embodiments, the curing flow channel comprises a tapered segment with a cross sectional area gradually decreasing from the curing flow channel inlet to the curing flow channel outlet. The arrangement enables the mixture in the curing flow channel to gradually stabilize at a preset curing temperature, thus facilitating the curing of the microdroplets.

In the embodiment, the curing device 160 comprises a coiled pipe 162 in which the curing flow channel is formed. The curing device inlet is an inlet of the coiled pipe 162, and the curing device outlet is an outlet thereof.

As shown in Figures 15 and 16, in the embodiment, the curing device 160 further comprises a disk body 161 which is provided with a helical groove coiled outwards or inwards, and the coiled pipe 162 is arranged in the helical groove. The arrangement enables the temperature of the curing flow channel to easily be controlled by regulating the temperatures of the disk body 161 and a groove wall of the helical groove, thus facilitating the curing control of the microdroplets.

In the embodiments of the present disclosure, the microdroplets are formed and cured into the microspheres in the shear liquid, so that the collection efficiency is improved and the subsequent operation efficiency is improved to improve the preparation efficiency of the microspheres. In the embodiments of the present disclosure, the microdroplets are cured in the shear liquid of oil phase and positioned on a liquid-liquid interface, the shear liquid forms a system maintaining the shape of the microdroplets to obtain relatively perfect spheres, thereby forming improved sphere-like microspheres. Therefore, the microspheres are improved in the consistency of the shape, and the quality of the bio-printing is perfected.

In the embodiment, during the curing process, since the microdroplets are completely immersed in the shear liquid with the same coefficient of heat conductivity, the microdroplets share the same temperature of surrounding shear liquid, so that the surfaces of the microdroplets are evenly heated in the shear liquid at the same curing rate as a whole. In this way, the biological activity is ensured for the microspheres containing the bioactive substances.

The collection device 170 is located downstream of the curing device 160 and configured to collect the microsphere-shear liquid mixture discharged from the curing device outlet. As shown in Figures 1, 2 and 17, the collection device 170 comprises a mixture collection container 171 and a shear liquid collection container 172.

In some embodiments, the microsphere preparation device comprises a collection device temperature control unit which keeps the collection device 170 at the preset temperature or within the preset temperature range. For example, the collection device can be maintained at a temperature from 37°C to 40°C to maintain the microspheres and the bio-blocks, assembled with a microsphere as core sphere, at a high activity.

In the embodiment, the microsphere preparation device is provided with a third temperature zone in which all collection containers of the collection device 170 are arranged, and the collection device temperature control unit adjusts the temperature of the collection device 170 and the mixture or the shear liquid therein by regulating the temperature of the third temperature zone.

To realize the accurate temperature control of the collection device 170, the collection device temperature control unit comprises a collection device temperature measuring element which is configured to monitor the temperature of the collection device 170. For example, the collection device temperature measuring element can monitor the temperature of the collection device 170 by monitoring the temperature of the third temperature zone.

The mixture collection container 171 comprises a mixture receiving port and a shear liquid output port. The mixture receiving port of the mixture collection container 171 is communicated with the curing device outlet to receive the microsphere-shear liquid mixture. The shear liquid collection container 172 comprises a shear liquid receiving port 1521. The shear liquid receiving port 1521 is communicated with the shear liquid output port of the mixture collection container 171 for receiving the shear liquid therefrom.

In addition, as shown in Figure 1, the collection device 170 further comprises a shear liquid circulating pump 173 with an inlet thereof in communication with the shear liquid collection container 172 and an outlet thereof in communication with the buffer flow channel. The shear liquid circulating pump 173 injects the shear liquid from the shear liquid collection container 172 back into the buffer flow channel of the buffer device 150 to guarantee the level of the shear liquid in the buffer flow channel. Optionally, the shear liquid circulating pump 173 can be a peristaltic pump.

The shear liquid circulating pump 173 is a power component configured to circulate the shear liquid from the mixture collection container 171 to the buffer flow channel in the microsphere preparation device. Due to different densities between the microspheres and the shear liquid, the microspheres naturally settle in the shear liquid in the mixture collection container 171, and a shear liquid layer is formed at an upper layer of the mixture collection container 171. With negative pressure generated by the peristaltic pump, the shear liquid in the upper layer of the mixture collection container 171 is introduced into the buffer flow channel by the shear liquid collection container 172 for recycling.

To prevent backflow of the shear liquid from impacting the buffer flow channel, the shear liquid circulating pump 173 should not run at a high rate. Preferably, the pump rate should ensure that the level in the buffer flow channel is higher than the level of the microdroplet-shear liquid mixture at the mixture receiving port 15111. When the microfluidic chip 140 is directly connected with the mixture receiving port 15111 through the third sealing structure 153, the level in the buffer flow channel is preferably above the chip outlet 1412 of the microfluidic chip 140.

As shown in Figures 1 and 2, the shear liquid circulating pump 173 is mounted on the microsphere preparation rack 110 and located between the collection device 170, the bio-ink cartridge assembly 130 and the microfluidic chip 140 in the transverse direction (i.e., the anterior-posterior direction as shown in Figures 1 and 2).

In the embodiment, the mixture receiving port and the shear liquid output port of the mixture collection container 171 are located at the top of the mixture collection container 171. The mixture collection container 171 receives the microsphere-shear liquid mixture output from the curing device outlet through a tube inserted in the mixture receiving port to a depth close to the bottom of the bottle. The mixture collection container 171 outputs the shear liquid through a tube inserted in the shear liquid output port to an upper portion of the mixture collection container 171. When the level of the shear liquid gradually rises in the mixture collection container 171, the cured microspheres sink at the bottom under the action of gravity, and the upper shear liquid is sucked into the shear liquid collection container 172 under the action of the peristaltic pump.

To accurately control the temperature of the collection device 170, the collection device 170 further comprises a thermal insulation structure 174 arranged outside the mixture collection container 171 and the shear liquid collection container 172.

The monitoring device 180 is configured to monitor the preparation process of the microspheres. As shown in Figures 1 and 18 to 20, in some embodiments, the monitoring device 180 comprises a position-adjustable camera 183.

The camera 183 is configured to observe the preparation process of the microspheres. With imaging by the camera 183, the size, dimensions, uniformity and formation efficiency of the microdroplets generated in the microfluidic chip 140 are observed in real time. Parameters such as pressure and temperature of relevant components can be adjusted according to the observation results to prepare the microspheres in different sizes and containing different types of cells.

The camera 183 is relocated by using a position adjusting bracket. As shown in Figure 1, the camera 183 is mounted on the position adjusting bracket.

The position adjusting bracket comprises a first bracket body 181 and a second bracket body 182 connected therewith. The first bracket body 181 is rotatably mounted on the microsphere preparation rack 110. A portion of the first bracket body 181 connected with the second bracket body 182 is provided with a vertical elongated slot through which a first adjusting screw 184 is connected onto the second bracket body 182, so that the second bracket body 182 and the camera 183 thereon are vertically relocated by adjusting the position of the first adjusting screw 184 relative to the vertical elongated slot. The second bracket body 182 is provided with a circumferential elongated slot through which a second adjusting screw 185 is fixedly connected with the camera 183. The camera 183 is circumferentially relocated by adjusting the position of the second adjusting screw 185 relative to the circumferential elongated slot. Therefore, the camera 183 realizes a large azimuth rotation by rotating the first bracket body 181 relative to the microsphere preparation rack 110, and supports fine adjustment in the vertical direction through the first adjusting screw 184 as well as the fine adjustment in the transverse direction through the second adjusting screw 185 to ensure accurate imaging.

In some embodiments, the microsphere preparation device further comprises a control device for controlling the microsphere preparation process. The control device can be coupled with the microsphere preparation material pumping device 120, the microsphere preparation material temperature control unit, the curing device temperature control unit, the collection device temperature control unit, the shear liquid circulating pump 173 and the monitoring device 180 to control the microsphere preparation process and realize automatic production of the microspheres.

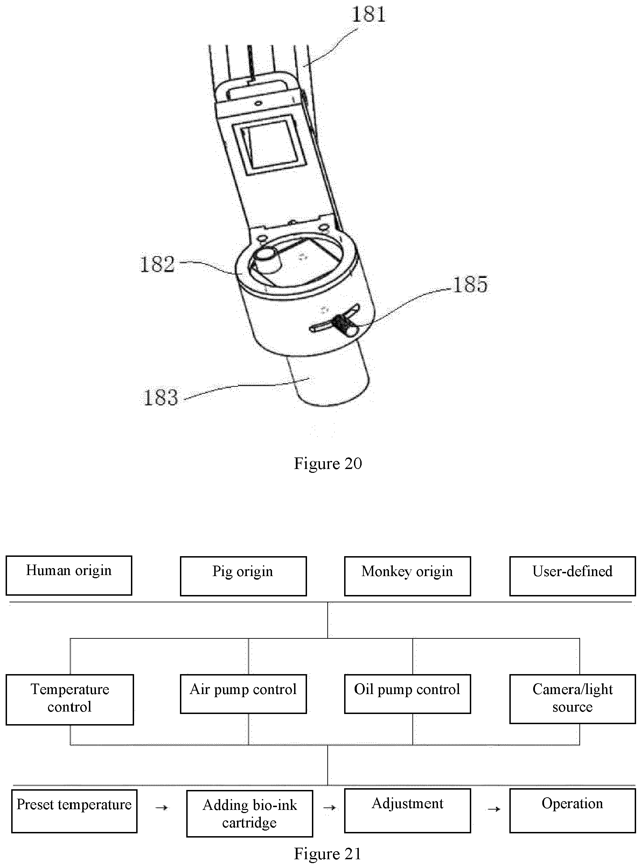

The process of preparing the microspheres by using the microsphere preparation device of the embodiment is described below in combination with Figure 21.

The microsphere preparation device of the embodiment is capable of preparing the microspheres for cells derived from different origins, and the formed microspheres are used as the core spheres of the bio-blocks. For example, the microspheres are formed for the cells derived from human, swine, monkey or custom origin. The pressure and other control parameters used in preparation of the microspheres are vary with the cells derived from different origins, because the cells derived from different origins are different in sizes and bearable to different shear forces. The preparation of the microspheres for the cells derived from monkey is taken as an example to illustrate the method for preparing core spheres of the bio-blocks. The methods for preparing the bio-blocks for the cells derived from other origins are similar to that for the cells derived from the monkey origin. The pressure and other control parameters are various due to different cell sizes, types, bearable shear forces and microsphere sizes, but the parameter ranges are substantially consistent. The preparation process comprises:
1. Preparation. The cells derived from monkey are incubated to form a cell solution which is then transferred to one chamber (e.g., the second accommodating chamber 1314) of the bio-ink cartridge 131 as the microsphere stock solution, and the other chamber (e.g., the first accommodating chamber 1313) of the bio-ink cartridge 131 is filled with sufficient oil as the shear liquid.
2. The microsphere preparation device is started for warming up, and the bio-ink cartridge 131 is placed in the ink cartridge mounting position after all temperature zones are detected to reach the preset temperature or the preset temperature range.
3. The microsphere preparation material pumping device 120 (particularly, an air pump as shown in Figure 21) is started up and a pipe line switch on the first pipe line 191 connected with the first accommodating chamber 1313 where the shear liquid flows is turned on, the shear liquid is driven to flow in the microsphere preparation device at a certain flow rate, and the chip outlet 1412 is kept below the shear liquid level in the buffer flow channel.
4. When all temperature zones of the microsphere preparation device are detected to reach an appropriate temperature, the preparation of the microspheres in a preset size is started. When the microspheres are prepared, the pipe line switch on the first pipe line 191 connecting the microsphere preparation material pumping device 120 with the first accommodating chamber 1313 where the shear liquid flows is turned on; meanwhile, the pipe line switch on the first pipe line 192 connecting the microsphere preparation material pumping device 120 with the second accommodating chamber 1314 where the microsphere stock solution flows is turned on; the flow rate of the microsphere stock solution is controlled within 2ml/h and the flow rate of the shear liquid controlled within 40ml/h; the microdroplets are prepared in the eight microfluidic units of the microfluidic chip 140 simultaneously, and the pumping pressure of the microsphere preparation material pumping device 120 is finely tuned according to the microdroplet preparation observed through the camera 183 located at the top of the microfluidic chip 140. When the camera 183 is used for observation, the camera 183 and the light source are adjusted as required.
5. The pressure value of the shear liquid circulating pump 173 (particularly, the oil pump as shown in Figure 21) is set according to the operation of the microsphere preparation device (e.g., the level of the buffer flow channel and the pumping pressure of the microsphere preparation material pumping device 120). For example, if the pressure value of the shear liquid circulating pump 173 ranges from 0 mbar to 2048 mbar, the pressure value of the shear liquid circulating pump 173 is 0 when necessary. The shear liquid circulating pump 173 can be adjusted at low speed, medium speed and high speed.
6. The prepared microspheres are collected in the collection device 170.

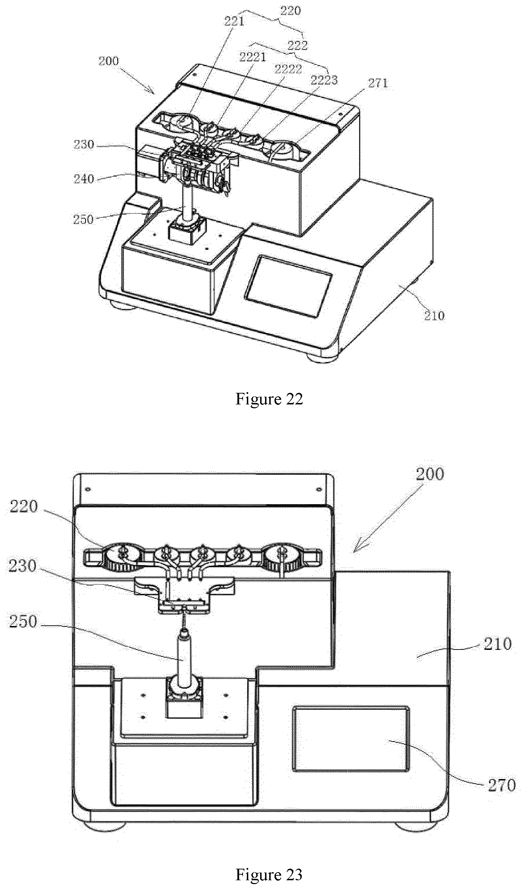

Figure 22 to Figure 31 show the structures of the shell assembly device 200 and the components thereof according to the embodiments of the present disclosure.

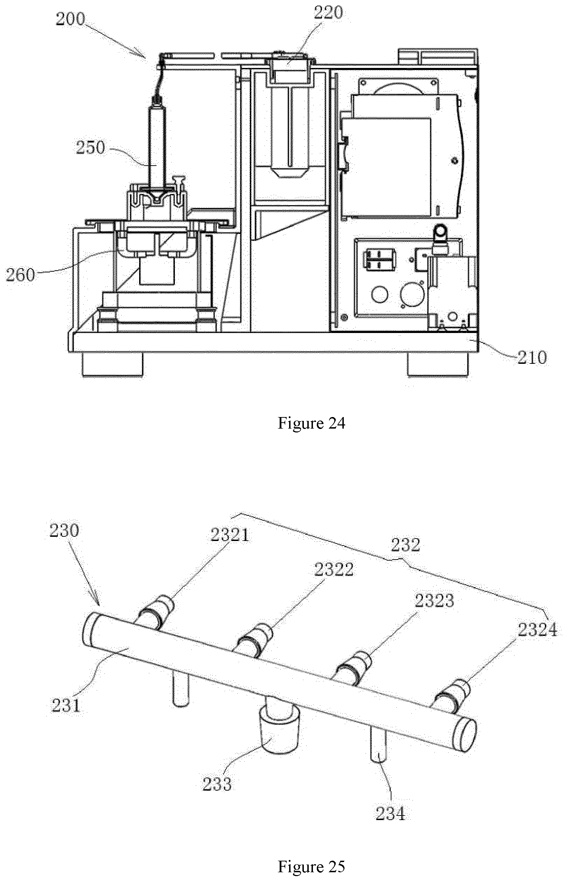

The shell assembly device 200 is configured to coat microspheres or compositional structures composed of a microsphere and at least one shell. As shown in Figures 22 to 31, the shell assembly device 200 of the embodiment comprises a shell assembly rack 210, the shell preparation material pumping device, the shell preparation material storage device 220, a fluid switching tube 230, a switching control device 240, an assembling device 250 and an oscillation device 260. In the embodiment as shown in Figure 22, the shell assembly device 200 further comprises the switching control device 240. In the embodiment as shown in Figures 23 and 24, a tube clamp is arranged to replace the switching control device 240 and perform the fluid switching function.

The shell preparation material storage device 220 comprises multiple storage spaces which comprise a cleaning liquid storage space for storing a cleaning liquid and a shell liquid storage space for storing a shell liquid.

As shown in Figure 22, in the embodiment, the shell preparation material storage device 220 comprises a cleaning liquid container 221 and a shell liquid storage container group 222. In the embodiment, the cleaning liquid container 221 comprises a cleaning liquid bottle in which the space forms the cleaning liquid storage space. The shell liquid storage container group 222 comprises three shell liquid storage bottles, namely, a first shell liquid storage bottle 2221, a second shell liquid storage bottle 2222 and a third shell liquid storage bottle 2223. The three shell liquid storage bottles respectively store the shell liquid required by different shells coating each of the microspheres. The space of each of the shell liquid storage bottles forms the shell liquid storage space. The three shell liquid storage bottles store the same shell liquid material or different shell liquid materials.

Apparently, in some embodiments, two or more than two cleaning liquid storage spaces are arranged for cleaning, and different cleaning liquid storage spaces can store the same cleaning liquid or different cleaning liquids. One or two shell liquid storage spaces or more than four shell liquid storage spaces can be arranged due to the number of shells of the bio-block and other reasons, and different shell liquid storage spaces store the same shell liquid or different shell liquids.

The shell preparation material pumping device drives the cleaning liquid and the shell liquid to flow. The shell preparation material pumping device can use various active pumping devices or passive pumping devices (e.g., the pressure pump, the peristaltic pump and the syringe pumps).

As shown in Figures 22 to 25, the fluid switching tube 230 comprises a tube body 231 as well as multiple inlet connectors and an outlet connector 233 arranged on and communicated with the tube body 231, the multiple inlet connectors comprise a cleaning liquid inlet connector connected with the cleaning liquid storage space and a shell liquid inlet connector connected with the shell liquid storage space, and the outlet connector 233 is in communication with the assembling space of the assembling device 250.

As shown in Figure 25, in the embodiment, multiple inlet connectors form an inlet connector group 232. Corresponding to the cleaning liquid storage space and the three shell liquid storage spaces, the inlet connector group 232 comprises one cleaning liquid inlet connector 2321 connected with the cleaning liquid storage space and three shell liquid inlet connectors connected with the shell liquid storage space. The three shell liquid inlet connectors include a first shell liquid inlet connector 2322 connected with the first shell liquid storage bottle 2221, a second shell liquid inlet connector 2323 connected with the second shell liquid storage bottle 2222 and a third shell liquid inlet connector 2324 connected with the third shell liquid storage bottle 2223.

The fluid switching tube 230 is arranged to enable the cleaning liquid storage spaces and the shell liquid storage spaces to be communicated with the assembling space of the assembling device 250 in a switchable way, so that the required cleaning liquid or the required shell liquid is selectively introduced into the assembling space without reconnection of these pipe lines for each cleaning or shell coating, thereby improving the shell assembly efficiency.

As shown in Figure 25, the fluid switching tube 230 further comprises a plug-in structure 234 arranged outside the tube body for fixing the fluid switching tube 230. Correspondingly, the shell assembly rack 210 is provided with a socket matched with the plug-in structure 234. The arrangement of the plug-in structure 234 enables the fluid switching tube 230 to be quickly positioned and fixed on the shell assembly rack 210.

In some embodiments, the multiple inlet connectors are arranged side by side on the tube body 231 and located on a side of the shell preparation material storage device 220, and the outlet connector 233 is arranged at an angle with the multiple inlet connectors and on a side of the tube body 231 far away from the inlet connectors with an offset from the vertical direction. The arrangement enables the outlet connector 233 to reduce or avoid an interference with operations involving the assembling tube 251 of the assembling device 250, including taking and arranging the assembling tube 251.

As shown in Figure 22, the switching control device 240 selectively enables one of the multiple inlet connectors of the fluid switching tube 230 to communicate with the corresponding storage space while the other inlet connectors are disconnected from the corresponding storage spaces.

As shown in Figures 22, 26 and 27, the switching control device 240 comprises a rotating shaft 242 and multiple control cams arranged on the rotating shaft 242 in a non-rotatable way; the multiple control cams are arranged one-to-one corresponding to the multiple inlet connectors; and the rotating shaft 242 rotates to change rotation angles of the multiple control cams to control on-off states of the pipe lines communicated with the corresponding inlet connectors.

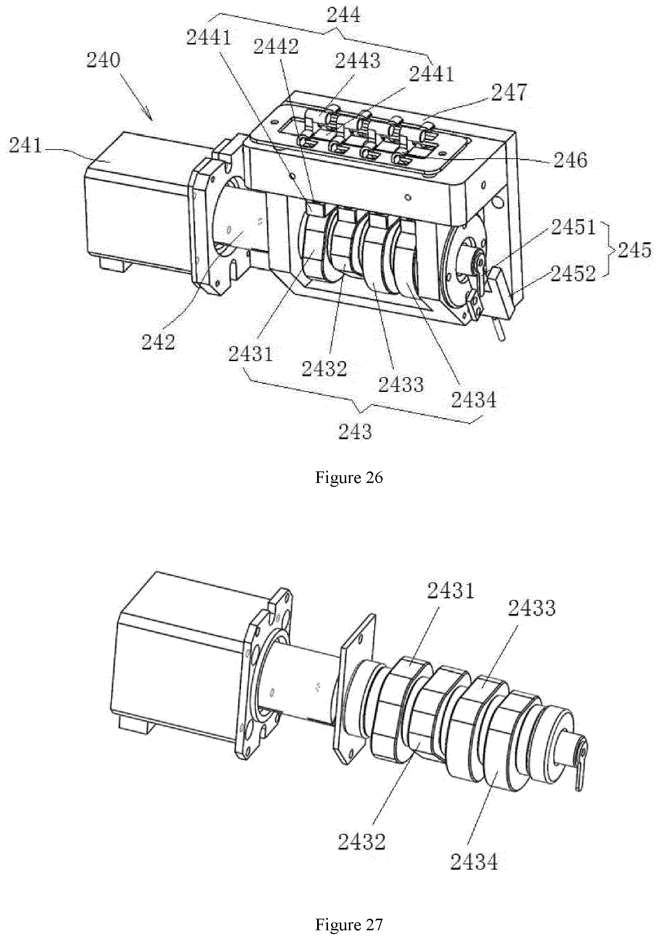

As shown in Figures 26 and 27, the multiple control cams comprise at least two control cams having identical cam surface profile and mounted at different angles, or comprise at least two control cams having different cam surface profiles.

In the embodiment, a control cam group 243 including multiple control cams comprises four control cams, namely, a first control cam 2431, a second control cam 2432, a third control cam 2433, and a fourth control cam 2434. For the four control cams, the first control cam 2431 and the third control cam 2433 have the identical cam surface profile but differ in phase by 144 degrees, the second control cam 2432 and the fourth control cam 2434 have the identical cam surface profile but differ in phase by 144 degrees, while the cam surface profiles of the first control cam 2431 and the third control cam 2433 are different from the cam surface profiles of the second control cam 2432 and the fourth control cam 2434.

The control principle of the switching control device 240 of the embodiment is illustrated as follows:
When the phase of the rotating shaft is at zero degree, corresponding controlled pipe line or pipe fitting to be closed. In that case, all storage spaces are not communicated with the corresponding assembling spaces.

When the phase of the rotating shaft is at 72 degrees, the first control cam 2431 rotates to a position where the corresponding controlled pipe line or pipe fitting is opened, and the each of remaining control cams is at a position where the corresponding controlled pipe line or pipe fitting is closed. At this time, the cleaning liquid storage space in the cleaning liquid storage bottle 221 is communicated with the cleaning liquid inlet connector 2321, while each of the shell liquid storage spaces is disconnected from the corresponding shell liquid inlet connector.

When the phase of the rotating shaft is at 144 degrees, the second control cam 2432 rotates to a position where the corresponding controlled pipe line or pipe fitting is opened, and the each of remaining control cams is at a position where the corresponding controlled pipe line or pipe fitting is closed. At this time, the shell liquid storage space in the first shell liquid storage bottle 2221 is communicated with the first shell liquid inlet connector 2322, while the cleaning liquid storage space in the cleaning liquid storage bottle 221 is disconnected from the cleaning liquid inlet connector 2321, and the remaining shell liquid storage spaces are disconnected from the corresponding shell liquid inlet connectors.

When the phase of the rotating shaft is at 216 degrees, the third control cam 2433 rotates to a position where the corresponding controlled pipe line or pipe fitting is opened, and the each of remaining control cams is at a position where the corresponding controlled pipe line or pipe fitting is closed. At this time, the shell liquid storage space in the second shell liquid storage bottle 2222 is communicated with the second shell liquid inlet connector 2323, while the cleaning liquid storage space in the cleaning liquid storage bottle 221 is disconnected from the cleaning liquid inlet connector 2321, and the remaining shell liquid storage spaces are disconnected from the corresponding shell liquid inlet connectors.

When the phase of the rotating shaft is at 288 degrees, the fourth control cam 2434 rotates to a position where the corresponding controlled pipe line or pipe fitting is opened, and the each of remaining control cams is at a position where the corresponding controlled pipe line or pipe fitting is closed. At this time, the shell liquid storage space in the third shell liquid storage bottle 2223 is communicated with the third shell liquid inlet connector 2324, while the cleaning liquid storage space in the cleaning liquid storage bottle 221 is disconnected from the cleaning liquid inlet connector 2321, and the remaining shell liquid storage spaces are disconnected from the corresponding shell liquid inlet connectors.

When the phase of the rotating shaft is at 360 degrees, which means it returns to 0 degree, each of the four control cams is again at a position where the corresponding controlled pipe line or pipe fitting is closed. Each of the storage spaces is not communicated with the assembling space.

Therefore, once the rotating shaft 242 is controlled at a proper angle, the pipe line or pipe fitting can be opened as required, thus opening the flow path corresponding to the pipe line or pipe fitting.

As shown in Figure 22, the inlet connector is connected with the corresponding storage space via a hose in the embodiment. The switching control device 240 also comprises multiple transmission units 244 which are arranged one-to-one corresponding to the multiple inlet connectors. The transmission unit 244 comprises a stopper 2443 and a moving member 2441. The moving member 2441 is located between the stopper 2443 and the corresponding control cam. The hose is located between the stopper 2443 and the moving member 2441 of the corresponding transmission unit 244. By changing the angle of the control cam, a gap between the moving member 2441 and the stopper 2443 is adjusted to open or close the hose.

As shown in Figure 26, the transmission unit 244 further comprises an elastic element 2442 which applies an acting force toward the cam surface to the moving member 2441. The elastic element 2442 is arranged to increase control stability of the switching control device 240.

In the embodiment, the switching control device 240 comprises a mounting base 247, the rotating shaft 242 and the control cam group 243 are mounted on the mounting base 247 and rotatable relative to the mounting base 247. The stopper 2443 is an L-shaped member. The L-shaped member comprises a first segment and a second segment connected at an angle to each other, one end of the first segment away from the second segment is fixed on the mounting base 247, an interval is arranged between the second segment and the mounting base 247 to arrange the corresponding hose.

The moving member 2441 is partially arranged in a through hole of the mounting base 247, and located below the second segment of the stopper 2443. One end of the moving member 2441 away from the stopper 2443 presses against the surface of the corresponding control cam.

In the embodiment, the elastic element 2442 is a spiral spring, and the moving member 2441 comprises a stop mating portion located at an end of the stopper 2443, a cam mating portion located at an end of the control cam and a spring mounting portion connected between the stop mating portion and the cam mating portion. The elastic element 2442 is sleeved on the spring mounting portion. Two ends of the elastic element 2442 presses against the mounting base 247 and the cam mating portion respectively.

As shown in Figure 26, the switching control device 240 further comprises a limit structure 246 for defining position of the hose. In the embodiment, the limit structure 246 comprises hooked structures respectively arranged at the front side and rear side of the stopper 2443.

As shown in Figure 26, the switching control device 240 further comprises a limit sensing device 245 by which the rotation angle of the rotating shaft 242 is limited.

The limit sensing device 245 comprises an angle indicating element 2451 and a limit element 2452 which are fixedly connected onto the rotating shaft 242, and the rotation angle of the rotating shaft 242 can be limited by limiting the rotation angle of the angle indicating element 2451.

The switching control device 240 further comprises a driving motor 241. The rotating shaft 242 is connected onto a driving shaft 242 of the driving motor 241 and driven by the driving motor 241.

The storage spaces in communication with the assembling space of the assembling device 150 can be randomly switched by the switching control device 240, which can avoid a step of replacing the storage space connected with the assembling space and improve the shell assembly efficiency.

It should be noted that the switching control devices of the shell assembly device and the bio-block preparation instrument having the shell assembly device of the present disclosure are not limited to the form of the switching control device 240 aforementioned. For example, the fluid switching tube 220 can be connected with/disconnected from each of the storage spaces by a solenoid valve, a tube clamp or other switching control devices (see embodiments shown in Figure 23 and Figure 24).

In addition, it is not necessarily to use the switching control device 240 of the present disclosure together with the fluid switching tube 230. In some embodiments, similar switching effect can be achieved by providing multiple connecting tubes in direct connection with the assembling space of the assembling device 150 and controlling the connection/disconnection of each connecting tube by the switching control device 240.

The assembling device 250 is configured to assemble a shell around each of the microsphere or the compositional structures composed of a microsphere and at least one shell.

In the embodiment, cleaning liquid cleans the microspheres or the compositional structures in the assembling device 250, wherein the cleaned microspheres or compositional structures in the assembling device 250 are coated with the shell liquid to form an outer shell around each of the microspheres or compositional structures.

As shown in Figures 22 to 24, Figures 28 to 31, the assembling device 250 comprises an assembling container and a filter element 252, and the microspheres are subject to cleaning and shell assembly in an assembling space defined by the assembling container and the filter element 252.

In the embodiment, the assembling device 150 comprises a single assembling space in which the microspheres or the compositional structures are cleaned with the cleaning liquid, and then coated by the shell liquid to form a shell around each of the microspheres or compositional structures.

In some embodiments, the assembling device can comprise a partitioned assembling space, and different steps of the shell assembly are completed in different partitions. For example, the microspheres or the compositional structures composed of a microsphere and at least one shell can be cleaned with the cleaning liquid in one or several partitions. The shell assembly step of forming the shell covering each of the microspheres or the compositional structures can be completed in one or several other partitions. Cleaning steps during different stages can be completed in one partition or different partitions. The shell assembly step during different stages can be completed in one partition or different partitions.

In the embodiment, the assembling container comprises an assembling tube 251 in which the assembling space is formed; the assembling tube 251 has a liquid receiving port 2511 at an upper end thereof and an opening 2512 at a lower end, and the filter element 252 is arranged at the bottom of the opening 2512.

The filter element 252 is connected hermetically with the assembling tube 251 to ensure filtration effect.

The bio-block or the intermediate structure thereof can be separated from the liquid waste via the filter element 252 arranged at the bottom of the assembling tube 251.

In the embodiment, the assembling space defined by the assembling tube 251 and the filter element 252 can either be used as a cleaning space of the microspheres or the compositional structures or a shell assembly space, thus reducing the potential losses of activity and raw materials when the microspheres or the compositional structures are transferred.

The assembling tube 251 is an open-end container having a liquid receiving port 2511 at the upper end and an opening 2512 at the lower end. After the shell assembly is completed to form a bio-block, the formed bio-block product can be enclosed in the assembling tube 251 by covering the liquid receiving port 2511 with a sealing plug, inverting the assembling tube 251, disassembling the assembling tube 251 and the filter element 252 and placing a piston in the opening 2512. When the bio-block needs to be taken out, the bio-block can be pushed out of the liquid receiving port 2511 by the piston. Therefore, the assembling tube 251 facilitates the temporary storage and removal of the formed bio-block.

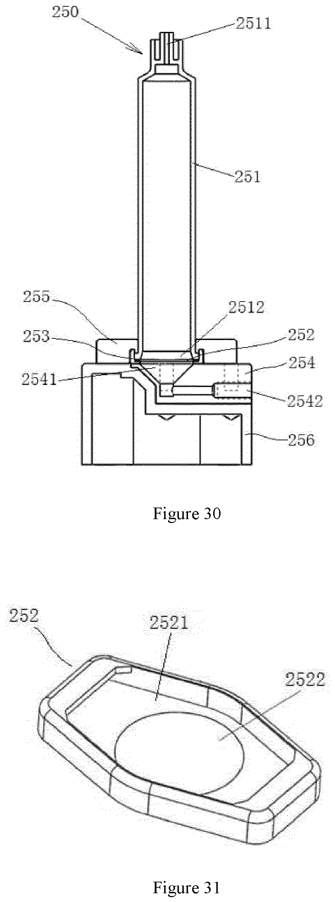

As shown in Figure 30, the assembling device 250 further comprises a receiving seat 254 which comprises a liquid receiving space, the assembling tube 251 and the filter element 252 are arranged on the receiving seat 254 and located above the liquid receiving space. The liquid receiving space can receive the cleaning liquid or the shell liquid separated from the filter element 252.

As shown in Figure 30, in the embodiment, the liquid receiving space comprises a funnel-shaped space 2541. The arrangement of the funnel-shaped space 2541 is conducive to saving the cleaning liquid and the shell liquid during the cleaning and shell assembly, and drainage of the liquid waste.

In some embodiments, the receiving seat 254 further comprises a drainage flow channel 2542 in communication with the liquid receiving space. The liquid waste can be drained out of the liquid receiving space via the drainage flow channel 2542.

The shell assembly device 200 further comprises a suction device and a liquid waste storage container 271. The suction device is connected between the drainage flow channel 2542 and the liquid waste storage container 271 to suck the liquid in the liquid receiving space to the liquid waste storage container 271. A suction device is arranged to speed up the shell assembly process.

As shown in Figure 31, the filter element 252 of the embodiment comprises a tube seat 2521 and a strainer 2522, the tube seat 2521 is mounted on the receiving seat 254 and has a central through hole; the strainer 2522 is mounted on the tube seat 2521 and located in the central through hole thereof; the assembling tube 251 is mounted in the tube seat 2521 and located above the strainer 2522; the opening 2512 of the assembling tube 251 is faced to the central through hole of the tube seat 2521, thus allowing the liquid in the assembling space of the assembling tube 251 to flow to the liquid receiving space under the strainer 2522 via the strainer 2522. Thanks to the structure of the filter element 252, the assembling tube 251 and the filter element 252 have a stable structure and accurate positioning after assembly.

The assembling device 250 further comprises a gland 255 which comprises a central through hole; the assembling tube 251 is matched with the central through hole of the gland 255; the gland 255 covers above the tube seat 2521, and fixes the assembling tube 251, the filter element 252 and the tube seat 2521 in the receiving seat 254.

In some embodiments, the tube seat 2521 is made of an elastic material (such as rubber). When the gland 255 is mounted in place, the tube seat 2521 is deformed due to pressure, thus achieving a good sealing connection with the assembling tube 251.

In addition, the assembling device 250 further comprises a transitional connection seat 256 on which the receiving seat 254 is mounted. The transitional connection seat 256 and the receiving seat 254 are arranged separately in the embodiment and can be arranged integrally in some embodiments.

The oscillation device 260 is configured to shake the cleaning liquid and/or the shell liquid when the microspheres or the compositional structures are cleaned or the shells are assembled. The design of the oscillation structure facilitates the rapid cleaning of the microspheres or the compositional structures, and even and rapid shell assembly.

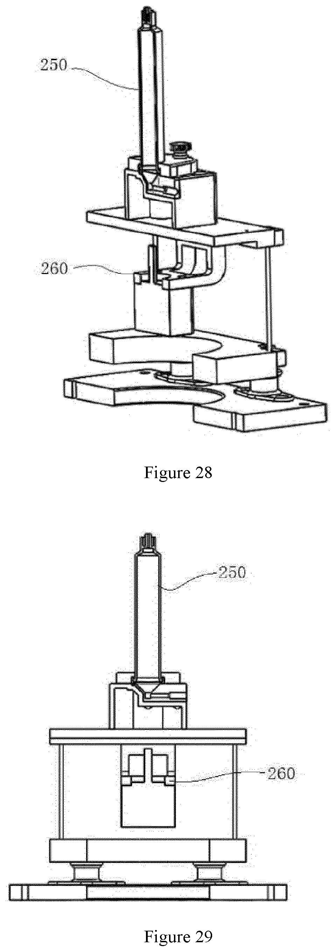

As shown in Figures 28 and 29, in the embodiment, the oscillation device 260 is mounted at the bottom of the assembling device 250 and connected with the transitional connection seat 256. The oscillation device 260 shakes the cleaning liquid or the shell liquid in the assembling space by applying an alternating force to the transitional connection seat 256.

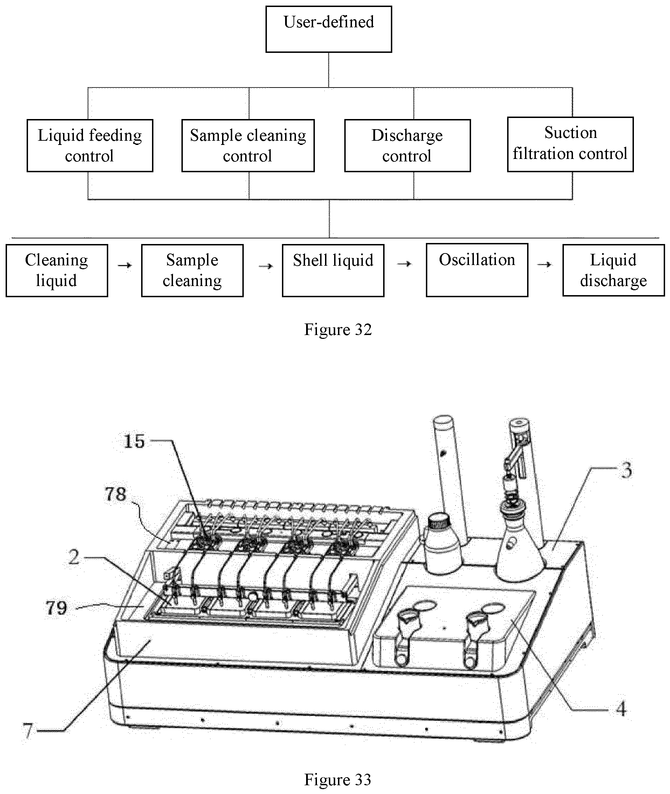

The shell assembly device of the embodiment can perform shell assembly for the microspheres or the compositional structures. With reference to Figure 32, the following illustrates the process of assembling the shells by using the shell assembly device of the embodiment with sequentially assembling each of the microsphere into three shells in layers as an example.
1. The microspheres are loaded into the assembling tube 251 after being separated from the shear liquid. The assembling tube 251 and the filter element 252 are assembled and jointly mounted on the shell assembly device 200. The microspheres and the shear liquid can be separated, for example, through centrifugal separation. To this end, the shell assembly device 200 may further comprise a separating unit for separating the microspheres from the shear liquid.
2. A proper amount of the cleaning liquid is added according to the amount of the microspheres in the assembling tube 251, and then the oscillation device 260 is turned on, for example, at an oscillation speed, lower than 1000rpm within 1000 seconds (for example). After cleaning, the suction device is turned on to discharge the liquid waste.
3. The shell liquid in the first shell liquid storage bottle 2221 is added to the assembling space of the assembling tube 251, and then the oscillation device 260 is turned on, for example, at an oscillation speed lower than 1000rpm within 1000 seconds. After the first shell is assembled around each of the microspheres, the remaining shell liquid in the assembling space is discharged. The cleaning liquid is added again to the assembling space, and then the oscillation device 260 is turned on, for example, at an oscillation speed lower than 1000rpm within 1000 seconds. After the cleaning, the suction device is turned on to discharge the cleaning liquid.
4. The shell liquid in the second shell liquid storage bottle 2222 is added to the assembling space of the assembling tube 251, and then the oscillation device 260 is turned on, for example, at an oscillation speed lower than 1000rpm within 1000 seconds. After the first shell is assembled around each of the microspheres, the remaining shell liquid in the assembling space is discharged. The cleaning liquid is added again to the assembling space, and then the oscillation device 260 is turned on, for example, at an oscillation speed lower than 1000rpm within 1000 seconds. After the cleaning, the suction device is turned on to discharge the cleaning liquid.
5. The shell liquid in the third shell liquid storage bottle 2223 is added to the assembling space of the assembling tube 251, and then the oscillation device 260 is turned on, for example, at an oscillation speed lower than 1000rpm within 1000 seconds. After the third shell is assembled around each of the microspheres, the remaining shell liquid in the assembling space is discharged. The cleaning liquid is added again to the assembling space, and then the oscillation device 260 is turned on, for example, at an oscillation speed lower than 1000rpm within 1000 seconds. After the cleaning, the suction device is turned on to discharge the cleaning liquid.
6. A sealing cover is placed on the liquid receiving port 2511 of the assembling tube 251; the assembling tube 251 and the filter element 252 are disassembled; the filter element 252 is removed from the assembling tube 251; and then a piston is added to the assembling tube 251 at the opening 2512 to block the assembling tube 251, thus obtaining the bio-block product enclosed in the assembling tube 251.

When assembling and cleaning different shells, the oscillation speed and time of the oscillation device 260 can be adjusted as required. For example, when the microspheres or the compositional structures are easy to clean or assemble, the oscillation speed and time of the oscillation device 260 can be reduced and shortened respectively. On the contrary, when the microspheres or the compositional structures are not easy to clean or assemble, the oscillation speed and time can be increased and prolonged respectively
In some embodiments, the shell assembly device further comprises a control device for controlling the shell assembly process. The control device can be coupled with the shell preparation material pumping device, the suction device, the switching control device and the oscillation device 260 to control the shell assembly process, thus achieving the automated production of the shell assembly.

The embodiments of the present disclosure further provide the switching control device 240 which controls the on-off states of the multiple flow channels. The switching control device 240 comprises the rotating shaft 242 and multiple control cams arranged on the rotating shaft 242 in a non-rotatable way. The multiple control cams are arranged one-to-one corresponding to multiple pipe lines or pipe fittings; and the rotating shaft 242 rotates to change the rotation angles of the control cams to further switch on-off state of the pipe line or pipe fitting corresponding to each control cam. The pipe line can be, for example, a hose, and the fitting can be, for example, a switching valve with an operating element. By controlling the operating element, the control cam can switch on-off state of the corresponding switching valve.

Refer to the relevant description aforementioned for the detailed structure of the switching control device 240 according to the embodiments of the present disclosure.

When a bio-block comprises multiple shells, different shells are generally formed by different shell liquids, thus requiring multiple assembly procedures and cleaning during each assembly procedure. When assembling the shells around a microsphere, the related art mostly focuses on a single shell, such as a sodium alga acid shell, even though the related art can be used to assemble the shells with multiple shell liquids, there will be cross contamination and residual liquid in each shell, resulting in poor assembly effect, and even failure of the assembly.

The shell assembly device of the embodiment can assemble multiple shells formed by multiple shell liquids, and avoid decreased activity of the bio-block and loss of raw materials caused by frequent transfer and operation. Besides, cross contamination can be avoided, that is, one shell liquid can be assembled in a single shell or multiple shells, and multiple shell liquids can be assembled in multiple shells.

According to the above description, the shell assembly device of the embodiments of the present disclosure delivers at least one of the following technical effects:
1. The shell assembly device is suitable for the shell assembly formed by single shell liquid and multiple shell liquids. It can realize multi-shell assembly for multiple shell liquids, which solves the inadequate and failed shell assembly, and avoids the cross contamination with the previous procedures, thus delivering the products that better satisfy biomedical requirements.
2. The shell liquid and the core sphere are mixed evenly, ensuring that the particle size of the shell assembly is uniform.
3. During the assembly, the shell liquid is added to the assembling tube containing the microspheres or the compositional structures, thus reducing the amount of the shell liquid and avoiding waste.
4. Frequent man-made operation and transfer are not required, thus avoiding the decreased activity of the bio-block core sphere and the loss of the raw materials.
5. Automatic shell assembly is realized, which can speed up the production and enhance the efficiency, and is suitable for industrial application.

The bio-block preparation instrument of the embodiments of the present disclosure comprises the microsphere preparation device for preparing the bio-block core spheres and the shell assembly device for assembling the bio-block shells. The two devices are independent from each other. The bio-block preparation instrument can accelerate the production of the bio-blocks, and guarantees the cell viability in the bio-block to the maximum extent, so that the prepared bio-blocks meet the activity requirements for 3D bio-printing and industrialization needs, and are also suitable for clinical application.

According to the above description, the bio-block preparation instrument of the embodiment achieves at least one of the following technical effects:
1. A dedicated device for preparing the bio-block is provided to prepare qualified bio-blocks which apply to, for example, 3D bio-printing to build tissue construction which can be implanted in human body to play a specific role.
2. The bio-block can be prepared at high efficiency for industrialization.
3. High-viscosity cell materials can be used to prepare the high-activity bio-block. At the same time, a proliferation and differentiation environment is available for the cells in the bio-blocks.
4. When the bio-block has multiple shells with different compositions, the multi-shell liquid system has a better cross assembly effect, thus avoiding the cross contamination.
5. The prepared bio-blocks can be used for 3D bio-printing of tissue constructs which play a specific role in clinic application.
6. The automatic production is realized, thus reducing the decreased activity of the bio-block core sphere and the loss of raw materials caused by man-made operation.
7. The bio-block core spheres and shells are prepared in a uniform manner.

As shown in Figures 33 to 45, in a still further embodiment of the present disclosure, the bio-block preparation instrument comprises a microsphere preparation device and a shell assembly device. The microsphere preparation device and the shell assembly device are arranged integrally.

As shown in Figure 33, the bio-block preparation instrument of the embodiment further comprises a bio-block preparation rack and a control device. The bio-block preparation rack is configured to support structures of parts of the bio-block preparation instrument. The control device is configured to control the microsphere preparation process.

As shown in Figure 33, the microsphere preparation device comprises a microsphere preparation material pumping device 11, a microsphere preparation material storage device 12, a microfluidic chip 15, a curing device, a preparation case 7, a microsphere preparation material temperature control unit, a curing device temperature control unit, a chip fixture 14, a connecting tube 16, a connecting pipe line 17 and an output pipe line 18.

As shown in Figure 33, the shell assembly device essentially comprises a shell preparation material storage device and an assembling device. The assembling device essentially comprises a flush module 3 and a shell formation module 4.

As shown in Figures 33 to 35, in the microsphere preparation device, the microsphere preparation material storage device 12, the microfluidic chip 15 and the curing device 2 are arranged in the preparation case 7. The preparation case 7, the shell preparation material storage device and the assembling device are arranged on the bio-block preparation rack.

The microsphere preparation material storage device 12 comprises a shear liquid storage space and a microsphere stock solution storage space. The shear liquid storage space is configured to store a shear liquid, and the microsphere stock solution storage space is configured to store a microsphere stock solution for microsphere preparation.

The microfluidic chip 15 comprises a first chip inlet communicated with the microsphere stock solution storage space and a second chip inlet communicated with the shear liquid storage space. The microsphere stock solution is sheared into microdroplets by the shear liquid in the microfluidic chip 15, and then a microdroplet-shear liquid mixture is discharged from a chip outlet of the microfluidic chip 15.

As shown in Figure 33, the curing device 2 comprises a curing device inlet. The curing device inlet is connected with the chip outlet of the microfluidic chip 15 to receive the microdroplet-shear liquid mixture. The microdroplets are cured in the curing device 2 to form microspheres.

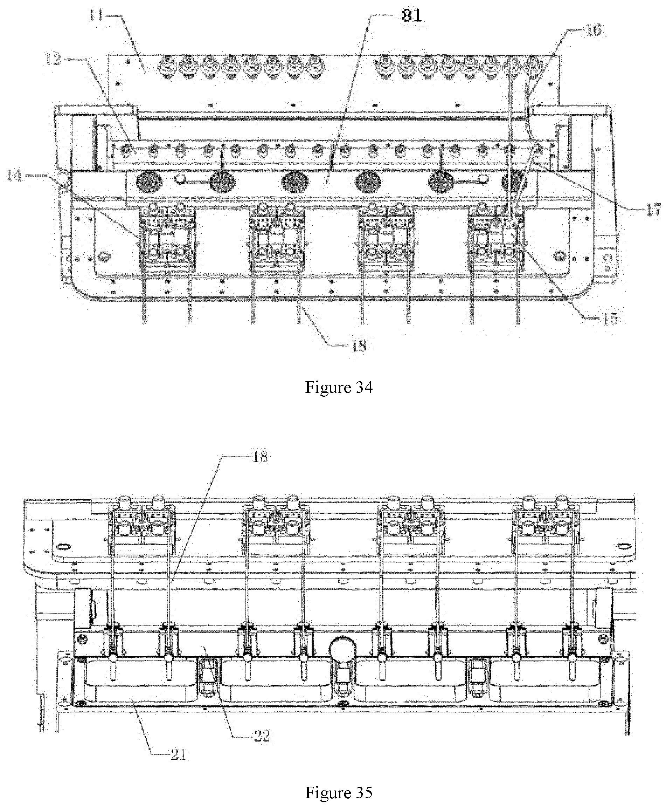

As shown in Figure 35, in the embodiment, the curing device 2 comprises a receiving container 21 and an isolation control unit.

The receiving container 21 is configured to accommodate a receiving liquid immiscible with the microdroplets, and allow the microdroplet-shear liquid mixture from the chip outlet to enter the receiving liquid, and the microdroplets are cured into the microspheres in the receiving liquid. The receiving liquid is preferably the same oil phase liquid as the shear liquid.

The isolation control unit is configured to isolate each microdroplet from the other microdroplets by the receiving liquid before forming the microspheres.

The bio-block preparation instrument and the microsphere preparation device thereof according to the embodiments of the present disclosure collect and cure the microdroplets with the receiving liquid, thus improving the efficiency of collection, subsequent operation and microsphere preparation. Each of prepared microspheres is close to spherical shape, so that the microspheres are more uniform in shape, thus improving the bio-printing quality. In addition, as for the microspheres with bioactive substances, the biological activity can be maintained.

Thanks to the viscosity, as long as there is a certain distance between the two new-made microdroplets in the receiving liquid, the microdroplets can be separated by the receiving liquid without physical separation. Free from the restriction of physical separation, the microdroplets can be collected at a faster speed. What's more, the microdroplets are cured in the receiving liquid to form the microspheres, so that they can be cleaned and assembled in a uniform manner, thus effectively improving the efficiency of the microdroplet collection and subsequent preparation and making the preparation of the microspheres more efficient.

The receiving container 21 of the microsphere preparation device integrates the collection and curing functions without physical separation, thus reducing the volume of the microsphere preparation device.

In the embodiments of the present disclosure, the microdroplets are cured in the receiving liquid, the microdroplets are on a liquid-liquid interface, and the receiving liquid forms a system of maintaining the shape of the microdroplets, thereby forming a desirable spherical shape.

In addition, during the curing process, since the microdroplets are completely immersed in the receiving liquid, the microdroplet share the same temperature of the surrounding receiving liquid and the same coefficient of heat conductivity, the surfaces of microdroplets can be evenly heated in the receiving liquid at the same curing rate as a whole. In this way, the biological activity can be ensured for the microspheres with bioactive substances.

As shown in Figures 33, 39, and 40, the preparation case 7 comprises a case body 71 and a case cover 72 matched with the case body 71. The preparation case 7 has a first preparation chamber 78 and a second preparation chamber 79 which are separately arranged in the preparation case 7. The microfluidic chip 15 is arranged in the first preparation chamber 78 to generate microdroplets in the first preparation chamber 78. The curing device 2 is arranged in the second preparation chamber 79 to maintain the spherical shape of the new-made microdroplets in the second preparation chamber 79, and finally cure the microdroplets to form microspheres.

The microfluidic chip 15 is configured to prepare the microdroplet. In the embodiment, the microfluidic chip 15 uses a continuous phase fluid (shear liquid) to shear the dispersed phase fluid (microsphere stock solution), thus separating the dispersed phase fluid into the microdroplets in corresponding sizes.

The microfluidic chip 15 comprises a first chip inlet, a second chip inlet, a chip outlet for discharging a microdroplet-shear liquid mixture, and a first chip channel and a second chip channel respectively in communication with the first chip inlet, the second chip inlet and the chip outlet. The continuous phase fluid shears the dispersed phase fluid into microdroplets at an intersection of the first chip channel and the second chip channel.

The dispersed phase fluid is a microsphere stock solution which forms the microdroplet, and the microsphere stock solution constitutes a component of the microdroplet. The continuous phase fluid is a shear liquid for preparing the microdroplets, and does not constitute a component of the microdroplet. In the embodiment, the microsphere stock solution is a collagen solution with bioactive substance (e.g. cells). The shear liquid is an oil phase liquid immiscible with the collagen solution.

The microsphere preparation material storage device 12 is configured to store the liquid for preparing the microdroplet. In the embodiment, the microsphere preparation material storage device 12 comprises a microsphere stock solution storage space for storing the microsphere stock solution and a shear liquid storage space for storing the shear liquid. An outlet of the microsphere stock solution storage space is communicated with the first chip inlet of the microfluidic chip 15 via the connecting pipe line 17. An outlet of the shear liquid storage space is in communication with the second chip inlet of the microfluidic chip 15 via another connecting pipe line 17.

As shown in Figure 33, in the embodiment, the microsphere preparation material storage device 12 has 16 storage containers arranged in parallel, each of which forms a storage space therein. Among them, 8 storage spaces serve as the microsphere stock solution storage space; and the remaining 8 serve as the shear liquid storage space. The 8 microsphere stock solution storage spaces and 8 shear liquid storage spaces are arranged alternately.

In the embodiment, the microsphere preparation material pumping device 11 controls pressure variation of each storage space to deliver the liquid in the storage space to the microfluidic chip 15.

The microsphere preparation material pumping device 11 comprises several pressure control portions, each of which is connected with the corresponding storage space of the microsphere preparation material storage device 12 via the connecting tube 16.

In the embodiment, the microsphere preparation material pumping device 11 comprises 16 pressure control portions, each of which is connected with a storage space via the connecting tube 16, provides flow dynamic for the microsphere stock solution and the shear liquid by adjusting the gas pressure in the storage space, and controls the flow rate of the microsphere stock solution and the shear liquid.

The pressure control portions are independent of each other, and can be selected as required, or operating parameters of each pressure control portion can be controlled independently, thereby independently controlling the pressure of the connected storage space and the flow rate of materials stored in the corresponding storage space. Parameters, such as the microdroplet size and the generation rate, can be controlled by adjusting the flow rate of the microsphere stock solution and the shear liquid.

The microsphere preparation material pumping device 11 can be, for example, a pressure pump. A gas pumped by the pressure pump applies certain pressure to the microsphere stock solution or the shear liquid, enabling the microsphere stock solution or the shear liquid to be pumped to the corresponding chip inlet of the microfluidic chip 15. After entering the corresponding chip channels of the microfluidic chip 15, the microsphere stock solution and the shear liquid form the microdroplet in the chip channel.

In addition, the key to prepare uniform-sized microspheres lies in stably driving the fluid. The microsphere preparation material pumping device 11 uses the pressure control portions to drive the microsphere preparation materials, which delivers high stability and instantaneous response velocity, thus accurately controlling the flow and flow rate of the microsphere stock solution and the shear liquid and quickly switching between different reaction materials. Once the flow rate parameters of the system are set, the production of the microdroplets is only slightly changed. The gas used by the microsphere preparation material pumping device 11 to change the pressure of the storage space can be, for example, nitrogen, compressed air and other inert gases.

In the embodiment, the microsphere preparation material pumping device 11 and the microfluidic chip 15 are used to accurately disperse the liquids, so that the liquid amount can be predicted based on the predetermined parameters of the microsphere and the liquid can be precisely added in a quantitative manner. In this way, the particle size of the prepared microspheres can be more accurate.

In the embodiment, the output pipe line 18 is an output capillary pipe. The chip output of the microfluidic chip 15 is connected with the output pipe line 18 through which the prepared microdroplets are delivered to the second preparation chamber 79. An opening for the output pipe line 18 to pass through is provided on a partition plate in the case body 71 for separating the first preparation chamber 78 from the second preparation chamber 79.

To better output high-viscosity materials, preferably, an inner surface of an outlet of the output pipe line 18 is a super-hydrophobic surface. The super-hydrophobic surface can smoothly output the microdroplets, prevent the microdroplet materials from remaining at the outlet of the output pipe line 18, and generate the microdroplets closer to spherical shape.

The microsphere preparation device further comprises a microsphere preparation material temperature control unit for controlling the microsphere preparation material storage device 11 and/or the microfluidic chip 15 at a preset temperature or within a preset temperature range. The microsphere preparation material temperature control unit controls the temperature of the first preparation chamber 78 at the preset temperature or within the preset temperature range to achieve the control purpose. In the embodiment, the microsphere preparation material temperature control unit is located in the first preparation chamber 78. The microsphere preparation material temperature control unit can independently control the ambient temperature of the first preparation chamber 78, so that the temperatures of the microsphere preparation material storage device 11 and the microfluidic chip 15 can be controlled in a uniform manner.

In the embodiment, the bio-block prepared by the bio-block preparation instrument is used to prepare a bio-ink. The core sphere of the bio-block is the microsphere prepared by the microsphere preparation device. As a raw material of the microsphere composition, the microsphere stock solution is a collagen solution containing bioactive substances. Collagen is thermosensitive, and can be cured or liquefied at a certain temperature. Collagen shows good fluidity below 4°C, and tends to be cured when the temperature is above 4°C. When the temperature is higher than 4°C, the fluidity of the collagen solution may be affected, thus adversely affecting the generation of the microdroplets. For this reason, preferably, the temperatures of the microsphere preparation material storage device 12 and/or the microfluidic chip 15 are controlled.

In the embodiment, by controlling the temperature of the first preparation chamber 78, the temperatures of the microsphere stock solution and the shear liquid are preferably controlled at around 4°C. If only the temperature of the microsphere stock solution is controlled and the temperature of the shear liquid is too high, the temperature of the microsphere stock solution will be affected by the shear liquid in the chip channel of the microfluidic chip 15, resulting in solidification and blocking at the shear port, thus preventing the microdroplet from being generated smoothly.

Besides, to prevent the chip channel of the microfluidic chip 15 from being blocked, the temperature of the microfluidic chip 15 needs to be controlled.

In the embodiment, the microsphere preparation material storage device 12 and the microfluidic chip 15 are located in the first preparation chamber 78, of which the temperature is controlled by the microsphere preparation material temperature control unit, so that the temperatures of the microsphere preparation material storage device 12 and the microfluidic chip 15 are also under control. For this reason, the temperature at which the microdroplet is generated varies slightly, better maintaining the biological activity.

The microsphere preparation material temperature control unit preferably comprises a cooling device arranged in the first preparation chamber 78 or for cooling a wall of the first preparation chamber 78, such as a thermoelectric cooler.

In some embodiments, the microsphere preparation material temperature control unit further comprises at least one fan which facilitates internal air circulation in the first preparation chamber 78, so that temperature distribution in the preparation chamber 78 can be more uniform.

In the embodiment, the microsphere preparation material temperature control unit comprises a cooling device for cooling a bottom wall of the first preparation chamber 78, and further comprises a fan group 81 arranged in the first preparation chamber 78. The fan group 81 comprises multiple fans which can circulate the low temperature air in the first preparation chamber 78, thus minimizing the temperature variation therein. In this way, the temperatures of the connecting tube 16 between the pressure control portion and the microsphere preparation material storage device 12, the connecting pipe line 17 between the microsphere preparation material storage device 12 and the microfluidic chip 15, and the output pipe line 18 connected with the outlet of the microfluidic chip 15 are better controlled.

The cooling device can further comprise a thermoelectric cooler directly acting on the microfluidic chip 15 and a chip fixure 14 made of a metallic material, the thermoelectric cooler can be arranged on the chip fixture 14 which can control the temperature of the microfluidic chip 15 by rapid heat transfer.

In the embodiment, microdroplets are generated in the first preparation chamber 78, throughout generation of microdroplets the temperatures of the microsphere stock solution, the shear liquid and relevant parts are under control, thus ensuring the fluidity of the microsphere stock solution to generate the microdroplets in a stable way.

The chip fixture 14 is configured to fix the microfluidic chip 15. The microfluidic chip 15 is detachably arranged on the chip fixture 14, which can better configure the microfluidic chip 15 with different functions and sizes to meet the preparation requirements of different microdroplets. At the same time, such arrangement facilitates the rapid disassembly and assembly of the microfluidic chip 15.

In the embodiment, the microfluidic chip 15 is mounted on the corresponding mounting base via the chip fixture 14. The chip fixture 14 is also configured to limit the position of the connecting pipe line 17 and the output pipe line 18, and to prevent the connecting pipe line 17 and the output pipe line 18 from being disconnected from the microfluidic chip 15, thus avoiding the leakage of the dispersed phase fluid, the continuous phase fluid or the microdroplet. Furthermore, sealing connection can be realized between the microfluidic chip 15 and the connecting pipe line 17 or the output pipe line 18 via the chip fixture 14.

As shown in Figures 33 to 38, in the embodiment, each chip fixture 14 has two chip fixing positions and can fix two microfluidic chips 15 at the same time.

The chip fixture 14 comprises a base plate 14D and a clamp plate 14E. Two clamp plates 14E are correspondingly arranged for each chip fixing position. Each clamp plate 14E is mounted on the base plate 14D via a fixture screw 14A. The microfluidic chip 15 is disposed between the clamp plate 14E and the base plate 14D, and the fixture screw 14A is tightened, so that the microfluidic chip 15 can be fixed on the base plate 14D.

The clamp plate 14E comprises a connecting port 14B for communicating with the chip inlet or outlet of the microfluidic chip 15. Two clamp plates 14E for fixing the same microfluidic chip 15 and the connecting ports 14B thereon are symmetrically arranged. The base plate 14D is fixedly connected with the corresponding mounting base, so that the microfluidic chip 15 can be detachably mounted on the mounting base.

The clamp plate 14E further comprises a connecting port 14B for communicating with the chip inlet or chip outlet of the microfluidic chip 15, and a seal ring 14C arranged corresponding to the connecting port 14B. The seal ring 14C comprises a tubular segment and a circular segment connected in sequence. The tubular segment is arranged inside the corresponding connecting port 14B, while the circular segment is arranged facing the base plate 14D at the connecting port 14B. When fixing the microfluidic chip 15, the microfluidic chip 15 is placed between the base plate 14D and the clamp plate 14E, while the connecting pipe line 17 and the output pipe line 18 are respectively inserted into the corresponding connecting port 14B and the tubular segment of the corresponding seal ring 14C, and the fixture screw 14A is tightened, thereby fixing the microfluidic chip 15 between the clamp plate 14E and the base plate 14D, and hermetically connecting the connecting pipe line 17 and the output pipe line 18.

In the embodiment, the control device is coupled with the microsphere preparation material pumping device 11 and the microsphere preparation material temperature control unit to control the formation process of the microdroplets.

In the embodiment, the control device is coupled with the pressure control portions to control the flow, flow rate and other parameters of the microsphere stock solution and the shear liquid, thereby controlling the parameters such as the size and generation rate of the microdroplet. In addition, the control device is also coupled with the microsphere preparation material temperature control unit through a temperature controller to control the ambient temperature of the first preparation chamber 78.

For example, the control device can control the gas pressure that drives the flow of the shear liquid based on the viscosity and other properties of the microsphere stock solution, thereby controlling the flow rate of the shear liquid and the shear rate of the microsphere stock solution by the shear liquid. In this way, microdroplets with identical shape and size can be generated, even if the properties of the microsphere change within a certain range. The property parameters of the microsphere stock solution can be input to the control device through external means, or related information can be collected during the preparation of the microdroplet. For example, a sensor can be arranged in the microsphere preparation material storage device 12 to collect viscosity, temperature or other data.

The curing device 2 is configured to cure the microdroplet generated by the microfluidic chip 15. The curing device 2 comprises the receiving container 21, the isolation control unit and an outlet position adjustment unit 22.

The receiving container 21 is used to contain the receiving liquid immiscible with the microdroplet. The receiving container 21 allows the microdroplets from the chip outlet to enter the receiving liquid and cured into the microspheres in the receiving liquid. The receiving container 21 collects generated microdroplets and maintains the shape of the microdroplets before curing thereof.

In the embodiment, the new-made microdroplet is still flowing fluid. The new-made microdroplet directly placed in a general container cannot maintain a spherical shape. Since the microdroplets are immiscible with the receiving liquid in the receiving container 21, receiving liquid can maintain the spherical shape throughout the curing until the microdroplets are completely cured to form the microspheres.

Since the microsphere stock solution for forming the microdroplet in the embodiment is a collagen solution which is an aqueous phase liquid with bioactive substances, the receiving liquid can be an oil phase liquid, preferably the same oil phase liquid as the shear liquid.

In some embodiments, the isolation control unit is configured to control at least one of the receiving container 21, receiving liquid, chip outlet or the outlet 18A of the output pipe line 18 connected with the chip outlet to separate the microdroplets from each other in the receiving liquid before forming the microspheres.

In the embodiment, the isolation control unit comprises a container shifting device, which is in driving connection with the receiving container 21 to drive the receiving container 21 to perform displacement when the receiving liquid receives the microdroplets.

In the embodiment, the container shifting device comprises a motion platform 23. The receiving container 21 is detachably connected onto the motion platform 23. The receiving container 21 can move with the motion platform 23, so as to move relative to the outlet 18A of the output pipe line 18. By this way, the microdroplets from the output pipe line 18 are sequentially distributed inside the receiving liquid in sequence and do not contact with each other before curing, thus avoiding agglomeration.

The container shifting device allows the relative movement between the chip outlet of the microfluidic chip 15 or the outlet 18A of the output pipe line 18 and the receiving container 21 of the curing device 2, thus ensuring that all the new-made microdroplets output into the receiving liquid in the receiving container 21 do not contact with the existing microdroplets therein, which can prevent two uncured microdroplets from contacting each other to agglomerate into one microdroplet.

The motion platform 23 of the embodiment comprises a motion mechanism which enables the receiving container 21 to move in a horizontal x direction and a y direction perpendicular to the x direction. The motion mechanism is driven by motors.

The container shifting device may also be in other forms, for example, include a driving device that makes the receiving container 21 rotate.

In some embodiments not illustrated, the isolation control unit may comprise a fluid circulation driving device which is configured to make the receiving liquid flow when the receiving liquid receives the microdroplets. With the fluid circulation driving device, the microdroplets will not contact each other before curing due to the flowing receiving liquid, even if the chip outlet and the receiving container 23 are relatively fixed.

For example, the receiving container 21 may comprise a circulation inlet, a circulation outlet and a hose connecting both the circulation inlet and the circulation outlet. The fluid circulation driving device comprises, for example, a peristaltic pump in driving connection with the connecting hose, through which the receiving liquid in the receiving container 21 keeps flowing, thereby preventing the microdroplet in the receiving liquid from contacting each other and avoiding agglomeration.

In some embodiments not illustrated, the isolation control unit may comprise a bubbling device which is configured to generate bubbles in the receiving liquid when the receiving liquid receives the microdroplets. The generated bubbles drive the receiving liquid to flow within a small range, thus preventing the microdroplets successively received by the receiving liquid from entering a same region thereof, and avoiding agglomeration.

In some other embodiments not illustrated, the isolation control unit can comprise a stirring device which is configured to stir the receiving liquid when the receiving liquid receives the microdroplets. The stirring device can also relocate the receiving liquid in the receiving container 21, so that the microdroplets successively received by the receiving liquid will not enter the same region thereof, thus avoiding agglomeration.

In addition, in some other embodiments not illustrated, the isolation control unit may comprise an outlet position driving device for driving the chip outlet to change the position thereof, so as to change the position of the chip outlet relative to the receiving container 21, thus separating the microdroplets before the microdroplets form the microspheres.

When separating the microdroplets from each other before the microspheres are formed by controlling the relative movement between the chip outlet and the receiving container 23, the direction of the relative movement can be horizontal, vertical, inclined relative to the horizontal or vertical direction or a combination thereof.

The receiving container 23 can be shallow but long or wide, so that the new-made microdroplets are arranged at intervals in the horizontal direction; The receiving container 23 can have a relatively great depth, so that the new-made microdroplets are arranged at intervals in the vertical direction.

In the embodiment, the microsphere stock solution that forms the microdroplet is a collagen solution. During the curing process, relatively high temperature can speed up the curing of the microdroplets. For example, when the curing temperature is controlled at 37C, the curing duration may last 15 to 30 minutes. Of course, the temperature can be controlled within a preset range, such as 35 to 37°C.

The temperature of the receiving liquid can be controlled by adjusting temperature of the space in which the curing device 2 is located. In the embodiment, the curing device 2 is arranged in the second preparation chamber 79. The microsphere preparation device comprises a curing device temperature control unit for controlling the second preparation chamber 79 at a preset temperature or within a preset temperature range. The curing device temperature control unit controls the temperature of the curing device 2 and the receiving liquid therein by controlling the temperature of the second preparation chamber 79.

In some embodiments not illustrated, the curing device temperature control unit is configured to control the temperature of the receiving liquid at a preset temperature or within a preset temperature range. The curing device temperature control unit can be a temperature control device directly acting on the receiving container 21, for example, a heating device arranged at the bottom of the receiving container 21.

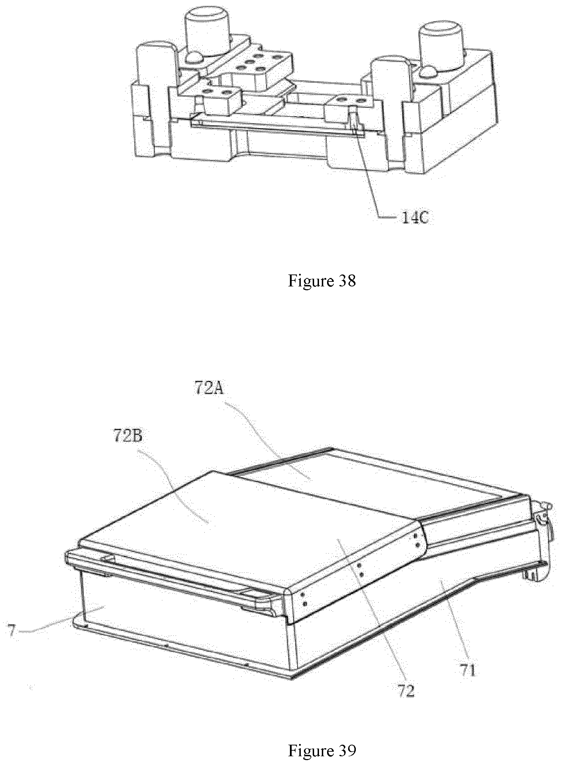

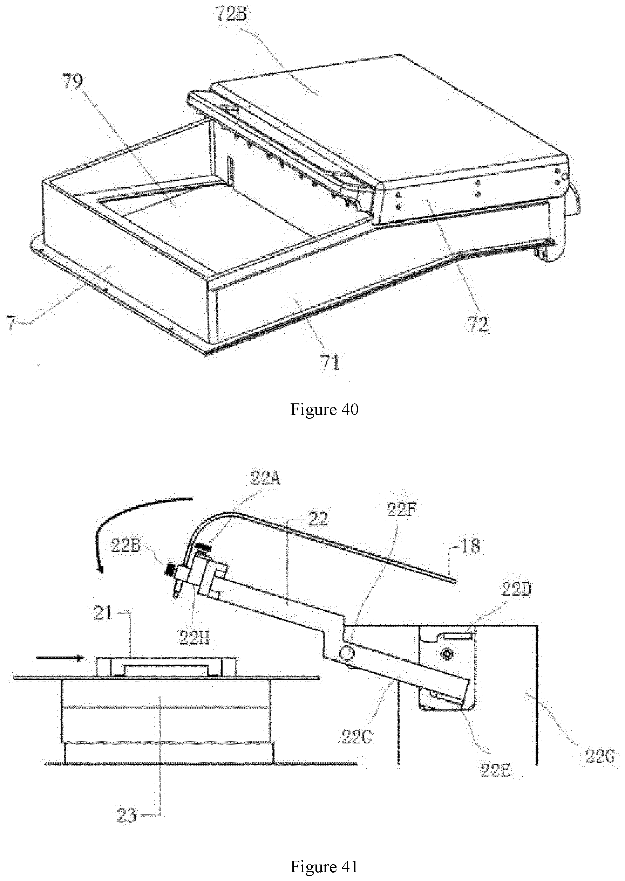

As shown in Figures 39 and 40, in the embodiment, the case cover comprises a first cover body 72A for covering the first preparation chamber 78 and a second cover body 72B for covering the second preparation chamber 79, wherein the second cover body 72B can be opened independently, and the first cover body 72A can be opened only when the second cover 72B is opened.

Since the temperature of the second preparation chamber 79 is close to room temperature, and the microspheres need to be taken out therefrom, which results in relatively frequent operation, the second cover body 72B is designed to be opened independently to better suit the operation requirements. Instead, the first preparation chamber 78 has a lower temperature and requires to be opened less frequently. Therefore, the first cover body 72A is designed to be opened only when the second cover body 72B is opened, thus reducing the opening frequency of the first cover body 72A, better maintaining the temperature of the first preparation chamber 78 and saving energy.

Preferably, a sealing structure is provided preferably between the first cover body 72A and the case body 71 to improve thermal insulation effect of the first preparation chamber 78.

In the embodiment, preferably, the outlet 18A of the output pipe line 18 is located below the level of the receiving liquid when the receiving liquid receives the microdroplets. Such arrangement enables the microdroplets to directly enter the interior of the receiving liquid to avoid deformation of microdroplets when dropping in the receiving liquid through the air, so that the microdroplets and the generated microspheres can better maintain the spherical shape.

In addition, preferably, the curing device 2 comprises an outlet position adjustment unit 22 for adjusting the position of the outlet 18A of the output pipe line 18.

In the embodiment, the outlet position adjustment unit 22 comprises a clamping device 22H for clamping a tail end of the output pipe line 18, a first adjustment device, and a second adjustment device. The first adjustment device is configured to control switching of the clamping device 22H between a first position and a second position above the first position. The second adjustment device is configured to control the height of the tail end of the output pipe line 18 relative to the clamping device 22H and thus controlling the height of the chip outlet relative to the receiving container 21.

As shown in Figures 41 to 43, in the embodiment, the first adjustment device comprises a rotating arm 22C, a first magnet 22D, a second magnet 22E, a rotating shaft 22F, and a support frame 22G, which are arranged in the second preparation chamber 79. The support frame 22G is fixedly arranged in the second preparation chamber 79. The first magnet 22D and the second magnet 22E are arranged up and down at intervals on the support frame 22G. The rotating shaft 22F is connected onto the support frame 22G. The rotating arm 22C is rotatably connected onto the rotating shaft 22F relative to the support frame 22G. A first end of the rotating arm 22C is connected with the clamping device 22H disposed above the receiving container 21. A second end of the rotating arm 22C is located between the first magnet 22D and the second magnet 22E. The tail end of the output pipe line 18 is clamped on the clamping device 22H, so that the outlet 18A is located above the receiving container 23.

When a portion of the rotating arm 22C next to the first end is lifted up or a portion of the rotating arm 22C next to the second end is pressed down, the rotating arm 22C is relieved of the attraction by the first magnet 22D and attracted by the second magnet 22E, thus making the clamping device 22H in the second position. By this way, the outlet 18A of the output pipe line 18 is in a standby position due to being at a relatively high position. Instead, when the portion of the rotating arm 22C next to the first end is pressed down or the portion of the rotating arm 22C next to the second end is lifted up, the rotating arm 22C is relieved of the attraction by the second magnet 22E and attracted by the first magnet 22D, thus making the clamping device 22H located in the first position. By this way, the outlet 18A of the output pipe line 18 is at a position suitable for releasing the microdroplet into the receiving liquid due to being at a relatively low position.

As shown in Figures 41 to 43, in the embodiment, the second adjustment device comprises a position adjustment knob 22A arranged on the clamping device 22H. Tightening or loosening the position adjustment knob can adjust the height of the tail end of the output pipe line 18, thus controlling the height of the outlet 18A of the output pipe line 18.

In addition, as shown in Figures 41 to 43, the present embodiment further comprises an opening adjustment knob 22B. Loosening the opening adjustment knob 22B can open the outlet 18A of the output pipe line 18, while tightening the knob 22B can close the outlet 18A of the output pipe line 18.

In the embodiment, the position of the outlet 18A of the output pipe line 18 is adjusted by the first adjustment device and the second adjustment device, thus controlling the output pipe line 18 to directly output the microdroplets below the liquid level of the receiving liquid in the receiving container 21.

The control device is also coupled with the motion platform 23 of the curing device 2 and the curing device temperature control unit to control the motion of the motion platform 23 and the temperature of the second preparation chamber 79, thereby controlling the curing process of the microdroplets.

The control device controls the motor of the motion platform 23 through a driver, thus controlling the motion of the motion mechanism on the motion platform 23. In addition, the control device can control an output speed of the motor to match the different generation rates of the microdroplets.

The control device also controls the curing device temperature control unit through a temperature controller, thereby controlling the temperature of the second preparation chamber 79.

The shell assembly device of the bio-block preparation instrument according to the embodiments of the present disclosure is configured to coat the microspheres or the compositional structures composed of a microsphere and at least one shell. The shell assembly device comprises a shell preparation material storage device and a assembling device.

The shell preparation material storage device comprises multiple storage spaces which comprise a cleaning liquid storage space for storing a cleaning liquid and a shell liquid storage space for storing a shell liquid.

A cleaning liquid cleans the microspheres or the compositional structures in the assembling device, and the cleaned microspheres or the compositional structures are coated with the shell liquid to form a shell around each of the microspheres or compositional structures.

The assembling device essentially comprises a flush module 3 and a shell formation module 4. As shown in Figure 33, the flush module 3 and the shell formation module 4 are arranged on the bio-block preparation rack.

The flush module 3 is designed for cleaning the residual shear liquid, receiving liquid or shell liquid on the microspheres or the compositional structures to facilitate the next operation.

In the embodiment, the shell preparation material storage device comprises a cleaning liquid container 91 and a shell liquid container 92. The cleaning liquid container 91 comprises a cleaning liquid storage space for storing the cleaning liquid. The shell liquid container 92 comprises a shell liquid storage space for storing shell liquid. The present embodiment comprises two shell liquid containers 92, thereby comprising two shell liquid storage spaces.

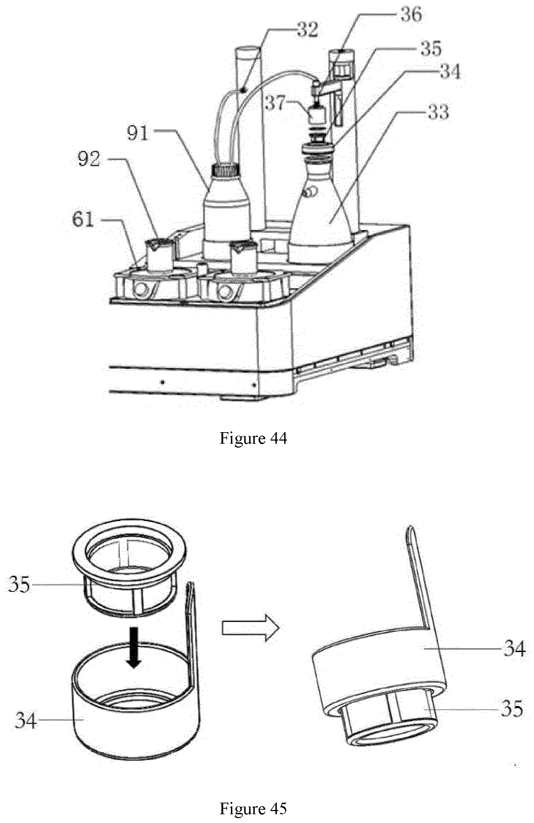

As shown in Figures 44 and 45, the flush module 3 comprises a sprayer 36, a first accommodating screen jacket 34, a first accommodating screen 35, a pneumatic valve 32, a filtrate container 33, a seal cover 37, a suction device, and a gas connecting tube and a cleaning liquid connecting tube.

The cleaning liquid container 91 is connected with one end of the gas connecting tube and one end of the cleaning liquid connecting tube. The gas connecting tube is connected with the pneumatic valve 32 at the other end thereof. The cleaning liquid connecting tube is connected with the sprayer 36 at the other end thereof, therefore, by controlling the gas pressure in the cleaning liquid container 91, the pneumatic valve 32 controls whether the cleaning liquid is sprayed from the sprayer 36 as well as the spraying rate of the sprayer 36. The cleaning liquid sprayed from the sprayer 36 is applied for rinsing the microspheres formed in the curing device 2.

In the embodiment, the microspheres or the compositional structures are placed in the first accommodating screen 35 before rinse. In the embodiment, the assembling device comprises a partitioned assembling space. The assembling space comprises the accommodating space of the first accommodating screen 35. The first accommodating screen jacket 34 is arranged at an opening of the filtrate container 33, while the first accommodating screen 35 is arranged on the first accommodating screen jacket 34. The sprayer 36 faces an upper screen opening of the first accommodating screen 35 to rinse the microspheres or the compositional structures in the accommodating space of the first accommodating screen 35. The filtrate container 33 is configured to receive the cleaning liquid discharged by the accommodating screen 35.

The seal cover 37 is arranged between the sprayer 36 and the screen opening of the first accommodating screen 35, and arranged around the sprayer 36 and screen opening of the first accommodating screen 35. The seal cover 37 is configured to form a partial seal between the sprayer 36 and the first accommodating screen 35, so that the cleaning liquid can be easily drawn into the filtrate container 33. The suction device is communicated with the filtrate container 33 to suck the fluid in the filtrate container 33, so that the cleaning liquid in the first accommodating screen 35 is sucked via pressure difference. The suction device can accelerate the separation of the microspheres and the cleaning liquid and enhance the separation effect.

In the embodiment, the control device is further coupled with the pneumatic valve 32 to control whether air is drawn into the cleaning liquid container 91 and the amount of air intake, thus controlling whether the cleaning liquid is sprayed and the flow and flow rate of the cleaning liquid.

In some embodiments not illustrated, the shell assembly device can further comprise a separation device for separating the microspheres and the receiving liquid. The separation device may comprise, for example, a filter element or a centrifugal device.

The shell formation module 4 is configured to coat the microspheres or the compositional structures composed of a microsphere and at least one shell to form at least one shell around each of the microspheres or the compositional structures. The material forming the shell is the shell liquid.

The shell formation module 4 comprises a second accommodating screen, a second accommodating screen jacket, and a oscillation device of the control device.

The shell liquid container 92 is configured to accommodate the shell liquid. When forming the shells, the second accommodating screen jacket 34 is arranged on the shell liquid container 92, and the second accommodating screen 35 is arranged on the second accommodating screen jacket 34, so that the second accommodating screen 35 is arranged on the shell liquid container 92. In the embodiment, the assembling space of the assembling device comprises an accommodating space of the second accommodating screen 35. The accommodating space of the second accommodating screen 35 is configured to place the microspheres or the compositional structures. When the shells are formed, the microspheres or the compositional structures in the second accommodating screen 35 are located below the level of the shell liquid to peripherally coat the microspheres or the compositional structures with the shell liquid.

The oscillation device of the control device is configured to shake the shell liquid in the shell liquid container 92 relative to microspheres or the compositional structures during the formation of the shells, so that the microspheres or the compositional structures and the shell liquid can be fully mixed to form the shells that are uniform in thickness.

In the embodiment, the oscillation device of the control device comprises a magnetic vibration generator 61. The magnetic vibration generator 61 having an adjustable and digitally displayed rotating speed from 0 to 2500rpm can be selected to adjust shaking degree of the shell liquid container 92 as required.

The second accommodating screen jacket allows the second accommodating screen to be suspended movably in the vertical direction in the shell liquid container 92.

It should be noted that when the same accommodating screen jacket 34 can be mounted on the filtrate container 33 and the shell liquid container 92, the flush module 3 and the shell formation module 4 can share one set of the accommodating screen and the accommodating screen jacket. After the rinse is completed, the first accommodating screen jacket 34 on the filtrate container, the first accommodating screen 35 thereon and the microspheres or the compositional structures in the accommodating screen 35 can be removed and directly placed on the shell liquid container 92 to serve as the second accommodating screen and the second accommodating screen jacket of the shell formation module 4.

Of course, the flush module 3 and the shell formation module 4 can share the same accommodating screen jacket but use different accommodating screens.

The control device is further coupled with the magnetic vibration generator 61 to control various parameters thereof.

The embodiments of the present disclosure also provide a method for preparing bio-blocks. The method for preparing bio-blocks according to the embodiment comprises preparing the bio-blocks with bioactive substances by the bio-block preparation instrument according to the described embodiments. The bio-block has a core sphere and a shell coating the core sphere. The core sphere has bioactive substances. The shell is configured to protect the core sphere.

The method for preparing bio-blocks according to the embodiment comprises a microdroplet formation step, a microsphere formation step, a microsphere cleaning step and a shell formation step.

In the microdroplet formation step, preferably, the microdroplets and/or microdroplet raw materials are controlled at a preset temperature or within a preset temperature range. More preferably, the temperatures of the microdroplets and/or the microdroplet raw materials are controlled at 49°C.

In the microsphere formation step, the receiving liquid immiscible with the microdroplet is used to receive the microdroplets, and the microdroplets can be cured in the receiving liquid to form the microspheres. Preferably, the microdroplets are separated from each other by the receiving liquid before forming the microspheres. Preferably, the microdroplets are separated from each other by the receiving liquid before forming the microspheres by controlling at least one of the receiving container 21, the receiving liquid, the chip outlet or the outlet 18A of the output pipe line 18 connected with the chip outlet.

Separating microdroplets from each other by the receiving liquid before forming the microspheres comprises relocating the receiving container 21 containing the receiving liquid when the receiving liquid receives the microdroplets; alternatively, the receiving liquid is driven to flow when the receiving liquid receives the microdroplets; alternatively, bubbles are generated inside the receiving liquid when the receiving liquid receives the microdroplets; alternatively, the receiving liquid is stirred when the receiving liquid receives the microdroplets.

Preferably, in the microsphere formation step, the temperature of the receiving liquid is controlled at a preset temperature or within a preset temperature range. More preferably, the temperature of the receiving liquid is controlled within 35°C to 37°C.

Preferably, in the microsphere formation step, when the receiving liquid receives the microdroplets, the microdroplets are directly introduced into the receiving liquid. Preferably, before the receiving liquid receives the microdroplets, the step comprises adjusting the height of the chip outlet for outputting the microdroplets to the receiving liquid to make the chip outlet located below the receiving liquid level.

The microsphere cleaning step comprises cleaning the microspheres after the microsphere formation step. Preferably, the microsphere cleaning step comprises a rinsing step for rinsing the microspheres. The rinsing step preferably comprises placing the first accommodating screen 35 which contains the microspheres on the filtrate container 33; forming a seal between the sprayer 36 and the screen opening of the first accommodating screen 35, and rinsing the microspheres with the cleaning liquid sprayed from the sprayer 36; and sucking the fluid in the filtrate container 33.

In addition, the microsphere cleaning step may further comprise a separation step in which the microspheres are separated from the receiving liquid.

The shell formation step comprises placing the microspheres or the compositional structures composed of a microsphere and at least one shell in the shell liquid to peripherally form a shell of each of the microspheres or the compositional structures. Preferably, the shell formation step comprises placing the microspheres or the compositional structures in the second accommodating screen in the shell liquid. Still preferably, the shell formation step preferably comprises shaking the shell liquid containing the microspheres or the compositional structures.

A specific method for preparing the bio-block by the bio-block preparation instrument of the embodiment is detailed as follows.

The bio-block preparation instrument is powered on, and the preparation of the bio-block is started when the temperatures of the first preparation chamber 78 and the second preparation chamber 79 are respectively constant at 4°C and 37°C;
The microsphere stock solution and the shear liquid are loaded. The microsphere stock solution is a collagen solution with cells, and the shear liquid is an oil phase liquid. The first cover body 72A and the second cover body 72B are opened to take out the microsphere preparation material storage device 12, and the materials are added from the outside of the bio-block preparation instrument. After the addition, the microsphere preparation material storage device 12 is mounted on a fixed position in the first preparation chamber 78. The microsphere preparation material storage device 12 is connected with the connecting tube 16 and the connecting pipe line 17 respectively. The microfluidic chip 15 is placed in the chip fixture 14. The connecting pipe line 17 and the output pipe line 18 are inserted into the connecting port 14B of the chip fixture 14 and the seal ring 14C corresponding to the connecting port 14B, and the fixture screw 14A is tightened. After the microfluidic chip 15 is mounted, the first cover body 72A is closed to isolate the first preparation chamber 78 from the outside.

The receiving container 21 is filled with the receiving liquid which is an oil phase liquid. The tail end of the output pipe line 18 is mounted on the clamping device 22H. The position of the outlet 18A of the output pipe line 18 is adjusted via the first adjustment device and the second adjustment device, so that the outlet 18A is located below the receiving liquid level. The second cover body 72B is closed to isolate the second preparation chamber 79 from the outside.

The cleaning liquid container 91 is placed at a preset position. The shell liquid container 92 containing the shell liquid is placed at a preset position.

The flush module 3 is mounted. The cleaning liquid container 91 is connected with the pneumatic valve 32 and the sprayer 36. The periphery of the sprayer 36 is covered with the seal cover 37. The sterile filtrate container 33 is placed at a preset position. The first accommodating screen 35 and the first accommodating screen jacket 34 are mounted at the preset positions. The suction device is connected with the filtrate container 33.

The shell formation module 4 is mounted. The second accommodating screen and the second accommodating screen jacket are mounted on the shell liquid container 92.

The microsphere preparation material pumping device 11 and the isolation control unit are started up. The control device controls the pressure control portions based on the specified gas pressure to drive the microsphere stock solution and the shear liquid to flow. The shear liquid shears the microsphere stock solution in the microfluidic chip 15 to obtain the microdroplets. The prepared microdroplets enter the receiving liquid of the receiving container 21 through the output pipe line 18 to be collected and cured.

The microdroplets are cured at a constant temperature in the receiving liquid for 15 minutes to form the microspheres. The microspheres are taken out and transferred to the first accommodating screen 35 of the flush module 3 for rinse by the sprayer 36. The suction device is turned on to suck the fluid in the filtrate container 33 to help separate the microspheres from the cleaning liquids.

Optionally, the microspheres and the receiving liquid can be initially separated through filtration, centrifugation or other means before rinse.

The cleaned microspheres are transferred from the first accommodating screen 35 of the flush module 3 to the second accommodating screen of the shell formation module 4, the microspheres served as core spheres are totally immersed in the shell liquid, the magnetic vibration generator 61 is turned on to allow the shell liquid to fully contact with the core spheres, thus forming a shell around each of the core spheres, and the assembly process is completed to obtain the required compositional structures or the bio-blocks.

The shell formation step is repeated with the proper shell liquid to obtain multi-shell bio-blocks.

The control device can be, for example, the control device such as a PLC control system or a computer to realize the corresponding control functions in the prior art.

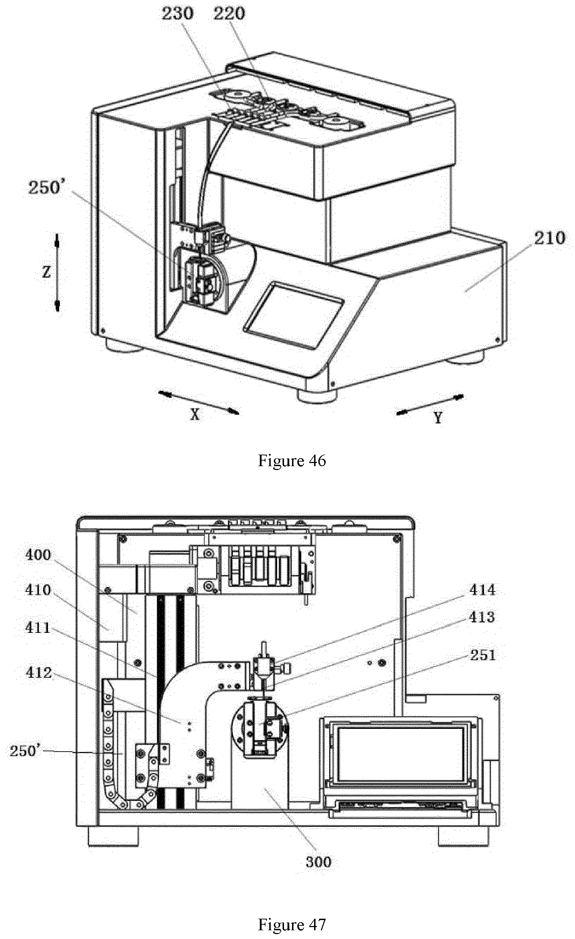

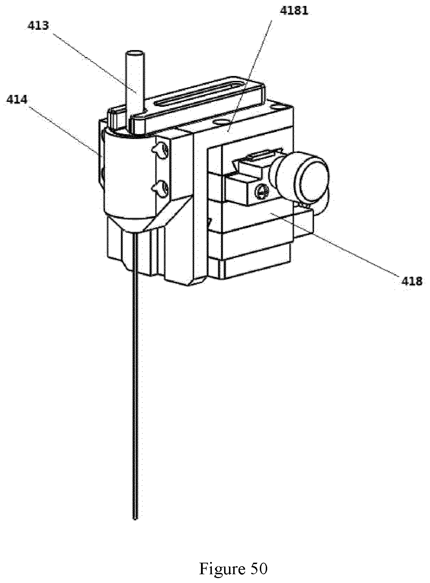

As shown in Figures 46 to 50, the present disclosure further provides a shell assembly device of another embodiment.

As shown in Figure 46, the shell assembly device comprises a shell assembly rack 210, a shell preparation material pumping device, a shell preparation material storage device 220, a fluid switching tube 230, a switching control device and an assembling device 250'. In the shell assembly device of the embodiment, the shell assembly rack 210, the shell preparation material pumping device, the shell preparation material storage device 220, the fluid switching tube 230 and the switching control device are the same as the corresponding structures in the shell assembly device of the embodiments shown in Figure 22 to 27, which are not repeated here. The structure and working principle of the assembling device 250' are different.

As shown in Figures 46 to 50, the assembling device 250' comprises an assembling tube 251, an assembling tube control unit 300 and a liquid adding/sucking unit 400.

An internal space of the assembling tube 251 forms an assembling space. The assembling space has the same functions as the assembling space of the aforementioned assembling tube 251.

The assembling tube control unit 300 comprises an assembling tube fixing portion and an assembling tube driving portion. The assembling tube fixing portion is configured to fix the assembling tube 251. The assembling tube driving portion is in driving connection with the assembling tube fixing portion to drive the assembling tube fixing portion and the fixed assembling tube 251 fixed thereto to rotate. Such arrangement facilitates the mixing of the substances in the assembling tube 251 by driving the assembling tube 251 to rotate or swing.

The assembling tube control unit 300 comprises a mounting base 310. The assembling tube driving portion and the assembling tube fixing portion are arranged on the mounting base 310, and output ends of the assembling tube driving portion and the assembling tube fixing portion are fixedly connected with each other.

The assembling tube fixing portion comprises an assembling tube accommodating base 312, a limit block mounting base 315, a limit stop 313 and a limit screw rod 314. The assembling tube accommodating base 312 is fixedly connected with the output end of the assembling tube driving portion and has an assembling tube accommodating space for accommodating the assembling tube 251. A limit stop mounting base 315 is arranged on the assembling tube accommodating base 312, and has a mounting base threaded hole communicated with the assembling tube accommodating space. The limit stop 313 is arranged in the limit block mounting base 315, and has a limit surface facing toward the assembling tube accommodating space. The limit screw rod 314 is in thread fit with the mounting base threaded hole, and an end of the limit screw rod 314 close to the assembling tube accommodating space is fixedly connected with the limit stop 313.

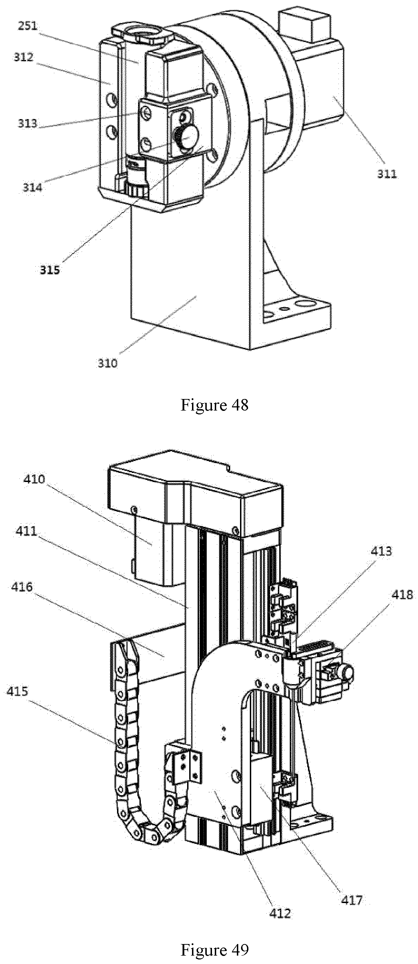

As shown in Figures 47 and 48, in some embodiments, the mounting base 310 is mounted on the shell assembly rack 210. An assembling tube driving motor 311 acting as the assembling tube driving portion is mounted on an upper portion of the mounting base 310. The assembling tube accommodating base 312 is fixedly connected with the output end of the assembling tube driving motor 311. Driven by the assembling tube driving motor 311, the assembling tube accommodating base 312 can rotate within a small angle range. The assembling tube 251 is mounted in the assembling tube accommodating space located in the assembling tube accommodating base 312. The limit stop mounting base 315 is mounted in the middle of the assembling tube accommodating base 312. The limit stop 313 is mounted in the limit block mounting base 315. A side wall of the limit stop mounting base 315 is provided with a threaded hole. The limit screw rod 314 is arranged in the threaded hole. The end portion of the limit screw rod 314 is connected with the limit stop 313. The limit screw rod 314 is rotated to move the limit screw rod 314 relative to the limit stop mounting base 315, thus driving the limit block 313 to move and fix the assembling tube 351.

The liquid adding/sucking unit 400 comprises a feeding needle 413 and a feeding needle moving mechanism. The feeding needle moving mechanism is in driving connection with the feeding needle 413, to make the feeding needle 413 move relative to the assembling tube fixing portion, thus changing the relative position of the feeding needle 413 and the assembling tube 251.

As shown in Figures 47, 49, and 50, the liquid adding/sucking unit 400 comprises a base mounted on the shell assembly rack 210, the feeding needle moving mechanism mounted on the base, and the feeding needle 413 and a limit mechanism mounted on the feeding needle moving mechanism.

The feeding needle moving mechanism comprises a feeding needle vertical driving mechanism for driving the feeding needle 413 to move in a vertical direction (z-axis direction). The feeding needle vertical driving mechanism comprises a vertical guide rail assembly, a translation driving portion and a moving arm 412. The vertical guide rail assembly comprises a guide rail and a translation portion moving vertically along the rail. The translation driving portion is in driving connection with the translation portion, thus making the translation portion move vertically along the guide rail. The moving arm 412 is fixedly connected with the translation portion of the vertical guide rail assembly, and the feeding needle 413 is arranged on the moving arm 412.

The liquid adding/sucking unit 400 comprises the limit mechanism for limiting the moving position of the feeding needle 413 in the vertical direction. The limit mechanism comprises a limiting chain connected with the moving arm 412.

The feeding needle moving mechanism further comprises a horizontal position adjustment mechanism 418 for regulating the horizontal position of the feeding needle 413. The feeding needle 413 is connected onto the moving arm 412 via the horizontal position adjustment mechanism 418.

The vertical guide rail assembly of the vertical driving mechanism of the feeding needle moving mechanism comprises a lead screw rail 411 vertically mounted on the base. The translation driving portion comprises a motor 410. The lead screw of the lead screw rail 411 is driven by the motor 410 to rotate. The moving portion is in thread fit with the lead screw to realize translation as the screw rotates. The moving arm 412 is connected with the moving portion, so that the moving arm 412 moves vertically by rotating the lead screw. The guide rail of the lead screw rail 411 is configured to prevent the moving portion from rotating and guiding moving direction of the moving arm 412. A mounting plate 416 is mounted in the middle of the guide rail, and the limiting chain 415 acting as the limit mechanism is mounted between the mounting plate 416 and the moving arm 412.

A needle clamping portion 414 is mounted at one end of the moving arm 412. A feeding needle 413 for adding and sucking the liquid is arranged on the needle clamping portion 414. The needle clamping portion 414 is provided with a connecting member 4181 and a horizontal position adjustment mechanism 418. The needle clamping portion 414 is connected by the connecting member 4181, and the feeding needle 413 is mounted on the horizontal position adjustment mechanism 418. The horizontal position adjustment mechanism 418 is configured to finely tune the position of the feeding needle 413 in a first horizontal direction (X-axis direction) and a second horizontal direction (Y-axis direction). The X-, Y- and Z-axis directions are shown in Figure 46. The position of the feeding needle 413 in the assembling tube 251 is finely tuned by the horizontal position adjustment mechanism 418, for example, the feeding needle 413 approaches a tube wall of the assembling tube 251, so that the liquid in the assembling tube 251 can be fully sucked. The horizontal position adjustment mechanism 418 can be a cross-roller sliding table or a dovetail groove sliding table.

A lower end of the feeding needle 413 is located directly above the assembling tube 251, and an upper end of the feeding needle 413 is communicated with the outlet connector 233 of the fluid switching tube 230.

The liquid adding/sucking unit 400 further comprises a liquid level sensor 417, and the shell assembly device comprises a control device. The liquid level sensor 417 is configured to acquire a detection signal characterizing a stratified interface position of the substances in the assembling tube (251). For example, the liquid level sensor 417 is configured to detect the position of the interface between the liquid and the microsphere sediment in the assembling tube 251. The liquid level sensor 417 is mounted at a lower portion of the moving arm 412. The control device is in signal connection with the liquid level sensor 417 and the feeding needle moving mechanism to operate the feeding needle moving mechanism to adjust the position of the feeding needle 413 according to the detection signal from the liquid level sensor 417.

The steps of assembling a single shell around each microsphere by the shell assembly device of the embodiment are as follows:
1. The microspheres are separated from the shear liquid and the microspheres are loaded into the assembling tube 251; the assembling tube 251 is mounted on the assembling tube accommodating base 312; the limit stop 313 is pushed to the assembling tube 251 by rotating the limit screw rod 314; and the assembling tube 251 is fixed in the assembling tube accommodating base 312. The microspheres and the shear liquid can be separated, for example, through centrifugal separation. A centrifuge and other devices can be used to separate the microspheres from the shear liquid in a centrifugal manner.
2. An appropriate amount of the cleaning liquid is added via the feeding needle 413 according to the quantity of the microspheres in the assembling tube 251, and then an upper cover of the assembling tube 251 is covered. The assembling tube control unit 300 is started to drive the assembling tube 251 to shake from side to side, thus cleaning the microspheres in the assembling tube 251. The cleaned microspheres and the liquid waste are stratified by centrifugation after the cleaning. The motor 410 is controlled to drive the moving arm 412 to move and drive the liquid level sensor 417 arranged on the moving arm 412 to approach the stratified interface between the microspheres and the liquid waste, and then the liquid level sensor 417 is started to detect the stratified interface between the microspheres and the liquid waste. The upper cover of the assembling tube 251 is opened when the position of the stratified interface is detected. The liquid level sensor 417 transmits the detection signals to the control device which controls the motor 410 to operate and drive the moving arm 412 to move along the lead screw rail 411, so that the feeding needle 413 can move to the stratified interface. The feeding needle 413 sucks the liquid waste while moving, until the tail end of the feeding needle 413 reaches the stratified interface, so that the liquid waste can be discharged. To better suck the liquid waste, the position of the feeding needle 413 in the assembling tube 251 can be adjusted during the suction.
3. The shell liquid is added to the assembling tube 251 via the feeding needle 413, and then the upper cover of the assembling tube 251 is closed. The assembling tube control unit 300 is started to drive the assembling tube 251 to shake from side to side, thus assembling the shells for the microspheres.
4. After the shells of the microspheres are assembled, the assembled bio-blocks and the residual shell liquid are stratified by centrifugation. And then the motor 410 is controlled to drive the moving arm 412 to move, and the moving arm 412 drives the liquid level sensor 417 to approach the stratified interface between the bio-blocks and the residual shell liquid. And then the liquid level sensor 417 is started to detect the stratified interface between the bio-blocks and the residual shell liquid. The upper cover of the assembling tube 251 is opened when the position of the stratified interface is detected. The liquid level sensor 417 transmits the detection signals to the control device which controls the motor 410 to operate and drive the moving arm 412 to move along the lead screw rail 411, so that the feeding needle 413 can move to the stratified interface. The feeding needle 413 sucks the residual shell liquid while moving, until the tail end of the feeding needle 413 reaches the stratified interface, so that the residual shell liquid can be discharged.
5. An appropriate amount of the cleaning liquid is added to the assembling tube 251 via the feeding needle 413, and then the upper cover of the assembling tube 251 is covered. The assembling tube control unit 300 is started to drive the assembling tube 251 to shake from side to side, thus cleaning the bio-blocks in the assembling tube 251. The microspheres and the liquid waste are stratified by centrifugation after the cleaning. And then the motor 410 is controlled to drive the moving arm 412 to move, and the moving arm 412 drives the liquid level sensor 417 to approach the stratified interface between the bio-blocks and the liquid waste. The liquid level sensor 417 is started to detect the stratified interface between the bio-blocks and the liquid waste. The upper cover of the assembling tube 251 is opened when the position of the stratified liquid level is detected. The liquid level sensor 417 transmits the detection signals to the control device which controls the motor 410 to operate and drive the moving arm 412 to move along the lead screw rail 411, so that the feeding needle 413 can move to the stratified interface. And then the feeding needle 413 starts to suck the liquid waste for discharge.
6. Finally, the upper cover of the assembling tube 251 is covered and the assembling tube 251 is removed from the assembling tube control unit 300, thus obtaining the bio-block products enclosed in the assembling tube 251. The shell assembly device of the embodiment can also provide multi-shell (e.g. two-shell or three-shell) assembly for a microsphere. For each additional shell, just repeat the step 3 to step 5 once.

The bio-block constructed by the bio-block preparation instrument of the present disclosure is a basic unit containing cells, and can be used in multiple fields including bio-printing such as 3D bio-printing, tissue engineering and regenerative medicine.

In the embodiment, the bio-block preparation instrument and the preparation method thereof only require simple manual transfer operations, thus reducing the manual involvement in key preparation steps and the error rate caused by manual operation, and making the microsphere preparation more controllable. The mechanized and automatic control ensures that the prepared microsphere products are uniform and the preparation process is stable, thus producing uniform products in different batches and delivering a better application effect of the microspheres in the 3D bio-printing.

In the above embodiments, cross reference can be made for description of components with corresponding functions, and such components is interchangeable and reconstructable. For example, the bio-ink cartridges or the bio-ink cartridge assemblies involved in the embodiments of Figure 1 to Figure 32 can be applied to the bio-block preparation instruments or the microsphere preparation devices involved in the embodiments of Figure 33 to 45, and vice versa.

In addition, although the present disclosure is suitable for preparing a shell-core structure with biological activity, it does not exclude the application of the bio-ink cartridge, the microsphere preparation device, the shell assembly device and the bio-preparation instrument of the disclosure in the preparation of the shell-core structure without biological activity.

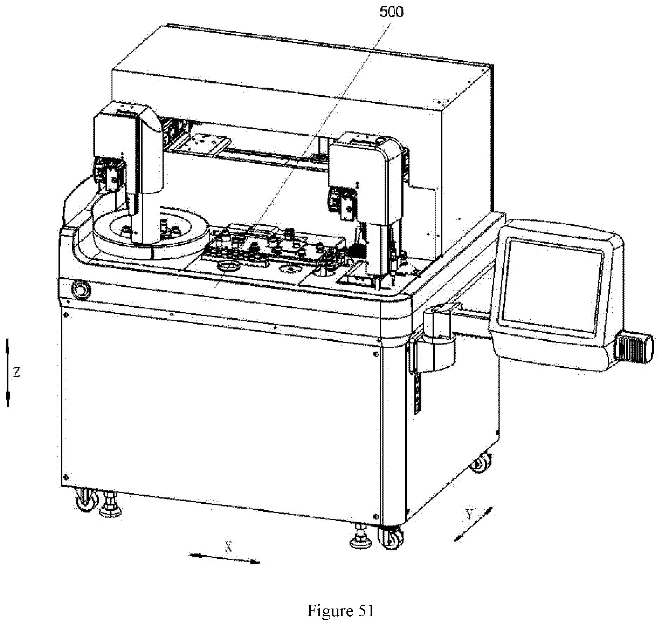

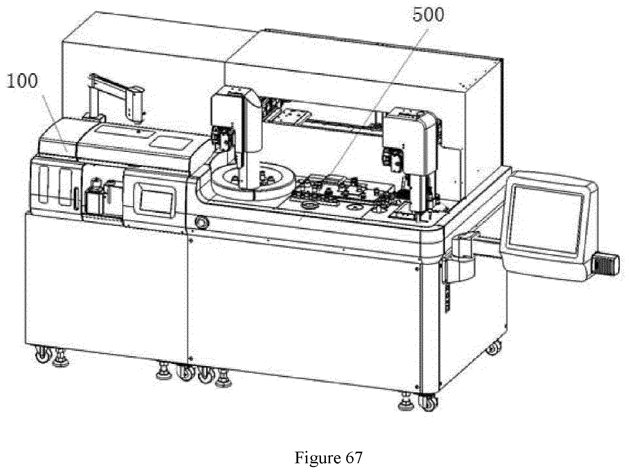

Figure 51 to 66 show a bio-ink preparation instrument according to an embodiment of the disclosure. The bio-ink preparation instrument comprises a shell assembly device according to an embodiment of the disclosure. Figure 67 shows a bio-ink preparation system according to an embodiment of the disclosure.

As shown in Figures 51 to Figure 54, the shell assembly device of the bio-ink preparation instrument is configured to coat the microspheres or the compositional structures composed of a microsphere and at least one shell. The shell assembly device comprises a shell preparation material storage device 55 and an assembling device. The shell preparation material storage device 55 comprises multiple storage spaces which comprise a cleaning liquid storage space 5503 for storing a cleaning liquid and a shell liquid storage space for storing a shell liquid. In the assembling device, cleaning liquid cleans the microspheres or the compositional structures, wherein the cleaned microspheres or compositional structures are coated with the shell liquid to form an outer shell around each of the microspheres or compositional structures. The shell assembly device is capable of producing bio-blocks which are a raw material of the bio-ink.

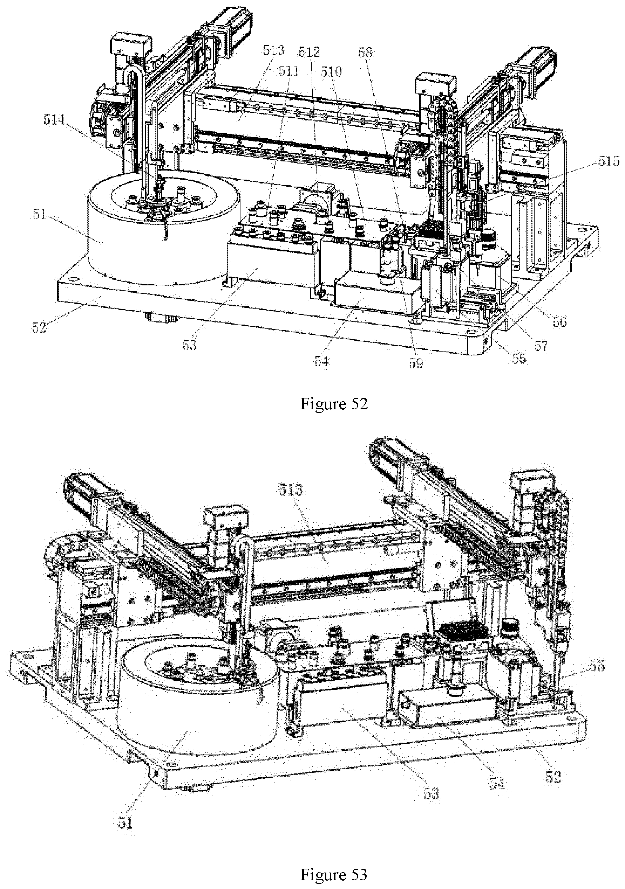

As shown in Figures 52 to 56, the shell preparation material storage device 55 comprises a storage box mounting base 5502 and a storage box 5501. The storage box 5501 is arranged on the storage box mounting base 5502, and has a cleaning liquid storage space 5503 and a shell liquid storage space.

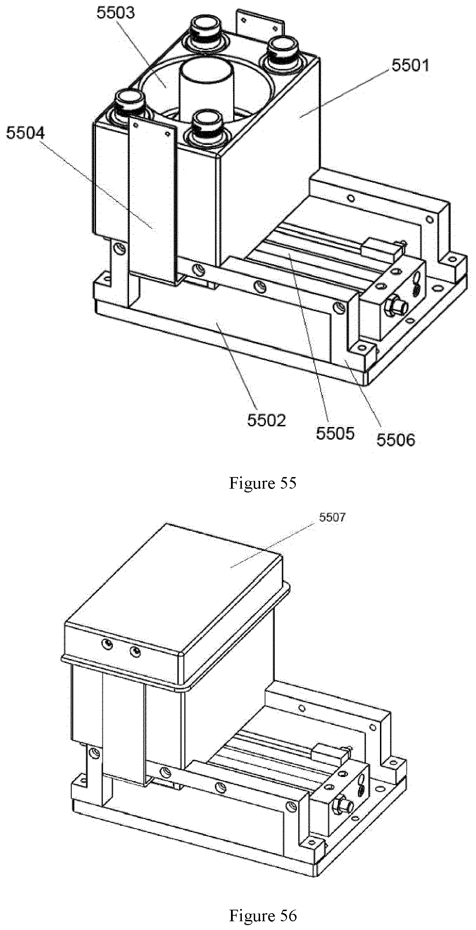

As shown in Figure 56, the shell preparation material storage device 55 further comprises a storage box mounting rail 5506, a storage box cover 5507 and a cover moving mechanism. Two storage box mounting rails 5506 are mounted side by side at both sides of the storage box mounting base 5502, and the storage box 5501 is mounted on the two storage box mounting rails 5506. The storage box cover 5507 is arranged movably relative to the storage box 5501, and covers above the storage box 5501 in a closure state and leaves above the storage box 5501 in an open state. The cover moving mechanism, which is in driving connection with the storage box cover 5507, enables the storage box cover 5507 to move relative to the storage box 5501, thus switching the storage box cover 5507 between the closure state and the open state. The cover moving mechanism and the related arrangement allow the storage box cover 5507 to be opened and closed automatically, thus operating the shell assembly device in a more automatic way.

As shown in Figures 52 to 54 and 57, the assembling device comprises a centrifuge tube and a centrifugal unit 51. The centrifuge tube comprises an assembling space, in which the cleaning liquid cleans the microspheres or the compositional structures. The centrifugal unit 51 is configured to centrifuge the substances in the centrifuge tube. The centrifugal unit 51 helps rapidly separate intermediates or finished products from the cleaning liquid or shell liquid, thereby improving the preparation efficiency.

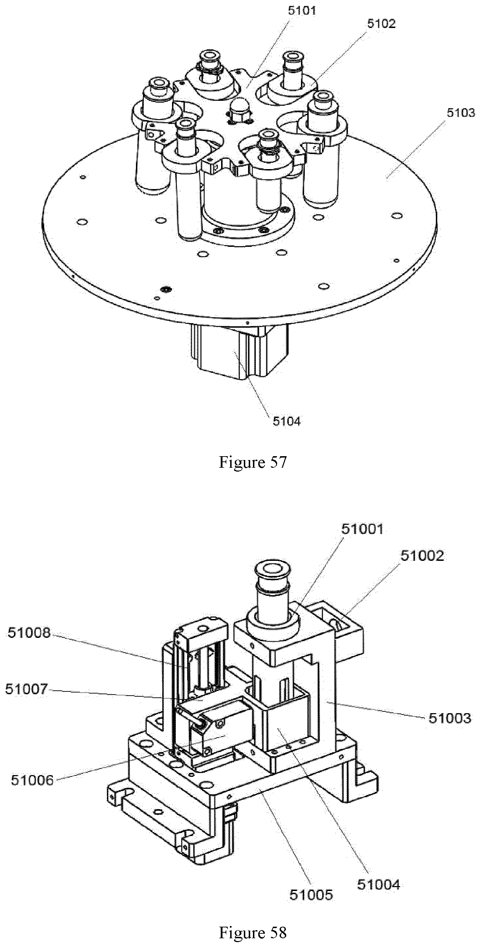

As shown in Figure 57, the centrifugal unit 51 comprises a centrifuge rack mounting portion 5103, a centrifuge rack 5101 and a centrifugal motor 5104. The centrifuge rack 5101 is rotatably mounted on the centrifuge rack mounting portion 5103 about the centrifugal axis. The centrifuge rack 5101 comprises a centrifuge tube bearing portion for mounting the centrifuge tube. The centrifuge tube bearing portion and the centrifugal axis are arranged at intervals. The centrifugal motor 5104 is installed on the centrifuge rack mounting portion 5103 and is in driving connection with the centrifuge rack 5101 for driving the centrifuge rack 5101 to rotate.

As shown in Figure 57, the centrifuge rack 5101 comprises multiple centrifuge tube bearing portions of different specifications distributed evenly around the centrifugal axis. Such arrangement helps separate the substances in multiple centrifuge tubes simultaneously. Alternatively, a balance tube can be provided to balance the centrifuge tube, which is conducive to the smooth progress of the centrifugation.

As shown in Figure 57, the centrifuge tube bearing portion is rotatably arranged relative to the centrifuge rack 5101 about a rotation axis perpendicular to the centrifugal axis. The centrifuge tube bearing portion is, for example, a first centrifuge tube mounting cylinder 5102. Such arrangement helps separate the substances in the centrifuge tube.

As shown in Figures 52 to 54, 58 and 59, the assembling device further comprises a centrifuge tube storage unit 510. The centrifuge tube storage unit 510 comprises a centrifuge tube mounting rack 51003. The centrifuge tube mounting rack 51003 has a centrifuge tube storage portion for storing the centrifuge tube. The centrifuge tube storage portion is, for example, a second centrifuge tube mounting cylinder 51001.

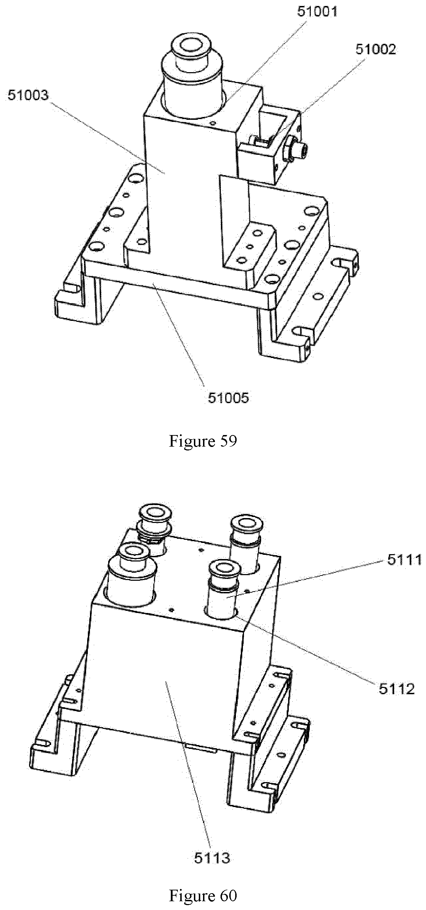

As shown in Figures 58 and 59, the centrifuge tube mounting rack 51003 also has a positioning member mounting hole communicated with the centrifuge tube storage portion. The centrifuge tube storage unit 510 comprises a centrifuge tube positioning member which is mounted in the positioning member mounting hole, and the centrifuge tube positioning member is configured to fix the centrifuge tube together with the centrifuge tube storage portion. The centrifuge tube positioning member is, for example, a finger cylinder 51002.

As shown in Figure 58, the centrifuge tube storage unit 510 comprises a liquid level sensor 51006 for detecting a stratified interface of the substances in the centrifuge tube.

As shown in Figure 58, the centrifuge tube storage unit 510 comprises a sensor mounting rack 51007. The sensor mounting rack 51007 has a centrifuge tube detection zone 51004. The liquid level sensor 51006 is arranged on the sensor mounting rack 51007, and a detection end of the liquid level sensor 51006 is located in the centrifuge tube detection zone 51004. A lower end of the centrifuge tube storage portion is located in the centrifuge tube detection zone 51004, and at least a side of the centrifuge tube storage portion facing the detection end of the liquid level sensor 51006 is communicated with the centrifuge tube detection zone 51004.

As shown in Figure 58, the centrifuge tube storage unit 510 comprises a sensor moving mechanism 51008. The sensor moving mechanism 51008 is in driving connection with the sensor mounting rack 51007, and is used to drive the liquid level sensor 51006 to change the position.

As shown in Figures 52 to 54 and 60, the assembling device further comprises a balance tube storage unit 511. The balance tube storage unit 511 comprises a balance tube 5111 and a balance tube storage space 5112 for storing the balance tube 5111. The balance tube 5111 is configured to balance the centrifuge tube when the centrifugal unit 51 centrifuges the substances in the centrifuge tube. When balancing the centrifuge tube, the balance tube 5111 is mounted on another centrifuge tube bearing portion in the same group as the centrifuge tube to be balanced.

The balance tube storage unit 511 comprises two or more than two balance tubes with different specifications and two or more than two corresponding balance tube storage spaces 5112 with different specifications. Each of the balance tubes is configured to balance the centrifuge tube with the same specification as the balance tube.

As shown in Figures 52 to 54 and 61, the assembling device comprises a liquid adding/sucking unit 515 for adding a liquid to or sucking the liquid from an assembling space. The liquid adding/sucking unit 515 comprises a pipette 5156.

As shown in Figures 52 to 54, the assembling device comprises a three-dimensional motion unit 513 on which the liquid adding/sucking unit 515 is mounted. The three-dimensional motion unit 513 realizes automatic control of the liquid adding/sucking unit 515 at accurate positions when drawing, adding and sucking the liquid.

As shown in Figures 52 to 54 and 62, the assembling device comprises a grabbing unit 514. The grabbing unit 514 is configured to operate the centrifuge tube. The grabbing unit 514 comprises a manipulator mounting rack 5141 and a manipulator 5142. The manipulator 5142 is mounted on the manipulator mounting rack 5141. The grabbing unit 514 is mounted on the three-dimensional motion unit 513. With the grabbing unit 514, the centrifuge tube, the balance tube and the barrel aftermentioned can move automatically, thus improving the automation level.

As shown in Figures 52 to 54 and 63, the three-dimensional motion unit 513 comprises a first driving component 5131, a second driving component 5132, a third driving component 5133, a fourth driving component 5134, and a fifth driving component 5135.

As shown in Figures 52 to 54 and 63, the grabbing unit 514 is mounted on the first driving component 5131. The first driving component 5131 is configured to drive the grabbing unit 514 to move in a vertical direction. The first driving component 5131 is mounted on the second driving component 5132, and the second driving component 5132 is configured to drive the first driving component 5131 to move in a second horizontal direction. The second driving component 5132 is mounted on the third driving component 5133, and the third driving component 5133 is configured to drive the second driving component 5132 to move in a first horizontal direction. The liquid adding/sucking unit 515 is arranged on the fourth driving component 5134, and the fourth driving component 5134 is configured to drive the liquid adding/sucking unit 515 to move in the vertical direction. The fifth driving component 5135 is arranged on the third driving component 5133. The third driving component 5133 is also configured to drive the fifth driving component 5135 to move in the first horizontal direction. The fourth driving component 5134 is arranged on the fifth driving component 5135, and the fifth driving component 5135 is configured to drive the fourth driving component 5134 to move in the second horizontal direction.

As shown in Figures 52 to 54 and 63, the first driving component 5131, the second driving component 5132, the third driving component 5133, the fourth driving component 5134 and the fifth driving component respectively comprise a lead screw guide rail, a motor and a moving block. The lead screw guide rail comprises a rail and a lead screw arranged on the guide rail. The motor is in driving connection with the lead screw to drive the lead screw to rotate. The moving block is in thread fit with the lead screw guide rail to move along the guide rail as the lead screw rotates.

As shown in Figures 52 to 54, the assembling device comprises a residual liquid absorbing unit 53. The residual liquid absorbing unit 53 comprises a residual liquid absorbing mounting rack 5301 and an oil absorption sponge 5302 arranged on the residual liquid absorbing mounting rack 5301.

As shown in Figures 52 to 54, the shell assembly device comprises a liquid waste collection unit 56. The liquid waste collection unit 56 comprises a liquid waste collection bottle.

As shown in Figures 52 to 54, the assembling device comprises a tip storage unit 57. The tip storage unit 57 is configured to store a tip required by the pipette 5156.

As shown in Figures 51 to 54, the bio-ink preparation instrument of the embodiment comprises the shell assembly device of the embodiment. The bio-ink preparation instrument further comprises a bio-ink generation device for mixing the bio-blocks formed by the shell assembly device and a carrier material to generate the bio-ink.

As shown in Figures 51 to 54 and 61, the bio-ink generation device comprises a barrel and a carrier material loading unit. The barrel has a mixing space for mixing bio-blocks and the carrier material. The carrier material loading unit is configured to inject the carrier material into the mixing space. The carrier material loading unit is mounted on the liquid adding/sucking unit 515 of the shell assembly device, so that the carrier material loading unit driven by the three-dimensional motion unit 513 can move.

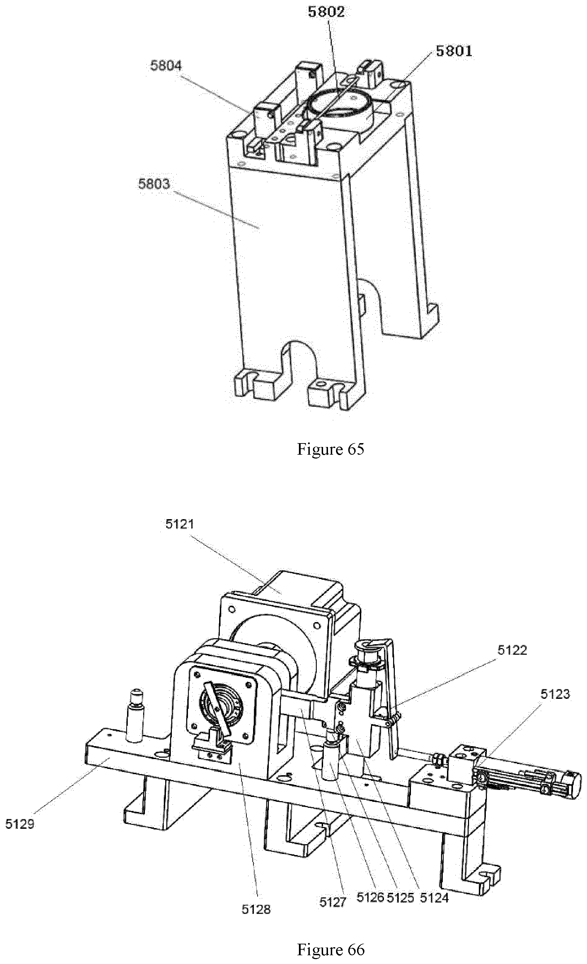

As shown in Figure 66, the carrier material loading unit comprises a syringe 5153. The carrier material loading unit further comprises a syringe pushing portion in driving connection with the syringe for pushing a piston of the syringe to move. The syringe pushing portion improves the automation level of the bio-ink generation device.

The syringe pushing portion can comprise, for example, a slider 5152 and a syringe driving motor 5151. The slider 5152 is movably arranged along the axis of the syringe 5153 to push the syringe 5153. The syringe driving motor 5151 is in driving connection with the slider 5152 for moving the slider 5152 to push the syringe 5153.

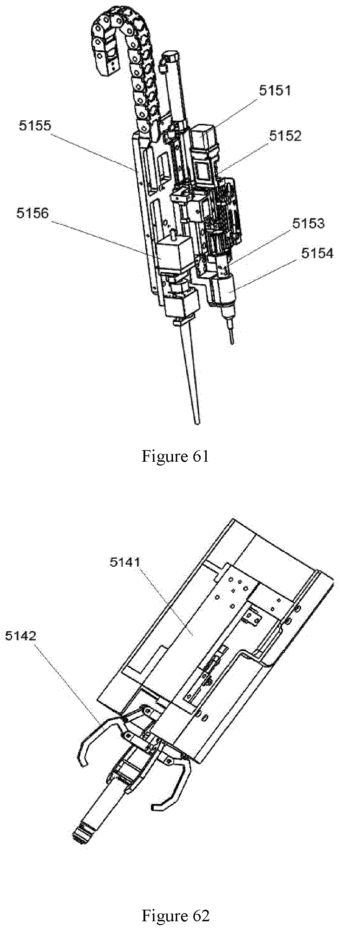

As shown in Figure 61, the carrier material loading unit comprises a syringe thermal insulation device 5154 arranged on a surface of the syringe 5153 for thermal insulation thereof. The syringe thermal insulation device 5154 helps maintain the activity of the bio-blocks in the bio-ink.

As shown in Figures 52 to 54 and 64, the bio-ink generation device comprises a weighing unit 54. The weighing unit 54 comprises an electronic scale 5402 and a weighing tray 5401 arranged on the electronic scale 5402. The weighing tray 5401 comprises a hollow cylinder structure for storing the syringe. The weighing unit 54 allows adding a proper amount of the carrier material to the bio-blocks, thus avoiding insufficient or excessive carrier material.

As shown in Figures 52 to 54 and 65, the bio-ink generation device comprises a scraping unit 58. The scraping unit 58 comprises a correction mounting rack 5803 as well as a scraping plate 5801, a scraping line 5802 and a distance measuring sensor 5804 mounted on the correction mounting rack 5803. The scraping unit 58 comprises two distance measuring sensors 5804 arranged oppositely. The scraping unit 58 scrapes off the liquid remaining at the bottom of the pipette 5156 and the syringe 5153, thus helping prevent the pipette 5156 and the syringe 5153 from being blocked. The distance measuring sensor 5804 is configured to fully and accurately scrape off the liquid remaining at the bottom of the pipette 5156 and the syringe 5153.

As shown in Figures 52 to 54, the bio-ink generation device comprises a barrel thermal insulation unit 59 for thermal insulation of the barrel. The barrel thermal insulation unit 59 comprises a thermal insulation box and a heating device for heating the thermal insulation box. The barrel thermal insulation unit 59 helps maintain the activity of bio-blocks in the bio-ink.

As shown in Figures 52 to 54 and 66, the bio-ink generation device comprises a mixing unit 512 for mixing the bio-blocks and the carrier material in the barrel. The mixing unit 512 improves the efficiency of the bio-ink preparation.

As shown in Figure 66, the mixing unit 512 comprises a mixing unit mounting rack 5129, a mixing motor 5121 and a swing arm 5127. The mixing motor 5121 is mounted on the mixing unit mounting rack 5129. The swing arm 5127 is mounted on an output shaft of the mixing motor 5121 and has a barrel bearing portion 5124 for mounting the barrel.

As shown in Figure 66, the mixing unit 512 comprises an angular sensor 5128. The angular sensor 5128 is mounted on the output shaft of the mixing motor 5121 to detect rotation position of the mixing motor 5121.

As shown in Figure 66, the mixing unit 512 comprises a buffer assembly. The buffer assembly is mounted on the mixing unit mounting rack 5129 to buffer and limit the swing arm 5127. The two buffer assemblies are respectively located at both ends of a swing path of the swing arm 5127. The buffer assembly comprises a buffer post 5126 and a buffer cushion 5125. The buffer post 5126 is mounted on the mixing unit mounting rack 5129. The buffer cushion 5125 is arranged on the buffer post 5126 and located between the buffer post 5126 and the corresponding end of the swing path of the swing arm 5127.

As shown in Figure 66, the barrel bearing portion 5124 comprises a barrel mounting hole and a fixing piece mounting portion. The mixing unit 512 comprises a fixing piece 5122, a torsion spring and a pneumatic cylinder 5123. The pneumatic cylinder 5123 is mounted on the mixing unit mounting rack 5129. The fixing piece 5122 comprises a pressing end, a driving end and a hinge portion arranged between the pressing end and the driving end. The hinge portion is hinged to the fixing piece mounting portion via the torsion spring. The driving end is matched with the pneumatic cylinder 5123. The pneumatic cylinder 5123 drives the fixing piece 5122 to rotate about the fixing piece mounting portion, so that the pressing end switches between a pressing state in which the pressing end is pressed against an inlet of the barrel mounting hole and a relief state in which the pressing end stays away from the inlet of the barrel mounting hole.

The embodiments of the present disclosure also provide a bio-ink preparation system which comprises the aforementioned bio-ink preparation instrument.

As shown in Figure 67, in some embodiments, the bio-ink preparation system comprises the microsphere preparation device and the bio-ink preparation instrument aforementioned.

The bio-ink preparation instrument, the shell assembly device thereof, the bio-ink generation device, and the bio-ink preparation system having the bio-ink preparation instrument according to the embodiments of the present disclosure will be further described below with reference to Figures 51 to 67.

As shown in Fig 67, the bio-ink preparation system comprises a microsphere preparation device 100 and a bio-ink preparation instrument 500. The microsphere preparation device 100 is configured to prepare microspheres. The bio-ink preparation instrument 500 is configured to clean the microspheres, coat the microspheres to form the bio-blocks, and mix the bio-blocks with the carrier material to generate the bio-ink. See above description for details about the microsphere preparation device 100. Only the bio-ink preparation instrument 500 comprising the shell assembly device will be described below.

As shown in Figures 51 to 54, the bio-ink preparation instrument 500 comprises a base 52, a shell assembly device, and a bio-ink generation device.

As shown in Figures 52 to 54, the shell assembly device comprises a preparation material storage device 55 and an assembling device. The assembling device comprises a centrifuge tube, a centrifugal unit 51, a centrifuge tube storage unit 510, a balance tube storage unit 511, a liquid adding/sucking unit 515, a three-dimensional motion unit 513, a grabbing unit 514, a residual liquid absorbing unit 53, a liquid waste collection unit 56 and a tip storage unit 57.

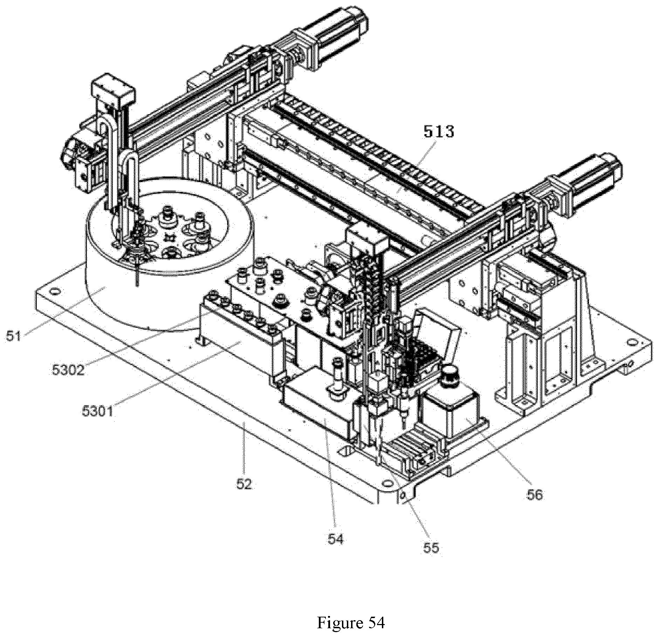

As shown in Figure 54, the residual liquid absorbing unit 53 comprises a residual liquid absorbing mounting rack 5301 on which several oil absorption sponges 5302 are arranged.

As shown in Figures 55 and 56, the shell preparation material storage device 55 has multiple storage spaces which comprise a cleaning liquid storage space for storing a cleaning liquid and a shell liquid storage space for storing a shell liquid. The shell preparation material storage device 55 comprises a storage box mounting base 5502 above which a storage box 5501 is arranged. The cleaning liquid storage space 5503 is arranged in the middle of the storage box 5501. The shell liquid storage space is arranged in the storage box 5501 and close to outer wall thereof. There are four shell liquid storage spaces respectively distributed at four corners of the storage box 5501. Storage box mounting rails 5506 are respectively arranged at both sides of the storage box mounting base 5502. The cover moving mechanism comprises a lead screw guide rail 5505 arranged between the two storage box mounting rails 5506. The lead screw guide rail 5505 is connected with a motor at one end and a slider 5504 at the other end. A storage box cover 5507 is arranged above the slider 5504. The storage box cover 5507 moves relative to the storage box 5501 through the movement of the slider 5504 on the storage box mounting rail 5506, thus switching between an closure state and an open state.

As shown in Figure. 57, the centrifugal unit 51 comprises a centrifuge rack mounting portion 5103 above which a centrifuge rack 5101 is mounted and below which a centrifugal motor 5104 is mounted. The centrifugal motor 5104 is connected with the centrifuge rack 5101 and drives the centrifuge rack 5101 to rotate. A first centrifuge tube mounting cylinder 5102 is arranged on the centrifuge rack 5101. There are six first centrifuge tube mounting cylinders 5102 in total. The six first centrifuge tube mounting cylinders 5102 are evenly distributed at the edge of the centrifuge rack 5101. Each of the first centrifuge tube mounting cylinders 5102 constitutes a centrifuge tube bearing portion. The six first centrifuge tube mounting cylinders 5102 are divided into three groups according to specifications, and each group comprises two first centrifuge tube mounting cylinders 5102 with the same specifications arranged at intervals of 180 degrees. One of the two first centrifuge tube mounting cylinders 5102 in each group is configured to place the centrifuge tube, and the other is configured to place the balance tube corresponding to the centrifuge tube.

In the embodiment, two structures of the centrifuge tube storage units 510 are provided.

As shown in Figure 58, the centrifuge tube storage unit 510 with a first structure is configured to store a 15ml centrifuge tube and a 5ml syringe. The centrifuge tube storage unit 510 with the first structure comprises a centrifuge tube mounting base 51005 on which a centrifuge tube mounting rack 51003 and a sensor moving mechanism 51008 are arranged. The centrifuge tube mounting rack 51003 is provided with a second centrifuge tube mounting cylinder 51001 acting as a centrifuge tube storage portion. A lower portion of the centrifuge tube mounting rack 51003 is provided with a sensor mounting rack 51007. A finger cylinder 51002 is mounted on a side wall of the centrifuge tube mounting rack 51003. The sensor mounting rack 51007 forms a centrifuge tube detection zone 51004 at the end of the second centrifuge tube mounting cylinder 5100. The centrifuge tube detection zone 51004 is surrounded by four plates. The sensor mounting rack 51007 is connected with the sensor moving mechanism 51008 at one side of the other end thereof. The sensor moving mechanism 51008 drives the sensor mounting rack 51007 to move up and down. A liquid level sensor 51006 is arranged at the other side of the other end of the sensor mounting rack 51007. A detection end of the liquid level sensor 51006 is located in the centrifuge tube detection zone 510007. The detection end is, for example, a transmission end that emits light. The light emitted from the transmission end enters the centrifuge tube detection zone 51004 and illuminates the centrifuge tube. The centrifuge tube storage unit 510 with the first structure is configured to store the centrifuge tube in which the position of the stratified interface needs to be detected.

As shown in Figure 59, the centrifuge tube storage unit 510 with a second structure is configured to store 50ml centrifuge tubes. The centrifuge tube storage unit 510 with the second structure comprises a centrifuge tube mounting base 51005 on which a centrifuge tube mounting base 51003 is mounted. A second centrifuge tube mounting cylinder 5100 acting as a centrifuge tube storage portion is arranged at an upper end of the centrifuge tube mounting rack 51003. A finger cylinder 51002 is mounted on a side wall of the centrifuge tube mounting rack 51003. The centrifuge tube storage unit 510 with the second structure is configured to store the centrifuge tubes in which the position of the stratified interface does not need to be detected.

As shown in Figure 60, the balance tube storage unit 511 comprises a balance tube storage box 5113. A balance tube storage space 5112 is arranged on the balance tube storage box 5113. There are four balance tube storage spaces 5112 with different specifications. The four balance tube storage spaces 5112 are evenly distributed at four corners of the balance tube storage box 5113. A balance tube 5111 with corresponding specification is mounted in the balance tube storage space 5112.

As shown in Figure 61, the liquid adding/sucking unit 515 comprises a fixing plate 5155 connected with the fourth driving component 5134. A pipette 5156 is arranged on the fixing plate 5155.

As shown in Figure 62, the grabbing unit 514 comprises a manipulator mounting rack 5141 which is connected with the first driving component 5131 and on which a manipulator 5142 is arranged.

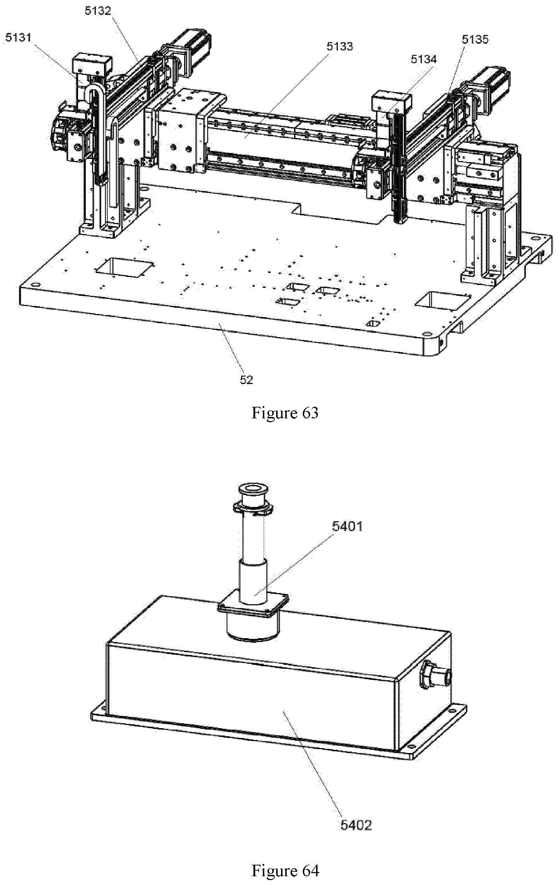

As shown in Figure 63, in the three-dimensional motion unit 513, a second driving component 5132 and a fifth driving component 5135 are respectively arranged at both ends of a third driving component 5133, and the third driving component 5133 is configured to drive the second driving component 5132 and the fifth driving component 5135 to move in a first horizontal direction (X-axis direction). A first driving component 5131 is arranged on the second driving component 5132, and the second driving component 5132 is configured to drive the first driving component 5131 to move in a second horizontal direction (Y-axis direction). A grabbing unit 514 is arranged on the first driving component 5131 which is configured to drive the grabbing unit 514 to move in a vertical direction (Z-axis direction). A fourth driving component 5134 is arranged on the fifth driving component 5135 which is configured to drive the fourth driving component 5134 to move along in the second horizontal direction. The fourth driving component 5134 is provided with a liquid adding/sucking unit 515 and is used to drive the liquid adding/sucking unit 515 to move in the vertical direction. Each of the first to the fifth driving component comprises a lead screw guide rail, a motor and a moving block.

The X-, Y- and Z-axis directions are shown in Figure 51.

As shown in Figures 52 to 54, the liquid waste collection unit 56 comprises a liquid waste collection bottle. The tip storage unit 57 comprises a tip storage box.

As shown in Figures 52 to 54, the bio-ink generation device comprises a carrier material loading unit, a scraping unit 58, a weighing unit 54, a barrel thermal insulation unit 59 and a mixing unit 512.

As shown in Figure 61, the carrier material loading unit is also arranged on the fixing plate 5155 of the liquid adding/sucking unit 515. The carrier material loading unit comprises a syringe driving motor 5151, a slider 5152, a syringe 5153, and a syringe thermal insulation device 5154. The syringe driving motor 5151 is connected with the slider 5152 at an output end for driving the slider 5152 to move. During movement, the slider 5152 is in contact with a piston of the syringe 5153, thus pushing the piston of the syringe 5153. The syringe thermal insulation device 5154 is arranged on a surface of the syringe 5153.

As shown in Figure 65, the scraping unit 58 comprises a scraping plate 5801, a scraping line 5802, a correction mounting rack 5803, and a distance measuring sensor 5804. The scraping plate 5801, the scraping line 5802 and the distance measuring sensor 5804 are arranged on the correction mounting rack 5803. The scraping line 5802 is located above the scraping plate 5801. There are two distance measuring sensors 5804 which are arranged oppositely. As shown in Figure 65, the distance measuring sensor 5804 is arranged beside the scraping line 5802 and the scraping plate 5801. The distance measuring sensor 5804 and the scraping line 5802 are located on the same horizontal plane. The distance measuring sensor 5804 is essentially configured to measure the height of lower ends of the pipette 5156 and the syringe 5153 respectively to keep the height of the lower ends of the pipette 5156 and the syringe 5153 at the same level as the scraping line 5802. Since residual liquid may be suspended at the lower ends of the pipette 5156 and the syringe 5153 during use (e.g., droplets with high viscosity, which may cause blocking), the liquid remaining at the lower ends of the pipette 5156 and the syringe 5153 needs to be scraped off. Before scraping, the pipette 5156 and the syringe 5153 need to be positioned, so that when the pipette 5156 and the syringe 5153 move to the scraping line 5802, the liquid remaining at the lower end of the pipette 5156 and the syringe 5153 can be scraped off via the scraping line 5802.

As shown in Figure 64, the weighing unit 54 comprises an electronic scale 5402 above which a weighing tray 5401 with hollow cylinder structure is arranged. The hollow cylinder structure is configured to store the 5ml syringe.

The barrel thermal insulation unit 59 comprises a thermal insulation box, and a heating device is arranged at the bottom thereof.

As shown in Figure 66, the mixing unit 512 comprises a mixing unit mounting rack 5129 on which a mixing motor 5121 and an pneumatic cylinder 5123 are arranged. A swing arm 5127 is arranged on an output shaft of the mixing motor 5121. An angular sensor 5128 for detecting rotation position of the mixing motor 5121 is also arranged on the output shaft of the mixing motor 5121. A buffer assembly configured to buffer and limit the swing arm 5127 is also arranged on the mixing unit mounting rack 5129. There are two buffer assemblies. The two buffer assemblies are located on both sides of the angular sensor 5128. The buffer assembly comprises a buffer post 5126 on which a buffer cushion 5125 is arranged. The swing arm 5127 is provided with a barrel bearing portion 5124 at one end thereof. A fixing piece mounting portion is arranged in the middle of the barrel bearing portion 5124. A fixing piece 5122 is L-shaped. An end close to the L corner of the fixing piece 5122 is a pressing end and the other end away from the L corner is a driving end. A hinge portion is arranged between the pressing end and the driving end. The fixing piece mounting portion is connected with the hinge portion of the fixing piece 5122 via a torsion spring. The driving end presses against a cylinder rod of the pneumatic cylinder 5123. When the cylinder rod extends, the cylinder rod drives the driving end to act, thus making the pressing end in a relief state. When the cylinder rod retracts, the pressing end is in a pressing state, thus fixing the barrel in the barrel mounting hole.

Working processes and principles of the shell assembly and the bio-ink generation by the bio-ink preparation instrument 500 and the bio-ink preparation system according to the embodiments of the present disclosure are described as follows:
1. Steps of sucking the shear liquid
   1.1 Placing the 50ml centrifuge tube, which contains the mixed solution of microspheres and shear liquid as a mixture collection container in the microsphere preparation device 100, to the centrifuge tube storage unit 510; The 50ml centrifuge tube is capped with a plug by the manipulator 5142.
   1.2 The balance tube 5111 corresponding to the 50ml centrifuge tube on the balance tube storage unit 511 is grabbed to the corresponding balancing position on the centrifugal unit 51 by the manipulator 5142. The capped 50ml centrifuge tube is grabbed to the centrifugal unit 51 by the manipulator 5142 to balance the centrifugal unit 51. Then the centrifugal unit 51 is started to centrifuge the 50ml centrifuge tube. After centrifugation, the balance tube 5111 and the 50ml centrifuge tube are placed to the proper positions by the manipulator 5142.
   1.3 The plug on the 50ml centrifuge tube is removed by the manipulator 5142, and then the pipette 5156 is moved to the tip storage unit 57 for mounting the tip. After the mounting, the pipette 5156 is moved to the 50ml centrifuge tube, and the shear liquid is sucked into the liquid waste collection bottle of the liquid waste collection unit 56. Then the tip is replaced, and the above processes are repeated for sucking and discharging the shear liquid at least three times to complete the sucking of the shear liquid. Finally, the oil absorption sponge 5302 is grabbed by the manipulator 5142 from the residual liquid absorbing mounting rack 5301, and the manipulator 5142 inserts the oil absorption sponge 5302 into the 50ml centrifuge tube to further suck the shear liquid (e.g. mineral oil) remaining on the surface of the microspheres.
2. Steps of cleaning the microspheres
   2.1 The tip of the pipette 5156 is replaced, the mixture in the 50ml centrifuge tube is transferred to the 15ml centrifuge tube, and the 15ml centrifuge tube is placed in the centrifuge tube storage unit 510 by the manipulator 5142.
   2.2 The tip of the pipette 5156 is replaced, the pipette 5156 is moved to the cleaning liquid storage space 5503, after a certain amount of the cleaning liquid is sucked, the pipette 5156 is moved to the 15ml centrifuge tube position, and then the cleaning liquid is added to the 15ml centrifuge tube. The pipette 5156 is moved into the 15ml centrifuge tube for suction, so that the cleaning liquid flows in the 15ml centrifuge tube to complete the cleaning of the microdroplets. The 15ml centrifuge tube is capped with a plug by the manipulator 5142. The balance tube on the balance tube storage unit 511 corresponding to the 15 ml centrifuge tube is grabbed to the corresponding position of the centrifugal unit 51 by the manipulator 5142. The capped 15ml centrifuge tube is grabbed to the centrifugal unit 51 by the manipulator 5142 to balance the centrifugal unit 51. Then the centrifugal unit 51 is started to centrifuge the 15ml centrifuge tube. After centrifugation, the balance tube and the 15ml centrifuge tube are placed to the proper positions by the manipulator 5142. After the centrifugation, the shear liquid, the cleaning liquid and the microdroplets in the 15 ml centrifuge tube are stratified.
   2.3 Removal of the plug of the 15 ml centrifuge tube by the manipulator 5142. The tip of the pipette 5156 is replaced, the pipette 5156 is moved to the 15ml centrifuge tube position, and then the shear liquid is sucked into the liquid waste collection unit 56 for discharge. The tip is replaced, and the processes of sucking and discharging the shear liquid in step 2.3 are repeated to complete the suction of the shearing fluid. Finally, the oil absorption sponge 5302 is grabbed from the residual liquid absorbing mounting rack 5301 by the manipulator 5142, and the manipulator inserts the oil absorption sponge into the 15ml centrifuge tube to further suck the residual shear liquid remaining on the surface of the microsphere.
   2.4 After the shear liquid is sucked, the sensor mounting rack 51007 is moved and the level sensor 51006 is started to detect the position of the stratified interface between the microspheres and the cleaning liquid. When the position of the stratified interface is detected, the tip of the pipette 5156 is replaced, and the pipette 5156 is moved to the stratified liquid interface. During the movement, the pipette 5156 starts to suck the cleaning liquid waste while moving, until the end of the pipette 5156 reaches the stratified interface, so that the cleaning liquid waste is sucked and discharged to the liquid waste collection bottle of the liquid waste collection unit 56.

   Steps 2.2 to 2.4 can be repeated to achieve a better cleaning effect.
3. Shell coating steps
   3.1 The tip of the pipette 5156 is replaced, the pipette 5156 is moved to the shell liquid storage space, after a certain amount of the cleaning liquid is sucked, the pipette 5156 is moved to the 15ml centrifuge tube position, and then the shell liquid is added to the 15ml centrifuge tube. The 15ml centrifuge tube can be left standing for a period of time (e.g. 8 minutes) to complete the coating. Alternatively, the coating can be completed by blowing, particularly, the pipette 5156 is moved into in the 15ml centrifuge tube for suction, so that the shell liquid can flow in the 15ml centrifuge tube to complete the coating.
   3.2 The 15ml centrifuge tube is capped with a plug by the manipulator 5142. The balance tube 5111 on the balance tube storage unit 511 corresponding to the 15 ml centrifuge tube is grabbed to the corresponding position of the centrifugal unit 51 by the manipulator 5142. The capped 15ml centrifuge tube is grabbed to the corresponding position of the centrifugal unit 51 by the manipulator 5142 to balance the centrifugal unit 51. Then the centrifugal unit 51 is started to centrifuge the 15ml centrifuge tube. After centrifugation, the balance tube and the 15ml centrifuge tube are placed to the proper positions by the manipulator 5142. The sensor mounting rack 51007 is moved and the level sensor 51006 is started to detect the position of the stratified interface between the microspheres and the residual shell liquid. When the position of the stratified interface is detected, the tip of the pipette 5156 is replaced, and the pipette 5156 is moved to the stratified interface. During the movement, the pipette 5156 starts to suck the residual shell liquid while moving, until the end of the pipette 5156 reaches the stratified interface, so that the residual shell liquid is sucked and discharged to the liquid waste collection bottle of the liquid waste collection unit 56.
   3.3 The tip of the pipette 5156 is replaced, the pipette 5156 is moved to the cleaning liquid storage space 5503, after a certain amount of the cleaning liquid is sucked, the pipette 5156 is moved to the 15ml centrifuge tube, and then adding the cleaning liquid into the 15ml centrifuge tube; The pipette 5156 is moved into the 15ml centrifuge tube for the suction, so that the cleaning liquid flows in the 15ml centrifuge tube to complete the cleaning of the bio-blocks. The 15ml centrifuge tube is capped with a plug by the manipulator 5142. The corresponding balance tube on the balance tube storage unit 511 is grabbed to the corresponding position of the centrifugal unit 51 by the manipulator 5142. The capped 15ml centrifuge tube is grabbed to the centrifugal unit 51 by the manipulator 5142 to balance the centrifugal unit 51. Then the centrifugal unit 51 is started to centrifuge the 15ml centrifuge tube. After centrifugation, placing the balance tube and the 15ml centrifuge tube to the proper place by the manipulator 5142.
   3.4 The plug of the 15 ml centrifuge tube was removed by the manipulator 5142. The tip of the pipette 5156 is replaced. The sensor mounting rack 51007 is moved and the level sensor 51006 is started to detect the position of the stratified interface between the bio-blocks and the cleaning liquid. When the position of the stratified interface is detected, the tip of the pipette 5156 is replaced, and the pipette 5156 is moved to the stratified interface. During the movement, the pipette 5156 starts to suck the cleaning liquid waste while moving, until the end of the pipette 5156 reaches the stratified interface, so that the cleaning liquid waste is sucked and discharged to the liquid waste collection bottle.
      To ensure the coating effect or obtain a multi-shell coating, the above steps 3.1 to 3.4 can be repeated.
4. Steps of generating the bio-ink by mixing the carrier material
   4.1 The barrel is moved to the weighing tray 5401 on the electronic scale 5402 by the manipulator 5142 to weigh the barrel.
   4.2. The tip of the pipette 5156 is replaced, and the pipette 5156 is moved to transfer the bio-blocks in the 15ml centrifuge tube to the barrel.
   4.3 The barrel is capped with a plug by the manipulator 5142. The balance tube corresponding to the barrel on the balance tube storage unit 511 is grabbed to the corresponding position of the centrifugal unit 51 by the manipulator 5142. The capped barrel is grabbed to the corresponding position of the centrifugal unit 51 by the manipulator 5142 to balance the centrifugal unit 51. Then the centrifugal unit 51 is started to centrifuge the barrel.
   4.4 The plug of the barrel is removed by the manipulator 5142. The tip of the pipette 5156 is replaced, the sensor mounting rack 51007 is moved and the level sensor 51006 is started to detect the position of the stratified interface between the microsphere and the cleaning liquid. When the position of the stratified interface is detected, the tip of the pipette 5156 is replaced, and the pipette 5156 is moved to the stratified interface. During the movement, the pipette 5156 starts to suck the cleaning liquid waste while moving, until the end of the pipette 5156 reaches the stratified interface, so that the cleaning liquid waste is sucked and discharged to the liquid waste collection bottle.
   4.4 The carrier material to be added to the syringe 5153 is weighed by the electronic scale 5402. And then the carrier material is added to the syringe 5153. The temperature of the carrier material is kept via the syringe thermal insulation device 5154 at preferably around 37°C.
   4.3 The barrel is moved to the barrel thermal insulation unit 59 by the manipulator 5142 to keep the temperature of bio-blocks in the barrel at preferably around 37C.
   4.4 After the thermal insulation, the syringe 5153 is moved to the barrel containing the bio-blocks. The syringe driving motor 5151 is started to drive the slider 5152 to move, thus moving the piston of the syringe 5153 and then pushing the carrier material in the syringe 5153 into the barrel. The tip of the pipette 5156 is replaced and placing the pipette 5156 in the barrel to perform suction, so that the carrier material flows in the barrel, and the carrier material and the bio-blocks are mixed. Alternatively, the barrel is shaken by the mixing unit 512 to mix the carrier material and the bio-blocks.
   4.5 The barrel is capped a plug by the manipulator 5142. Grabbing the balance tube on the balance tube storage unit 511 to the proper position of the centrifugal unit 51 by the manipulator 5142; Grabbing the capped barrel to the proper position of the centrifugal unit 51 by the manipulator 5142, to balance the centrifugal unit 51; Then the centrifugal unit 51 is started to centrifuge the barrel.
   4.6 The plug of the barrel is removed by the manipulator 5142. The tip of the pipette 5156 is replaced, the sensor mounting rack 51007 is moved and the level sensor 51006 is started to detect the position of the stratified interface between the bio-ink and the residual carrier material; When the position of the stratified interface is detected, the tip of the pipette 5156 is replaced, and the pipette 5156 is moved to the stratified interface. During the movement, the pipette 5156 starts to suck the residual carrier material while moving, until the end of the pipette 5156 reaches the stratified interface, so that the residual carrier material is sucked and discharged to the liquid waste collection bottle. In this way, the preparation of the bio-ink is completed.

The bio-ink generated by the bio-ink preparation instrument and the bio-ink preparation system of the embodiments of the present disclosure meets the requirements of biological activity and bio-printing.

Finally, it should be noted that the above embodiments are only used to explain the technical solutions of the present disclosure and shall not be construed as limitations thereto. Although the present disclosure is described in detail with reference to preferred embodiments, those of ordinary skill in the art should understand the specific embodiment of the present disclosure may still be modified or some technical features may be replaced by equivalent, and without breaking away from the spirit of the technical solutions of the present disclosure, all modified embodiments and replaced technical features shall be covered in the protection scope of the present disclosure.

## Claims

1. A bio-ink cartridge (131), comprising:
an ink cartridge shell (1311) with a cavity;
a dividing wall (1312) arranged in the cavity and dividing the cavity into a first accommodating chamber (1313) and a second accommodating chamber (1314) which are fluidly isolated from each other;
a first ink cartridge inlet (13151) and a first ink cartridge outlet (13153) arranged on the ink cartridge shell (1311) and communicated with the first accommodating chamber (1313);
a second ink cartridge inlet (13152) and a second ink cartridge outlet (13154) arranged on the ink cartridge shell (1311) and communicated with the second accommodating chamber (1314); and
an inner flow channel (1316) located in the second accommodating chamber (1314) and isolated from a fluid therein, and communicating the first accommodating chamber (1313) with the first ink cartridge outlet (13153).

2. The bio-ink cartridge (131) according to claim 1, wherein the first accommodating chamber (1313) is arranged to match with the second accommodating chamber (1314), the first ink cartridge inlet (13151) is arranged adjacent to the second ink cartridge inlet (13152), and the first ink cartridge outlet (13153) is arranged adjacent to the second ink cartridge outlet (13154), and the first ink cartridge inlet (13151), the second ink cartridge inlet (13152), the first ink cartridge outlet (13153) and the second ink cartridge outlet (13154) are arranged on the top or side of the ink cartridge shell (1311).

3. The bio-ink cartridge (131) according to claim 1, wherein the dividing wall (1312) comprises multiple wall segments arranged at an angle, and the inner flow channel (1316) is connected between the first ink cartridge outlet (13153) and one of the multiple wall segments minimizing length of the inner flow channel (1316).

4. The bio-ink cartridge (131) according to claim 1, wherein an inner bottom surface of the ink cartridge shell (1311) comprises a first bottom surface located in the first accommodating chamber (1313) and a second bottom surface located in the second accommodating chamber (1314);
wherein the first bottom surface comprises a first low zone or a first low point to which the first bottom surface gradually descends from a location away therefrom; and/or
the second bottom surface comprises a second low zone or a second low point to which the second bottom surface gradually descends from a location away therefrom.

5. The bio-ink cartridge (131) according to claim 1,
wherein the ink cartridge shell (1311) comprises an ink cartridge positioning structure (13112) and an elastic snap-in structure (13113) externally arranged on opposite sides; and/or
the ink cartridge shell (1311) comprises a connecting structure (13114) configured to connect multiple bio-ink cartridges (131) to form a bio-ink cartridge assembly (130).

6. The bio-ink cartridge (131) according to claim 1, wherein the bio-ink cartridge (131) comprises first sealing structures (135) having a central through-hole, which are arranged in the first ink cartridge inlet (13151), the first ink cartridge outlet (13153), the second ink cartridge inlet (13152) and the second ink cartridge outlet (13154), respectively.

7. The bio-ink cartridge (131) according to claim 1, wherein the bio-ink cartridge (131) further comprises:
a first guide wall (1317) which is arranged in the first accommodating chamber (1313) and extends downwards from a radial outside of the first ink cartridge outlet (13153); and/or
a second guide wall (1318) which is arranged in the second accommodating chamber (1314) and extends downwards from a radial outside of the second ink cartridge outlet (13154).

8. The bio-ink cartridge (131) according to claim 1, wherein the bio-ink cartridge (131) further comprises a reinforcing structure (1319) which is arranged in the cavity and located at at least one of the first ink cartridge inlet (13151), the first ink cartridge outlet (13153), the second ink cartridge inlet (13152) or the second ink cartridge outlet (13154).

9. The bio-ink cartridge (131) according to claim 1, wherein at least one of a chamber wall surface of the first accommodating chamber wall or a chamber wall surface of the second accommodating chamber is hydrophobic.

10. The bio-ink cartridge (131) according to claim 1, wherein a volume ratio of the first accommodating chamber (1313) to the second accommodating chamber (1314) is 10:1 to 25:1.

11. A bio-ink cartridge assembly (130), comprising the bio-ink cartridge (131) according to any one of claims 1 to 10.

12. The bio-ink cartridge assembly (130) according to claim 11, wherein the bio-ink cartridge assembly (130) further comprises an integrated connector (133) which comprises:
a connector body (1331) having at least one first connector flow channel (13311) and/or at least one second connector flow channel (13312), wherein an outlet end of the first connector flow channel (13311) is communicated with the first ink cartridge inlet (13151) of the bio-ink cartridge (131), and an outlet end of the second connector flow channel (13312) is communicated with the second ink cartridge inlet (13152) of the bio-ink cartridge (131); and
fluid connectors (1332) connected onto the connector body (1331), wherein an inlet end of the first connector flow channel (13311) is connected with a fluid connector (1332), and an inlet end of the second connector flow channel (13312) is connected with a fluid connector (1332).

13. The bio-ink cartridge assembly (130) according to claim 11, wherein the bio-ink cartridge assembly (130) further comprises an outlet integration head (134) which comprises an integration head body (1341) having:
at least one first through hole (13413) for a pipe line connected with the first ink cartridge outlet (13153) of the bio-ink cartridge (131) to pass through; and/or
at least one second through-hole (13415) for a pipe line connected with the second ink cartridge outlet (13154) of the bio-ink cartridge (131) to pass through.

14. The bio-ink cartridge assembly (130) according to claim 13, wherein the integration head body (1341) has:
at least one third through hole (13414) for a pipe line connecting the first ink cartridge outlet (13153) of the bio-ink cartridge (131) with a second chip inlet (1421) of a microfluidic chip to pass through; and/or
at least one fourth through hole (13416) for a pipe line connecting the second ink cartridge outlet (13154) of the bio-ink cartridge (131) with a first chip inlet (1411) of the microfluidic chip to pass through.

15. The bio-ink cartridge assembly (130) according to claim 13, wherein the outlet integration head (134) comprises a guide structure, the guide structure is configured to lead the pipe line passing through the first through hole (13413) through the first ink cartridge outlet (13153) of the bio-ink cartridge (131) into the first accommodating chamber (1313), and/or lead the pipe line passing through the second through hole (13415) through the second ink cartridge outlet (13154) of the bio-ink cartridge (131) into the second accommodating chamber (1314).

16. A microsphere preparation device, comprising the bio-ink cartridge (131) according to any one of claims 1 to 10, or comprising the bio-ink cartridge assembly (130) according to any one of claims 11 to 15.

17. A bio-block preparation instrument, comprising the bio-ink cartridge (131) according to any one of claims 1 to 10, or comprising the bio-ink cartridge assembly (130) according to any one of claims 11 to 15.

18. A microsphere preparation device, comprising:
a microsphere preparation material storage device which comprises a shear liquid storage space for storing a shear liquid and a microsphere stock solution storage space for storing a microsphere stock solution for preparation of the microspheres;
a microfluidic chip which comprises a first chip inlet communicated with the microsphere stock solution storage space and a second chip inlet communicated with the shear liquid storage space, wherein the microsphere stock solution is sheared into microdroplets by the shear liquid in the microfluidic chip, and a microdroplet-shear liquid mixture is output from a chip outlet of the microfluidic chip; and
a curing device which comprises a curing device inlet connected with the chip outlet of the microfluidic chip to receive the microdroplet-shear liquid mixture, wherein the microdroplets form the cured microspheres in the curing device.

19. The microsphere preparation device according to claim 18, comprising a microsphere preparation material pumping device connected with the microsphere preparation material storage device to drive the microsphere stock solution and the shear liquid into the microfluidic chip, drive the shear liquid to shear the microsphere stock solution in the microfluidic chip to form the microdroplets, and to drive the microdroplet-shear liquid mixture to flow out of the microfluidic chip; wherein the microsphere preparation material pumping device pumps a gas to the shear liquid storage space of the microsphere preparation material storage device and the microsphere stock solution storage space thereof respectively to enable the shear liquid to flow by changing pressure in the shear liquid storage space, and enable the microsphere stock solution to flow by changing pressure in the microsphere stock solution storage space; and gas on-off switches are arranged between the microsphere preparation material pumping device and the shear liquid storage space as well as between the microsphere preparation material pumping device and the microsphere stock solution storage space, respectively.

20. The microsphere preparation device according to claim 18, wherein the microsphere preparation material storage device comprises the bio-ink cartridge (131) according to any one of claims 1 to 10, or comprises the bio-ink cartridge assembly (130) according to any one of claims 11 to 15, the first accommodating chamber (1313) of the bio-ink cartridge (131) forms the shear liquid storage space, and the second accommodating chamber (1314) of the bio-ink cartridge (131) forms the microsphere stock solution storage space.

21. The microsphere preparation device according to claim 18, wherein microsphere preparation material storage device comprises at least two storage containers with a storage space formed thereinto respectively, and storage space acts as the microsphere stock solution storage space or the shear liquid storage space.

22. The microsphere preparation device according to claim 18, wherein the microsphere preparation device comprises a buffer device (150) which is arranged between the microfluidic chip and the curing device and comprises a buffer flow channel communicated with the chip outlet (1412) of the microfluidic chip and the curing device inlet of the curing device, and the buffer flow channel is configured to buffer or guide flow of the microdroplet-shear liquid mixture.

23. The microsphere preparation device according to claim 22, wherein the buffer device (150) comprises a buffer body (151) which comprises:
a mixture receiving port (15111) communicated with the chip outlet (1412) of the microfluidic chip to receive the microdroplet-shear liquid mixture;
a mixture output port (15121) communicated with the curing device inlet to output the microdroplet-shear liquid mixture from the buffer device (150); and
the buffer flow channel arranged between the mixture receiving port (15111) and the mixture output port (15121) and comprising a tapered segment with cross sectional area gradually decreasing from the mixture receiving port (15111) to the mixture output port (15121).

24. The microsphere preparation device according to claim 23, wherein the tapered segment comprises an arc flow channel with cross sectional area gradually decreasing from the mixture receiving port (15111) to the mixture output port (15121).

25. The microsphere preparation device according to claim 23, wherein the buffer flow channel further comprises an equal cross-sectional segment located downstream of the tapered segment in the flow direction.

26. The microsphere preparation device according to claim 23, wherein the microfluidic chip comprises multiple chip outlets (1412) which are simultaneously communicated with the mixture receiving port (15111).

27. The microsphere preparation device according to claim 26, wherein the buffer device (150) comprises a third sealing structure (153) through which the mixture receiving port (15111) is directly connected with the chip outlet (1412) of the microfluidic chip hermetically.

28. The microsphere preparation device according to claim 23, wherein the buffer device (150) further comprises a shear liquid receiving port (1521) configured to receive the shear liquid made up to the buffer flow channel to maintain a level in the buffer flow channel equal to or above a level of the microdroplet-shear liquid mixture received at the mixture receiving port (15111).

29. The microsphere preparation device according to claim 18, wherein the curing device comprises a curing device outlet and a curing flow channel connecting the curing device inlet with the curing device outlet, the curing device inlet is communicated with the chip outlet (1412) of the microfluidic chip to receive the microdroplet-shear liquid mixture; the microdroplets form cured microspheres in a process that the microdroplet-shear liquid mixture flows through the curing flow channel; and a microsphere-shear liquid mixture is formed of the microspheres and the shear liquid and output from the curing device outlet.

30. The microsphere preparation device according to claim 29, wherein the curing flow channel is helically coiled outwards or inwards.

31. The microsphere preparation device according to claim 29, wherein the curing flow channel comprises a tapered segment with a cross sectional area gradually decreasing from the curing flow channel inlet to the curing flow channel outlet.

32. The microsphere preparation device according to claim 29, wherein the curing device comprises a coiled pipe (162) in which the curing flow channel is formed.

33. The microsphere preparation device according to claim 32, wherein the curing device further comprises a disk body (161) which is provided with a helical groove coiled outwards or inwards, and the coiled pipe (162) is arranged in the helical groove.

34. The microsphere preparation device according to claim 18, wherein the curing device comprises:
a receiving container (21) which is configured to accommodate a receiving liquid immiscible with the microdroplets, allow the microdroplets output from the chip outlet of the microfluidic chip to enter the receiving liquid, the microdroplets cured into the microspheres in the receiving liquid; and
an isolation control unit which is configured to separate the microdroplets apart from each other in the receiving liquid before the cured microspheres are formed.

35. The microsphere preparation device according to claim 34, wherein the isolation control unit separates the microdroplets apart from each other in the receiving liquid to form the microspheres by manipulating at least one of the receiving container (21), the receiving liquid, the chip outlet of the microfluidic chip, or an outlet (18A) of an output pipe line (18) connected with the chip outlet of the microfluidic chip.

36. The microsphere preparation device according to claim 34, wherein the isolation control unit comprises at least one of following devices:
a container shifting device, which is in driving connection with the receiving container (21) for changing the position of the receiving container (21) when the receiving liquid receives the microdroplets;
a fluid circulation driving device which is configured to drive the receiving liquid to circulate when the receiving liquid receives the microdroplets;
a bubbling device which is configured to generate bubbles in the receiving liquid when the receiving liquid receives the microdroplets; or
a stirring device which is configured to agitate the receiving liquid when the receiving liquid receives the microdroplets.

37. The microsphere preparation device according to claim 34, wherein the microsphere preparation device comprises the output pipe line (18) connected with the chip outlet of the microfluidic chip, and the curing device further comprises an outlet position adjustment unit (22) for adjusting position of an outlet (18A) of the output pipe line (18).

38. The microsphere preparation device according to claim 34, wherein when the receiving liquid receives the microdroplets, at least one of the chip outlet of the microfluidic chip or the outlet (18A) of the output pipe line (18) connected with the chip outlet of the microfluidic chip are located below the receiving liquid level.

39. The microsphere preparation device according to claim 18, wherein the microsphere preparation device comprises a collection device (170) located downstream of the curing device and configured to collect the microsphere-shear liquid mixture output therefrom.

40. The microsphere preparation device according to claim 39, wherein the microsphere preparation device comprises a collection device temperature control unit for keeping the collection device (170) at a preset temperature or within a preset temperature range.

41. The microsphere preparation device according to claim 39, wherein the collection device (170) comprises:
a mixture collection container (171) which comprises a mixture receiving port and a shear liquid output port, wherein the curing device is provided with the curing device outlet, and the mixture receiving port of the mixture collection container (171) is communicated with the curing device outlet to receive the microsphere-shear liquid mixture; and
a shear liquid collection container (172) which comprises the shear liquid receiving port (1521) communicated with the shear liquid output port of the mixture collection container (171) to receive the shear liquid therefrom.

42. The microsphere preparation device according to claim 41, wherein
The microsphere preparation device comprises a buffer device (150) which is arranged between the microfluidic chip and the curing device and comprises the buffer flow channel communicated with the chip outlet (1412) of the microfluidic chip and the curing device inlet of the curing devicerespectively, and the buffer flow channel is configured to buffer or guide the flow of the microdroplet-shear liquid mixture; and
the collection device (170) comprises a shear liquid circulating pump (173) which is provided with an inlet communicated with the shear liquid collection container (172) and an outlet communicated with the buffer flow channel; and the shear liquid circulating pump (173) injects the shear liquid from the shear liquid collection container (172) back to the buffer flow channel of the buffer device (150).

43. The microsphere preparation device according to claim 18, wherein the microsphere preparation device further comprises a monitoring device (180) for monitoring preparation process of the microspheres, and the monitoring device (180) comprises a position-adjustable camera (183).

44. The microsphere preparation device according to claim 18, wherein the microsphere preparation device further comprises a control device for controlling microsphere preparation process.

45. The microsphere preparation device according to claim 18, wherein the microsphere preparation device comprises a microsphere preparation material temperature control unit for keeping at least one of the microsphere preparation material storage device or the microfluidic chip at a preset temperature or within a preset temperature range; and/or the microsphere preparation device comprises a curing device temperature control unit for keeping the curing device at a preset temperature or within a preset temperature range.

46. A bio-block preparation instrument, comprising the microsphere preparation device according to any one of claims 18 to 45.

47. A shell assembly device for coating microspheres or compositional structures composed of a microsphere and at least one shell, comprising:
a shell preparation material storage device comprising multiple storage spaces which comprise a cleaning liquid storage space for storing a cleaning liquid and a shell liquid storage space for storing a shell liquid; and
an assembling device in which the microspheres or the compositional structures are cleaned with the cleaning liquid, and then coated by the shell liquid to form a shell around each of the microspheres or the compositional structures.

48. The shell assembly device according to claim 47, wherein the shell assembly device comprises a shell preparation material pumping device configured to drive the cleaning liquid and/or the shell liquid to flow.

49. The shell assembly device according to claim 47, wherein the assembling device comprises a single assembling space or a partitioned assembling space in which the microspheres or the compositional structures are cleaned with the cleaning liquid, and then coated by the shell liquid to form a shell around each of the microspheres or the compositional structures.

50. The shell assembly device according to claim 49, wherein the assembling device (250) comprises the assembling space in communication with the cleaning liquid storage space and the shell liquid storage space; and the shell assembly device comprises a fluid switching tube (230) which comprises a tube body (231) as well as multiple inlet connectors and an outlet connector (233) arranged on and communicated with the tube body (231), the multiple inlet connectors comprise a cleaning liquid inlet connector connected with the cleaning liquid storage space and a shell liquid inlet connector connected with the shell liquid storage space, and the outlet connector (233) is in communication with the assembling space of the assembling device (250).

51. The shell assembly device according to claim 49, wherein the assembling device (250) comprises the assembling space in communication with the cleaning liquid storage space and the shell liquid storage space, and the shell assembly device further comprises a switching control device (240) which selectively controls the assembling space to be communicated with one of the storage spaces and disconnect from the others.

52. The shell assembly device according to claim 51, wherein the switching control device (240) comprises a rotating shaft (242) and multiple control cams arranged on the rotating shaft (242) in a non-rotatable way; the multiple control cams are arranged one-to-one corresponding to the multiple inlet connectors; and the rotating shaft (242) is configured to rotate to change rotation angles of the multiple control cams to manipulate on-off states of the pipe lines connecting the assembling space with the storage spaces.

53. The shell assembly device according to claim 51, wherein the assembling space is connected with the corresponding storage space through a hose, and the switching control device (240) further comprises multiple transmission units (244) arranged one-to-one corresponding to the multiple inlet connectors; the transmission unit (244) comprises a stopper (2443) and a moving member (2441), the moving member (2441) is located between the stopper (2443) and the corresponding control cam; and the hose is located between the stopper (2443) and the moving member (2441) of the corresponding transmission unit (244), and manipulated to open or close by adjusting a gap between the moving member (2441) and the stopper (2443) through changing the angle of the control cam.

54. The shell assembly device according to claim 49, wherein the assembling device (250) comprises an assembling container and a filter element (252), and the microspheres are subject to cleaning and shell assembly in the assembling space defined by the assembling container and the filter element (252).

55. The shell assembly device according to claim 54, wherein the assembling container comprises an assembling tube (251) in which the assembling space is formed; a liquid receiving port (2511) is provided at an upper end of the assembling tube (251), and an opening (2512) is provided at a lower end thereof; and the filter element (252) is arranged at the bottom of the opening (2512).

56. The shell assembly device according to claim 55, wherein the assembling device (250) further comprises a receiving seat (254) which comprises a liquid receiving space, and the assembling tube (251) and the filter element (252) are arranged on the receiving seat (254) and located above the liquid receiving space.

57. The shell assembly device according to claim 56, wherein the receiving seat (254) further comprises a drainage flow channel (2542) communicated with the liquid receiving space; the shell assembly device further comprises a suction device and a liquid waste storage container (271); and the suction device is connected between the drainage flow channel (2542) and the liquid waste storage container (271) to suck the liquid from the liquid receiving space to the liquid waste storage container (271).

58. The shell assembly device according to claim 47, further comprising an oscillation device which oscillates the cleaning liquid and/or the shell liquid when the microspheres or the compositional structures are cleaned and the shells are assembled.

59. The shell assembly device according to claim 49, wherein the assembling device comprises a sprayer (36) in communication with the cleaning liquid storage space, and the sprayer (36) is configured to spray the cleaning liquid to rinse the microspheres or the compositional structures in the assembling space.

60. The shell assembly device according to claim 59, wherein the assembling device comprises a filtrate container (33) and a first accommodating screen (35) which is located on the filtrate container (33) to accommodate the microspheres or the compositional structures; the assembling space comprises an accommodating space of the first accommodating screen (35); the sprayer (36) is arranged towards a screen opening on the first accommodating screen (35) to rinse the microspheres or the compositional structures in the accommodating space of the first accommodating screen (35); and the filtrate container (33) is configured to receive the cleaning liquid from the first accommodating screen (35).

61. The shell assembly device according to claim 60, wherein the assembling device comprises a seal cover (37) and a suction device; the seal cover (37) is located between the sprayer (36) and the screen opening of the first accommodating screen (35), and peripherally covers the sprayer (36) and the screen opening of the first accommodating screen (35); and the suction device is communicated with the filtrate container (33) to suck a fluid in the filtrate container (33).

62. The shell assembly device according to claim 49, wherein the shell assembly material storage device comprises a shell liquid container (92) having the shell liquid storage space; the assembling device comprises a second accommodating screen which is arranged in the shell liquid container (92); the assembling space comprises an accommodating space of the second accommodating screen; and the microspheres or the compositional structures in the accommodating space of the second accommodating screen are below the liquid level of the shell liquid container (92) so as to be coated by the shell liquid to form a shell around each of the microspheres or the compositional structures.

63. The shell assembly device according to claim 47, wherein the shell assembly device further comprises a control device for manipulating shell assembly process.

64. The shell assembly device according to claim 47, wherein the assembling device (250') comprises:
an assembling tube (251) with an assembling space;
an assembling tube control unit (300) which comprises an assembling tube fixing portion and an assembling tube driving portion; wherein the assembling tube fixing portion is configured to fix the assembling tube (251), and the assembling tube driving portion is in driving connection with the assembling tube fixing portion to drive the assembling tube fixing portion and the assembling tube (251) fixed thereby to rotate; and
a liquid adding/sucking unit (400) which comprises a feeding needle (413) and a feeding needle moving mechanism; wherein the feeding needle moving mechanism is in driving connection with the feeding needle (413) to drive the feeding needle (413) to move relative to the assembling tube fixing portion so as to change a relative position between the feeding needle (413) and the assembling tube (251).

65. The shell assembly device according to claim 64, wherein the assembling tube control unit (300) comprises a mounting base (310) on which the assembling tube driving portion and the assembling tube fixing portion are arranged, and the assembling tube fixing portion is fixedly connected with an output end of the assembling tube driving portion.

66. The shell assembly device according to claim 64, wherein the feeding needle moving mechanism comprises a feeding needle vertical driving mechanism configured to drive the feeding needle (413) to move in a vertical direction; and the feeding needle vertical driving mechanism comprises:
a vertical guide rail assembly which comprises a guide rail and a translation portion vertically moving along the guide rail;
a driving device in driving connection with the translation portion and configured to drive the translation portion to vertically move along the guide rail; and
a moving arm (412) which is fixedly connected with the translation portion of the vertical guide rail assembly, and the feeding needle (413) is arranged on the moving arm (412).

67. The shell assembly device according to claim 66, wherein the feeding needle moving mechanism further comprises a horizontal position adjustment mechanism (418) for adjusting horizontal position of the feeding needle (413); and the feeding needle (413) is connected with the moving arm (412) through the horizontal position adjustment mechanism (418).

68. The shell assembly device according to claim 64,
wherein the liquid adding/sucking unit (400) comprises a liquid level sensor (417) which is configured to acquire a detection signal characterizing a stratified interface position of substances in the assembling tube (251); and
the shell assembly device comprises a control device which is in signal connection with the liquid level sensor (417) and the feeding needle moving mechanism to operate the feeding needle moving mechanism to adjust the position of the feeding needle (413) according to the detection signal from the liquid level sensor (417).

69. The shell assembly device according to claim 47, wherein the shell preparation material storage unit (55) comprises:
a storage box mounting base (5502); and
a storage box (5501) which is arranged on the storage box mounting base (5502) and has the cleaning liquid storage space (5503) and the shell liquid storage space.

70. The shell assembly device according to claim 69, wherein the shell preparation material storage device (55) comprises:
two storage box mounting rails (5506) mounted at both sides of the storage box mounting base (5502) side by side, wherein the storage box (5501) is mounted on the two storage box mounting rails (5506);
a storage box cover (5507) which is movably arranged relative to the storage box (5501), and covers above the storage box (5501) in a closure state and leaves above the storage box (5501) in an open state; and
a cover moving mechanism which is in driving connection with the storage box cover (5507) and configured to drive the storage box cover (5507) to move relative to the storage box (5501) so as to switch the storage box cover (5507) between the closure state and the open state.

71. The shell assembly device according to claim 47, wherein the assembling device comprises: a centrifuge tube having an assembling space;
a centrifugal unit (51) which is configured to centrifuge substances in the assembling space.

72. The shell assembly device according to claim 71, wherein the centrifugal unit (51) comprises:
a centrifuge rack mounting portion (5103);
a centrifuge rack (5101) which is rotatably mounted on the centrifuge rack mounting portion (5103) about a centrifugal axis and comprises a centrifuge tube bearing portion for mounting the centrifuge tube, wherein the centrifuge tube bearing portion is spaced apart from the centrifugal axis; and
a centrifugal motor (5104) which is mounted on the centrifuge rack mounting portion (5103) and in driving connection with the centrifuge rack (5101) to drive the centrifuge rack (5101) to rotate.

73. The shell assembly device according to claim 71, wherein the assembling device further comprises a centrifuge tube storage unit (510); the centrifuge tube storage unit (510) comprises a centrifuge tube mounting rack (51003); and the centrifuge tube mounting rack (51003) has a centrifuge tube storage portion for storing the centrifuge tube.

74. The shell assembly device according to claim 73,
wherein the centrifuge tube mounting rack (51003) further has a positioning member mounting hole in communication with the centrifuge tube storage portion;
the centrifuge tube storage unit (510) comprises a centrifuge tube positioning member which is mounted in the positioning member mounting hole; and the centrifuge tube positioning member is configured to fix the centrifuge tube together with the centrifuge tube storage portion.

75. The shell assembly device according to claim 73, wherein the centrifuge tube storage unit (510) comprises a liquid level sensor (51006) which is configured to detect a stratified interface of substances in the centrifuge tube.

76. The shell assembly device according to claim 71, wherein the assembling device further comprises a balance tube storage unit (511) which comprises a balance tube (5111) and has a balance tube storage space (5112) for storing the balance tube (5111); and the balance tube (5111) is configured to balance the centrifuge tube when the centrifugal unit (51) centrifuges the substances in the centrifuge tube.

77. The shell assembly device according to claim 71, wherein the assembling device comprises a liquid adding/sucking unit (515) which is configured to add a liquid to or suck the liquid from the assembling space.

78. The shell assembly device according to claim 77, wherein the assembling device comprises a three-dimensional motion unit (513) on which the liquid adding/sucking unit (515) is mounted.

79. The shell assembly device according to claim 71, wherein the assembling device comprises a grabbing unit (514) for operating the centrifuge tube.

80. The shell assembly device according to claim 79, wherein the assembling device comprises the three-dimensional motion unit (513) on which the grabbing unit (514) is mounted.

81. The shell assembly device according to claim 71, wherein the assembling device comprises:
a three-dimensional motion unit (513);
a liquid adding/sucking unit (515) which is configured to add a liquid to or suck a liquid from the assembling space, and mounted on the three-dimensional motion unit (513); and
a grabbing unit (514) which is configured to operate the centrifuge tube and mounted on the three-dimensional motion unit (513).

82. The shell assembly device according to claim 81, wherein the three-dimensional motion unit (513) comprises:
a first driving component (5131) on which the grabbing unit (514) is arranged, and configured to drive the grabbing unit (514) to move in a vertical direction;
a second driving component (5132) on which the first driving component (5131) is arranged, and configured to drive the first driving component (5131) to move in a second horizontal direction;
a third driving component (5133) on which the second driving component (5132) is arranged, and configured to drive the second driving component (5132) to move in a first horizontal direction perpendicular to the second horizontal direction;
a fourth driving component (5134) on which the liquid adding/sucking unit (515) is arranged, and configured to drive the liquid adding/sucking unit (515) to move in the vertical direction; and
a fifth driving component (5135) arranged on the third driving component (5133), wherein the third driving component (5133) is further configured to drive the fifth driving component (5135) to move in the first horizontal direction; the fourth driving component (5134) is arranged on the fifth driving component (5135), and the fifth driving component (5135) is configured to drive the fourth driving component (5134) to move in the second horizontal direction.

83. The shell assembly device according to claim 71, wherein the assembling device comprises a residual liquid absorbing unit (53).

84. The shell assembly device according to claim 83, wherein the residual liquid absorbing unit (53) comprises a residual liquid absorbing mounting rack (5301) and an oil-absorbing sponge arranged thereon.

85. A bio-block preparation instrument, comprising the shell assembly device according to any one of claims 47 to 84.

86. A bio-block preparation instrument, comprising the microsphere preparation device according to any one of claims 18 to 45 and the shell assembly device according to any one of claims 47 to 84, wherein the microsphere preparation device and the shell assembly device are arranged integrally or separately.

87. A bio-ink preparation instrument, comprising:
the shell assembly device according to any one of claims 71 to 84; and
a bio-ink generation device which is configured to mix the bio-block formed by the shell assembly device with a carrier material to form the bio-ink.

88. The bio-ink preparation instrument according to claim 87, wherein the bio-ink generation device comprises:
a barrel having a mixing space for mixing the bio-block and the carrier material;
and a carrier material loading unit for injecting the carrier material into the mixing space.

89. The bio-ink preparation instrument according to claim 88, wherein the assembling device of the shell assembly device comprises the liquid adding/sucking unit (515) for adding the liquid to or sucking the liquid from the assembling space, and the carrier material loading unit is mounted on the liquid adding/sucking unit (515).

90. The bio-ink preparation instrument according to claim 88, wherein the carrier material loading unit comprises a syringe (5153) and a syringe pushing portion in driving connection with the syringe and configured to push a piston of the syringe to move.

91. The bio-ink preparation instrument according to claim 90, wherein the syringe pushing portion comprises:
a slider (5152) movably arranged along an axis of the syringe (5153) for pushing the piston of the syringe (5153); and
a syringe driving motor (5151) in driving connection with the slider (5152) and configured to drive the slider (5152) to move to push the piston of the syringe (5153).

92. The bio-ink preparation instrument according to claim 88, wherein the carrier material loading unit comprises a syringe (5153) and a syringe thermal insulation device (5154) arranged on the surface of the syringe (5153) for thermal insulation thereof.

93. The bio-ink preparation instrument according to claim 88, wherein the bio-ink generation device comprises a weighing unit (54), and the weighing unit (54) comprises an electronic scale (5402) and a weighing tray (5401) arranged thereon, and the weighing tray (5401) comprises a hollow cylinder structure for storing the syringe.

94. The bio-ink preparation instrument according to claim 88, wherein the bio-ink generation device comprises a scraping unit (58) which comprises a correction mounting rack (5803) as well as a scraping plate (5801), a scraping line (5802) and a distance measuring sensor (5804) arranged thereon.

95. The bio-ink preparation instrument according to claim 88, wherein the bio-ink generation device comprises a barrel thermal insulation unit (59) for thermally insulating the barrel, and the barrel thermal insulation unit (59) comprises a thermal insulation box and a heating device for heating the thermal insulation box.

96. The bio-ink preparation instrument according to claim 88, wherein the bio-ink generation device comprises a mixing unit (512) for mixing the bio-block and the carrier material in the barrel.

97. The bio-ink preparation instrument according to claim 96, wherein the mixing unit (512) comprises:
a mixing unit mounting rack (5129);
a mixing motor (5121) mounted on the mixing unit mounting rack (5129); and
a swing arm (5127) mounted on an output shaft of the mixing motor (5121) and having a barrel bearing portion (5124) for mounting the barrel.

98. The bio-ink preparation instrument according to claim 97, wherein the mixing unit (512) comprises an angular sensor (5128) mounted on the output shaft of the mixing motor (5121) for detecting a rotating position of the mixing motor (5121).

99. The bio-ink preparation instrument according to claim 97, wherein the mixing unit (512) comprises a buffer assembly mounted on the mixing unit mounting rack (5129) for buffering and limiting the swing arm (5127).

100. The bio-ink preparation instrument according to claim 99, wherein the two buffer assemblies are respectively located at two ends of a swing path of the swing arm (5127), and each of the buffer assemblies comprises:
a buffer post (5126) mounted on the mixing unit mounting rack (5129); and
a buffer cushion (5125) arranged on the buffer post (5126) and located between the buffer post (5126) and a corresponding end of the swing path of the swing arm (5127).

101. The bio-ink preparation instrument according to claim 97,
wherein the barrel bearing portion (5124) comprises a barrel mounting hole and a fixing piece mounting portion;
the mixing unit (512) comprises a fixing piece (5122), a torsion spring and an pneumatic cylinder (5123); the pneumatic cylinder (5123) is mounted on the mixing unit mounting rack (5129); the fixing piece (5122) comprises a pressing end, a driving end and a hinge portion therebetween; the hinge portion is hinged to the fixing piece mounting portion through the torsion spring; the driving end is matched with the pneumatic cylinder (5123); and the pneumatic cylinder (5123) drives the fixing piece (5122) to rotate about the fixing piece mounting portion so as to switch the pressing end between a pressing state that the pressing end approaches an inlet of the barrel mounting hole and a relief state that the pressing end is away from the inlet of the barrel mounting hole.

102. A bio-ink preparation system, comprising the bio-ink preparation instrument according to any one of claims 87 to 101.

103. A bio-ink preparation system according to claim 102, comprising the microsphere preparation device according to any one of claims 18 to 45.
